(19) Europäisches Patentamt
European Patent Office
Office européen des brevets

(11) EP 3 719 129 A1

(12) EUROPEAN PATENT APPLICATION

(43) Date of publication:
07.10.2020 Bulletin 2020/41

(21) Application number: 20165687.3

(22) Date of filing: 12.03.2015

(51) Int Cl.:
*C12N 15/113* (2010.01) *C12N 15/29* (2006.01)
*C12N 15/82* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA**

(30) Priority: 12.03.2014 US 201461951569 P

(62) Document number(s) of the earlier application(s) in accordance with Art. 76 EPC:
**15761116.1 / 3 116 998**

(71) Applicant: **The University of Sydney**
**NSW 2006 (AU)**

(72) Inventors:
- **GLEN, John**
  **New South Wales, 2250 (AU)**
- **WATERHOUS, Peter Michael**
  **Queensland, 4064 (AU)**
- **NARVA, Kenneth E.**
  **Zionsville, IN 46077 (US)**
- **LARRINUA, Ignacio Mario**
  **Indianapolis, IN 46254 (US)**

(74) Representative: **Zwicker, Jörk**
**ZSP Patentanwälte PartG mbB**
**Hansastraße 32**
**80686 München (DE)**

Remarks:
•This application was filed on 25-03-2020 as a divisional application to the application mentioned under INID code 62.
•Claims filed after the date of filing of the application / after the date of receipt of the divisional application (Rule 68(4) EPC).

# (54) RNA PRODUCTION IN HIGHER PLANTS

(57) The invention relates to RNA production and processing in higher plants.

EP 3 719 129 A1

## Description

### Field of the invention

The invention relates to RNA production and processing in higher plants, including RNA interference in higher plants, and to expression of genes in chloroplasts and related plastids of higher plants.

### Background of the invention

Reference to any prior art in the specification is not an acknowledgment or suggestion that this prior art forms part of the common general knowledge in any jurisdiction or that this prior art could reasonably be expected to be understood, regarded as relevant, and/or combined with other pieces of prior art by a skilled person in the art.

Utilisation of host RNA for silencing essential genes of a cognate pest is an emerging approach for pest control. This approach may be referred to as '*transkingdom RNA interference*' or '*transkingdom RNAi*'. Generally it is understood that when double stranded RNA (dsRNA) produced by the host is ingested by the pest, the pest RNA interference (RNAi) machinery converts the ingested dsRNA into siRNA, or other RNA form that is capable of inducing essential gene silencing or related events in the pest that impacts pest survival. See: Terenius O. et al. 2011 J. Insect Physiol. 57:231-245 for examples of this approach applicable to Lepidoptera. RNA interference (RNAi) is a process utilizing endogenous cellular pathways, whereby an interfering RNA (iRNA) molecule (*e.g.*, a dsRNA molecule) that is specific for all, or any portion of adequate size, of a target gene sequence results in the degradation of the mRNA encoded thereby. In recent years, RNAi has been used to perform gene "knockdown" in a number of species and experimental systems; for example, *Caenorhabditis elegans,* plants, insect embryos, and cells in tissue culture. *See, e.g.*, Fire et al. (1998) Nature 391:806-811; Waterhouse et al (1998) Proc Natl Acad Sci USA 95: 13959-139; Wesley V et al (2001) Plant J. 27: 581-590; Martinez et al. (2002) Cell 110:563-574; McManus and Sharp (2002) Nature Rev. Genetics 3:737-747.

It is well established that high levels of dsRNA are required to obtain silencing of pest genes including lepidopteran genes (Terenius O *supra*; Mao Y. et al. 2007 Nat. Biotechnol. 25: 1307-1313; Kumar P. et al. 2012 PLoS ONE 7:e31347).

WO2012/054919 is a study that transforms chloroplasts and nuclei of a lower plant (*Chlamydomonas*) with constructs containing essential genes for mosquito survival. The transformed *Chlamydomonas* is then fed to mosquitoes to demonstrate that the feed can be used to control mosquito populations.

WO2012/054919 finds that both chloroplast transformation and nuclear transformation of lower plants provide for a useful feed for control of mosquito population. Specifically, according to WO2012/054919, the results show that feeding transgenic *Chlamydomonas* expressing 3HKT inverted repeats expressed either from the chloroplast or nuclear genome was effective in inhibiting the growth of mosquito larvae and ultimately causing their death (WO2012/054919 at [00219]).

Elsewhere it is hypothesised from studies of nuclear transformants that dsRNA must be delivered to the insect as it feeds on the host for induction of transkingdom RNAi. See Mao and Kumar, *supra.* This means that effective pest control requires a host that produces and maintains a steady state level of dsRNA so that when the pest feeds on the host at a particular time, dsRNA is available to be fed to the pest. How to best produce and maintain a steady state level of dsRNA in higher or lower plants remains unclear.

In lower plants, WO2012/054919 hypothesises at [00220] that expression of silencing RNA in chloroplasts may be important for induction of transkingdom RNAi. However, WO2012/054919 does not conclude whether this is because chloroplast expression enables a steady state level of silencing RNA, or whether it is the plastid packaging of the chloroplast that protects the silencing RNA as it is ingested. The importance of plastid packaging has been proposed by others. See Bogarad L. 2000 TIBTECH 18: 257- 263; McBride K et al., 1995 Nature Biotechnology 13: 362-365; Verma D and Daniell H 2007 Am Soc Plant Biol. 145:1129-1143.

It remains unclear from WO2012/054919 whether RNA expressed by a lower plant chloroplast transformant consists of dsRNA. Specifically, WO2012/054919 does not measure dsRNA or siRNA in *Chlamydomonas* chloroplast transformants, or in the insects that ingest the transformants. Therefore, WO2012/054919 does not show that dsRNA can be produced in chloroplasts of *Chlamydomonas* or other lower plants in amounts sufficient to facilitate transkingdom RNAi.

Further, it appears from WO2012/054919 that dsRNA is produced in the *Chlamydomonas* nuclear transformants as this transformant, according to WO2012/054919 was ostensibly as useful for transkingdom RNAi induction as a chloroplast transformant. As dsRNA is essential for transkingdom RNAi, one implication may be that dsRNA is better able to accumulate in the nucleus of a lower plant than in the nucleus of a higher plant. This would point to a divergence in the RNA processing pathways of higher and lower plants. There are many examples of differences between RNA processing in higher and lower plants. See in particular: Casas-Mollano J. et al. 2008 Genetics 179: 69-81. On the understanding that chloroplast and nuclear transformants equally accumulate dsRNA, it would seem that the slight difference in efficacy of chloroplast and nuclear transformants for induction of transkingdom RNAi could arise from the plastid packaging unique to chloroplasts, rather than superior accumulation of silencing RNA in the plastid. This is in fact a hypothesis of WO2012/054919 at [00220].

Importantly, plastid packaging is not of itself a mechanism that enables production and maintenance of steady state level of dsRNA. Packaging merely protects what has been produced after ingestion. Therefore, it is understood that plastid packaging itself is not sufficient for the production of high levels of dsRNA that are known to be required for induction of transkingdom RNAi.

A further important difference between the chloroplast RNA processing pathways of lower and higher plants is that lower plants, as exemplified by *Chlamydomonas,* do not have an RNA editing system, whereas this system is present in the chloroplast RNA processing pathways of higher plants. See Stern et al. 2010 Annu. Rev. Plant Biol. 61:125-55. Thus, RNA corresponding to the target gene of a cognate pest that is produced in the lower plant chloroplast is understood to have the same sequence of the relevant pest target gene, increasing the likelihood of formation of appropriately sequence specific siRNA in the pest and induction of transkingdom RNAi. The presence of an RNA editing system in higher plant chloroplasts raises the question of whether such an outcome would be possible using chloroplast transformants of higher plants.

As discussed, it is difficult to maintain a steady state level of dsRNA in higher organisms because it is rapidly broken down. In higher plants this degradation is thought to involve siRNA, but other as yet undefined RNAi related mechanisms, or other mechanisms could be involved.

There have been some advances in our understanding of RNA processing pathways in chloroplasts of higher plants. However, there is much that remains unknown. Researchers often ask why the expression of so few genes, fewer than 100 in a typical chloroplast genome, is so complex. See Stern *supra.*

It is understood that the RNA processing pathways of the higher plant chloroplast are different from those in the higher plant cell nucleus and cytosol. This is because the plastid is of prokaryotic origin, whereas the plant cell nucleus and cytosol are eukaryotic. Some consider that the chloroplast, being derived from a cyanobacterial ancestor, combines prokaryotic and eukaryotic features of gene expression and is regulated by many nucleus-encoded proteins. See Stern *supra.* However, the extent to which the pathways are different and the relevance of those differences to RNA processing are not known.

Some have suggested that given the differences between prokaryotes and eukaryotes, those mechanisms that are seen to operate in eukaryotic cells, but not prokaryotic cells, should not operate in chloroplasts. RNAi is one particular mechanism. At the same time, others have observed the presence of siRNA derived from RNAs that accumulate in the chloroplast, pointing to RNAi mechanisms operating in the chloroplast. See Martinez de Alba A.E. et al. 2002 Am. Soc Microbiol. 76: 13094-13096. Further a chloroplast homolog of RNase III may also be involved in mRNA processing. RNase III is a member of a large family of dsRNA-dependent endoribonucleases including enzymes related to RNA silencing such as Dicer. See: Stern *supra.* The observance of nucleus derived factors in the chloroplast is perhaps unsurprising given the finding of many nucleus encoded *trans* acting factors in the chloroplast, including RNA editing and splicing factors.

Generally, it is not known how the steady state level of an RNA of an endogenous gene in the chloroplast of higher plants is controlled. It is also not known whether the relevant mechanisms would equally apply to the expression of exogenous genes in chloroplasts of higher plants. Further, the impact of overexpression of transgene in a chloroplast on steady state levels of RNA is not known. It is perhaps for these reasons that many have embarked on a transkingdom RNAi approach in higher plants on the basis of nuclear transformation, as the RNA processing machinery of the nucleus is more clearly understood in higher plants, and the outcome is predictable.

These studies initially focused on the use of cells that lack the dsRNA processing machinery, thereby avoiding degradation of dsRNA. One problem has been the impact on growth performance of a plant lacking dsRNA processing machinery. This problem has been improved with the provision of modified constructs that enable formation and protection of dsRNA in the nucleus of higher plants. See for example US20090263364, US20070011775, US20090263364, US20080194504; US20060095987. Fenner B. et al. 2006 J. Virol. 80: 6822-6833; and Salomon W. et al 2010 Nucl. Acids. Res. 38: 3771-3779. These improvements and developments in RNAi inducing constructs have ostensibly established nuclear transformation as a preferred approach to dsRNA production for transkingdom RNAi.

There remains a need for alternative approaches, or improved approaches for the production and accumulation of dsRNA in higher plants.

**Summary of the invention**

The invention seeks to address the above mentioned need and in one embodiment provides a plastid of a vascular plant, the plastid comprising:

- a nucleic acid construct including:

    - a coding region for encoding a double stranded RNA (dsRNA) molecule;
    - a promoter operable in the plastid for enabling production of the dsRNA molecule;

- wherein the dsRNA has a sequence enabling the production of siRNA therefrom.

In another embodiment there is provided a plastid of a vascular plant, the plastid comprising:

- a nucleic acid construct including:

    - a coding region for encoding a double stranded RNA (dsRNA) molecule;
    - a promoter operable in the plastid for enabling production of the dsRNA molecule;

- wherein the coding region has a nucleotide sequence that is not found in the genome of the plant.

In another embodiment there is provided a plastid of a vascular plant, the plastid comprising:

- a nucleic acid construct including:

    - a coding region for encoding a double stranded RNA (dsRNA) molecule;
    - a promoter operable in the plastid for enabling production of the dsRNA molecule;

- wherein the plastid does not comprise siRNA molecules including a sequence of the dsRNA.

In another embodiment there is provided a plastid of a vascular plant, the plastid comprising:

- a nucleic acid construct including:

    - a coding region for encoding a double stranded RNA (dsRNA) molecule;
    - a promoter operable in the plastid for enabling production of the dsRNA molecule;

- dsRNA
- wherein the dsRNA contains substantially all of the nucleotide sequence encoded by the coding region.

In further embodiments there is provided a nucleic acid construct for transformation of a vascular plant, comprising:

- a coding region for encoding a double stranded RNA (dsRNA) molecule;
- a promoter operable in the plastid for enabling production of the dsRNA molecule;
- one or more integration sites selective for integration of the construct into a plastid genome of a vascular plant;
- wherein the dsRNA has a sequence enabling the production of siRNA therefrom.

In further embodiments there is provided a nucleic acid construct for transformation of a vascular plant, comprising:

- a coding region for encoding a double stranded RNA (dsRNA) molecule;
- a promoter operable in the plastid for enabling production of the dsRNA molecule;
- one or more integration sites selective for integration of the construct into a plastid genome of a vascular plant;

    - wherein the coding region has a nucleotide sequence that is not found in the genome of the plant.

In further embodiments there is provided a plant, or propagating material derived therefrom containing a plastid or construct as described above.

In further embodiments there is provided a method of accumulating dsRNA in a cell of a vascular plant, the method including:

- transforming a plastid of a vascular plant with a nucleic acid construct of any one of the above described embodiments;

    - providing conditions to the cell for production of dsRNA in the plastid from the nucleic acid construct,

thereby accumulating dsRNA in a cell of a vascular plant.

Further aspects of the present invention and further embodiments of the aspects described in the preceding paragraphs will become apparent from the following description, given by way of example and with reference to the accompanying drawings.

**Brief description of the drawings**

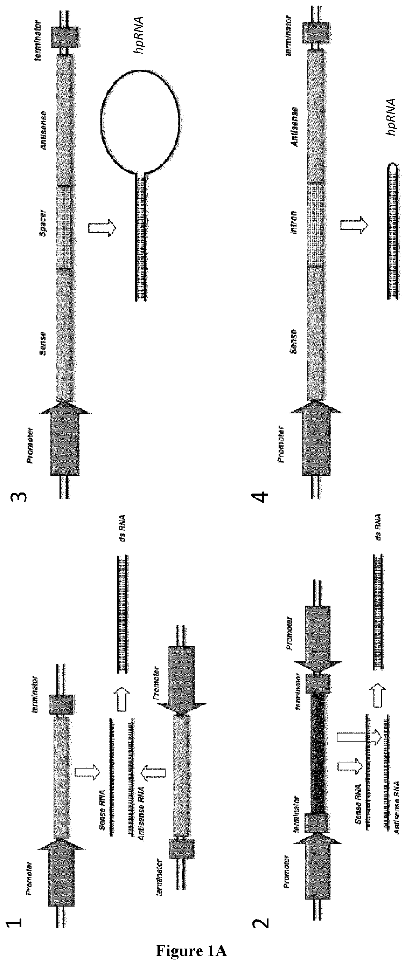

Figure 1A. **Schematic of the** ways **to produce dsRNA or hairpin (hpRNA) from integration into nuclear or chloroplast genomes.**

**Fig1A-1.** Two independent transcription units in which one produces a sense transcript and the other produces an antisense transcript (as first described in Waterhouse PM et al. 1998 Proc Natl Acad Sci USA 95: 13959-139). The transcription units may be co-located in the genome (such as produced by the units being introduced on the same T-DNA) or in unrelated locations (such as on different chromosomes). The sense and antisense RNAs are required to meet and hybridise to form the dsRNA.

**Fig 1A-2.** One "coding region" sequence is placed between two "inwardly-pointing/convergent" promoters (as first described by Timmons L and Fire A. 1998 Nature 395: 854) such that one will cause the transcription of one DNA strand to produce sense RNA and the other promoter will direct transcription of the other DNA strand to make antisense RNA. The two RNAs are required to meet and hybridize to form dsRNA.

**Fig 1A-3.** One transcription unit driven by one promoter that generates a single RNA that has the capacity to fold-back and self hybridize to make a hairpin (hp) RNA with a duplex structured stem resembling dsRNA (as first described in Waterhouse et al. 1998 Proc Natl Acad Sci USA 95: 13959-139). The first region of the transcript may be in the sense orientation with respect to the last region of the transcript (or vice-versa). It is important to have a "spacer" region to separate the sense and antisense regions in order to stabilise the DNA construct. In this instance the spacer region will be transcribed as part of the hpRNA transcript and will form a loop.

**Fig1A-4.** One transcription unit driven by one promoter that generates a single RNA that has the capacity to fold-back and self hybridize to make a hairpin (hp) RNA with a duplex structured stem resembling dsRNA. The first region of the transcript may be in the sense orientation with respect to the last region of the transcript (or vice-versa). It is important to have a "spacer" region to separate the sense and antisense regions in order to stabilise the DNA construct. In this instance the spacer region encodes an intron which will be transcribed but then spliced out to produce a hpRNA transcript with a very small loop (as first described in Smith NA et al 2000 Nature 407: 319-320).

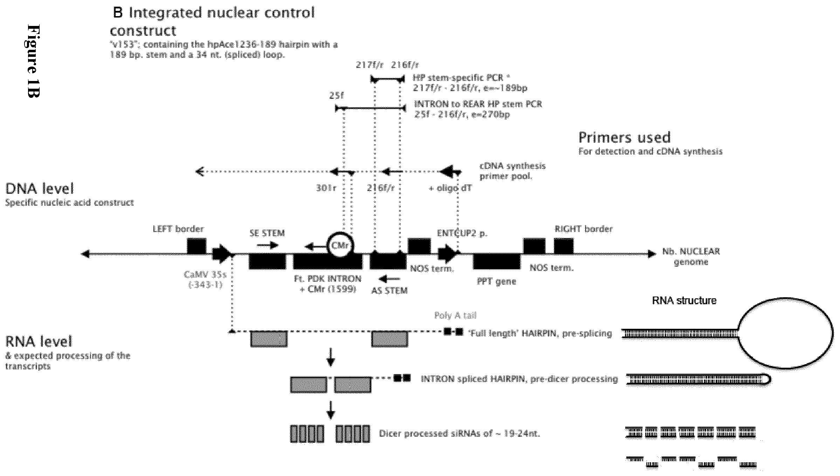

**Figure 1B. Schematic of the nuclear integration constructs when integrated into the nuclear genome.** The v153 nuclear transformation construct is depicted in its integrated form. This construct is designed to express the Ha-HpAce1236-189 hairpin from the CaMV 35s promoter. The backbone is a derivative of the standard binary agrobacterium transformation vector, pORE-03. The integration site in the host nuclear genome will vary depending upon the standard integration dynamics of agrobacterium mediated transformation. The primers used for subsequent cDNA synthesis steps (301r, 216f/r, and oligo dT), and RT-PCR detection steps (217f/r + 216 f/r, and 25f + 216 f/r) are depicted with the expected product sizes (e=..) indicated for the latter sets. Shown is the expected RNA products and structures including those of and the species that will be producedremain by following intron splicing and those following processing by the standard RNAi-type pathways known to operate on standard nuclear / cytoplasmic RNAs.

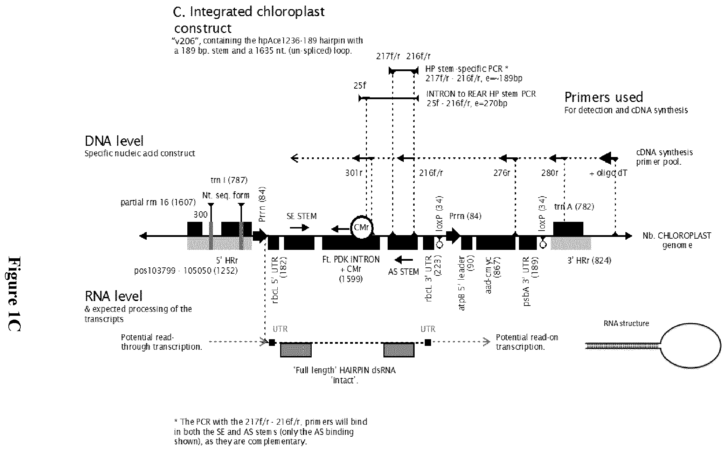

**Figure 1C. Schematic of the chloroplast expression constructs (with nuclear intron) when integrated into the chloroplast genome.** The v206 chloroplast transformation construct is depicted in its integrated form. This construct is designed to express the Ha-HpAce1236-189 hairpin from the Prrn promoter. The backbone is a derivative of the standard chloroplast transformation vector, pPRV323Clox. The integration site in the host chloroplast genome is precise, being defined by the included recombination regions at trnI / trnA. The primers used for subsequent cDNA synthesis steps (301r, 216f/r, 276r, 280r, and oligo dT) and RT-PCR detection steps (217f/r + 216 f/r, and 25f + 216 f/r) are depicted with the expected product sizes (e=..) indicated for the latter sets. Shown is the expected RNA product produced. In this case the included nuclear / cytoplasmic intron will not be spliced as the standard splicing machinery does not operate in the chloroplast. In the additional absence of the RNAi processing machinery, the product remaining will include a substantial portion or all of the sequence as originally included to cover the intended target by the designed hairpin. In this case there may be additional longer RNA species formed if transcription can continue ("read-through") from native upstream transcriptional units, as known to commonly occur for many chloroplast transcripts.

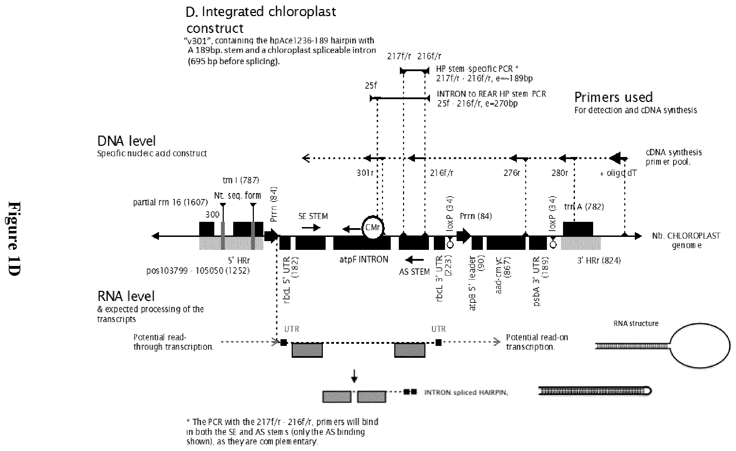

**Figure 1D. Schematic of the chloroplast expression constructs (with chloroplastic intron) when integrated into the chloroplast genome.** The v301 chloroplast transformation construct is depicted in its integrated form. This construct is designed to express the Ha-HpAce1236-189 hairpin from the Prrn promoter. The backbone is a derivative of the standard chloroplast transformation vector, pPRV323Clox. The integration site in the host chloroplast genome is precise, being defined by the included recombination regions at trnI / trnA. The primers used for subsequent cDNA synthesis steps (301r, 216f/r, 276r, 280r, and oligo dT) and RT-PCR detection steps (217f/r + 216 f/r, and 25f + 216 f/r) are depicted with the expected product sizes (e=..) indicated for the latter sets. Shown is the expected RNA product and structure produced. In this case the included chloroplastic intron will be spliced out in the chloroplast. In the absence of the RNAi processing machinery, the product remaining will include a substantial portion or all of the sequence as originally included to cover the intended target by the designed hairpin but not the intron sequence. As a result of the splicing the RNA structure will likely form a rod-shaped RNA duplex with a small loop at one end. In this case there may be additional longer RNA species formed if transcription can continue ("read-through") from native upstream transcriptional units, as known to commonly occur for many chloroplast transcripts.

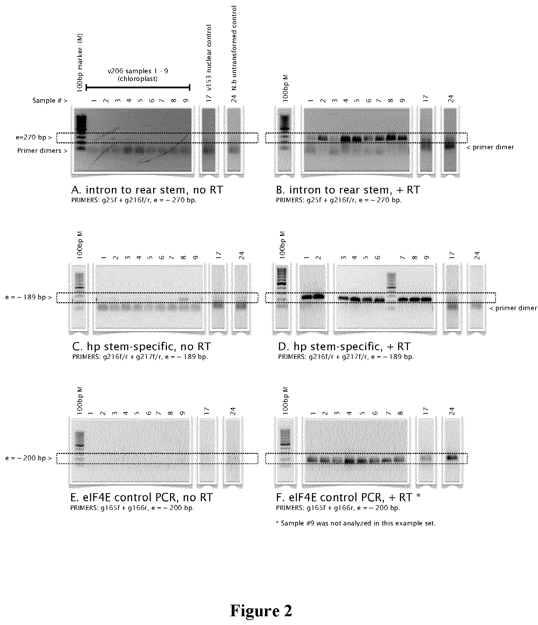

**Figure 2. RT-PCR of RNA extracts from transformants demonstrating expression of transgenes.** Shown are the results from 6 different reverse transcription (RT) assays using RNA samples extracted from *Nicotiana benthamiana* chloroplast transformed plant tissue (v206 samples #1 - 9), *N. benthamiana* nuclear transformed tissue (v153 nuclear control sample #17), and untransformed *N. benthamiana* tissue (untransformed control sample #24). Each sample was amplified with 3 different primer sets, using 2 different template conditions; the extracted RNA before cDNA synthesis as the template (to test for the possibility of DNA contamination in the samples), and the corresponding cDNA sample sets. The expected product sizes are indicated as e=.. The included maker is an MBI Fermentas 100 bp marker, with bands at 100 bp intervals from 100 bp up to 1 kb. **(A-B)** The first PCR primer set was designed to amplify across the intron and rear stem junction to test for the presence of the large RNA species containing both the intron loop at the anti-sense (AS) stem (primers g25f and g216f/r). The boxed region indicates the area that a product would appear if there was specific amplification. **(C-D)** The second set was internal to the dsRNA stem region, but binding at the extremities of the stem to test for the presence of the full length sense (SE) or anti-sense (AS) stem independent of the loop sequence (primers g216f/r and g217f/r). **(E-F)** The third set was a control reaction to test for the presence of the ubiquitous house-keeping sequence for eIF4E (primers g165f and g166r).
Chloroplast sample #9 was not analyzed in this set. It was, however, already confirmed to be positive in the earlier PCR assays.

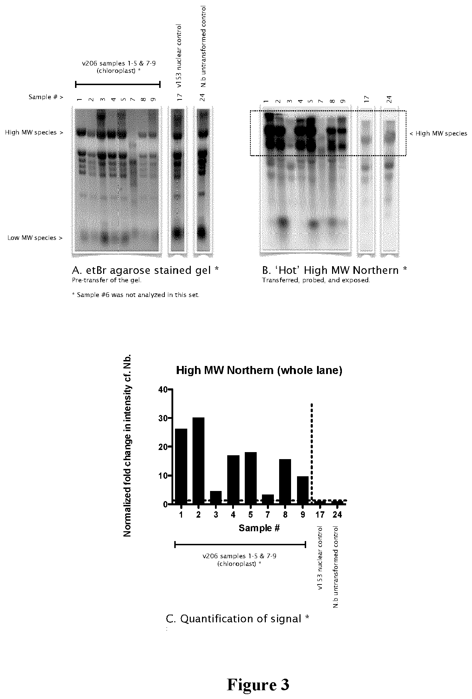

**Figure 3. Northern-based quantification of dsRNA in chloroplast transformants and controls.**
The chloroplast transformation samples were subjected to high molecular weight (MW) or 'large RNA' Northern analysis. (A) RNA extractions were re-prepared for each of the samples #1-9, 17 and 24 (each sample was resuspended in formamide). Approximately 10μl of each sample + 6μl of formamide RNA loading dye was loaded per lane (except for sample 7, where only 4 μl of sample was available) onto a 1.4 % TBE agarose gel and run at 100 V until the 2 lower dye fronts were suitably separated. The gel was stained with ethidium bromide (EtBr) and visualized to gauge relative loading amounts. **(B)** The gel was capillary transferred overnight onto Hybond N+ (Amersham), and the samples were UV cross-linked to the membrane. The membrane was pre-hybridized and hybridized in with PerfectHyb at 65 °C. The probe (total length of 538 nt with ~ 357 nt contiguously matched to HaAce) was a partial length SE strand UTPp32 riboprobe run off SP6 from a reverse inserted HaAce1236 pGEM vector (v158). The membrane was exposed for 1 hour. **(C)** The RNA amounts were quantified using ImageJ, and were normalised to the amounts loaded (using the EtBr image). The relative expression levels were calculated as normalised fold changes in band intensity relative to the *N. benthamiana* untransformed control (set as 1).
Sample #6 was omitted in this example set as there was insufficient material remaining for additional RNA extractions.

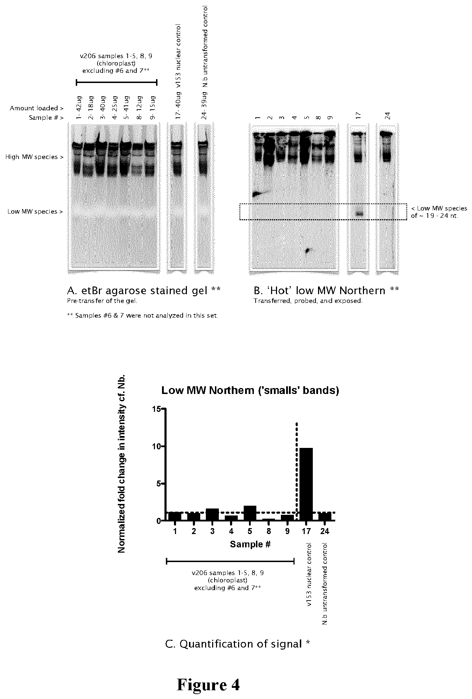

**Figure 4. Northern-based quantification of siRNA in chloroplast transformants and controls.** The chloroplast transformation samples were subjected to low molecular weight (MW) or 'small RNA' Northern analysis to detect and quantify the relative amounts of siRNA-sized processed products. The same RNA extractions as used in example 4 were used here. (A) Approximately 25 μl of each sample + 25 μl of formamide RNA loading dye was loaded per lane onto a 17 % PAGE gel. This resulted in an estimated range of amount loaded of ~ 18 - 42 ug. Prior to loading, the PAGE gel was pre-run at 150 V / 40 min. Once loaded the gel was run at 150V / ~30 min. + 200 V /~4-5 hours (until the dye front was suitably migrated). The gel was stained with ethidium bromide (EtBr) and visualized to gauge relative loading amounts. (B) The gel was electro-transferred at 45 V / 60 min. onto Hybond N+ (Amersham), and the samples were UV cross-linked to the membrane. The membrane was pre-hybridized and hybridized with PerfectHyb at 42 °C. The probe was prepared the same as described previously, except it was also 'carbonated' by standard methods to fractionate it into ~ 50 nt fragments. The membrane was exposed for 1 hour and 60 hours, with no difference in the detection limit between the 2 time points (the 1 hour time point showed herein). (C) The RNA amounts were quantified using ImageJ and were normalised to the amounts loaded (using the EtBr image). The relative expression levels were calculated as normalised fold changes in band intensity relative to the *N. benthamiana* untransformed control (set as 1).
Samples #6 and 7 were omitted in this example set as there was insufficient material remaining.

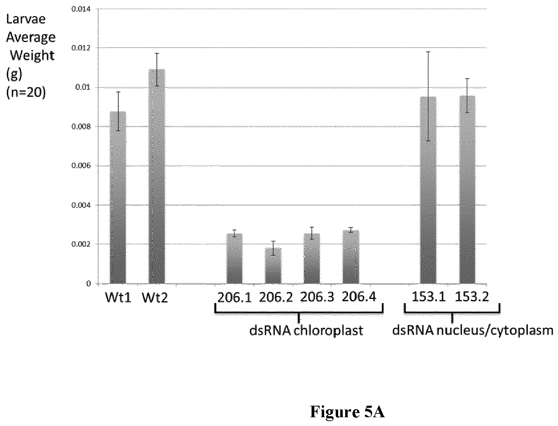

**Figure 5a Growth rate of Helicoverpa armigera larvae on transgenic and non-transgenic seedlings.** Average Helicoverpa larvae weight (n=4-13; original n=20/treatment at start of experiment) after 4 days feeding on washed 6-10 day-old seedlings in plastic petri dishes. The petridishes containing seedling infested with the larvae were held under controlled environmental conditions (28 °C, ~60% Relative Humidity, 16:8 [Light:Dark]). This data shows that in all the four independent lines transformed with v206 (expressing hpRNA in the chloroplast), the larvae were significantly reduced ($p<0.005$ n= 7 - 13) in their growth rate compared to those fed on wild type N.benthamiana seedlings. The growth rate of larvae fed on two lines transformed with v153 (expressing hpRNA in the nucleus/cytoplasm) were not significantly different ($p>0.9$; n =4-7) to those fed on wt.

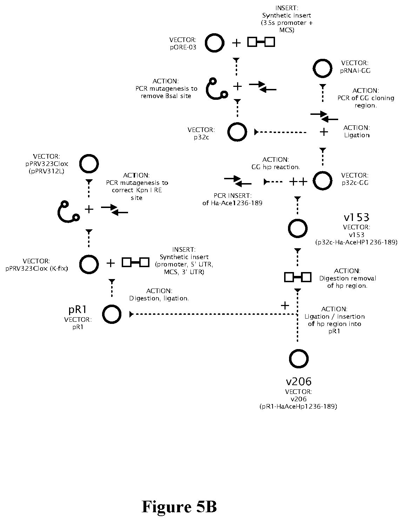

**Figure 5b. Schematic of the relationship path of the vectors referenced herein.** The chloroplast transformation constructs assembled in example 1 were derivatives of several pre-existing vectors, pPRV323Clox, pORE-03, and pRNAi-GG. pPRV323Clox was first modified to remove an erroneous Kpn I RE site. Into this was inserted a chloroplast promoter, 5' UTR, MCS and 3' UTR, to create pR1. pORe-03 was first modified by the insertion of a CaMV 35s promoter plus the removal of a Bsa I site present in the backbone to create p32c. pRNAi-GG was used as a source of the Golden Gate (GG) cloning cassette. The GG cassette was copied into p32c to create p32c-GG. p32c-GG was then used in a GG reaction to create a hairpin construct against the *Helicoverpa armigera* acetylcholinesterase target gene (Ha-Ace1236-189), v153. v153 was the construct used in the nuclear transformation example. This construct was further used to transfer the GG Ha-HpAce1236-189 element into pR1, thus creating v206. v206 was the construct used in the chloroplast transformation example.

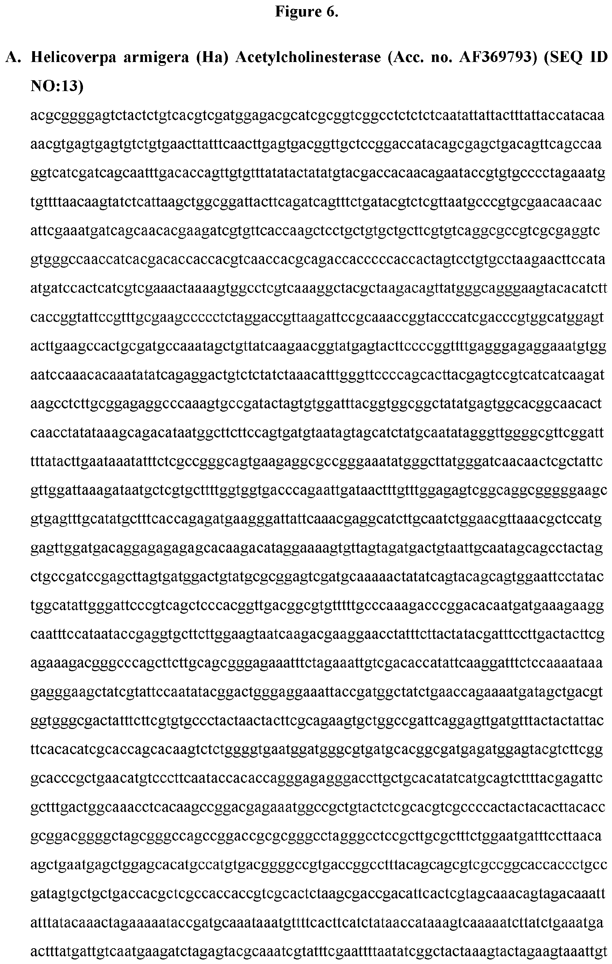

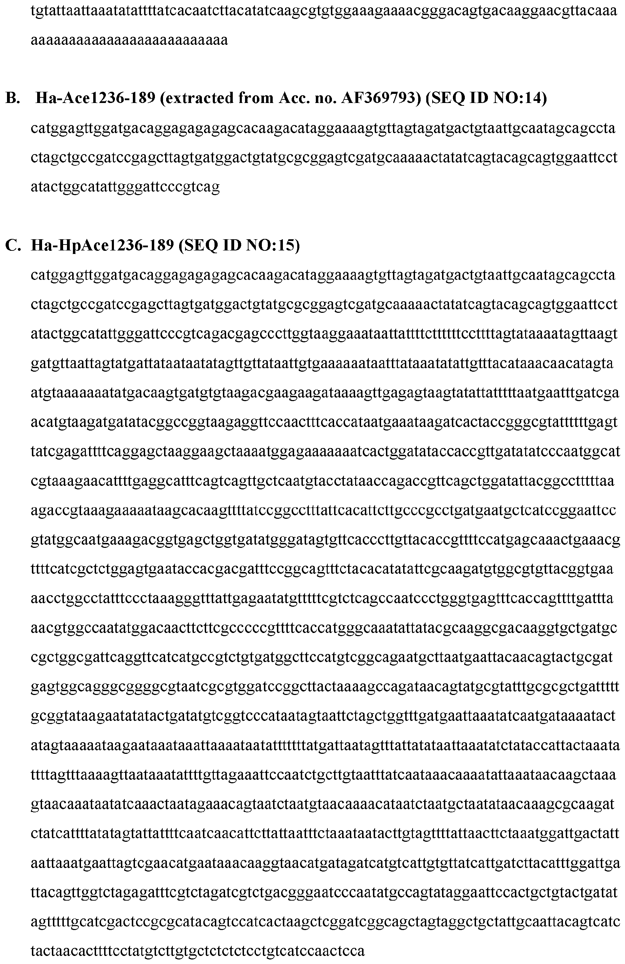

**Figure 6. Exemplary sequences. (A)** The mRNA sequence for the acetylcholinesterase (Ac) gene from *Helicoverpa armigera* (Ha), corresponding to Genbank Accession no: AF369793 (the target gene). **(B)** Ha-Ace1236-189, derived from the sequence of the Ac gene from Ha (the target sequence). (C) Ha-HpAce1236-189, the hairpin of the sequence Ha-Ace1236-189, using the intron spacer regions as present in pRNAi-GG. The sequence shown is the sequence as it appears in the DNA construct (pre-spliced), from the terminal ends of the target sequence.

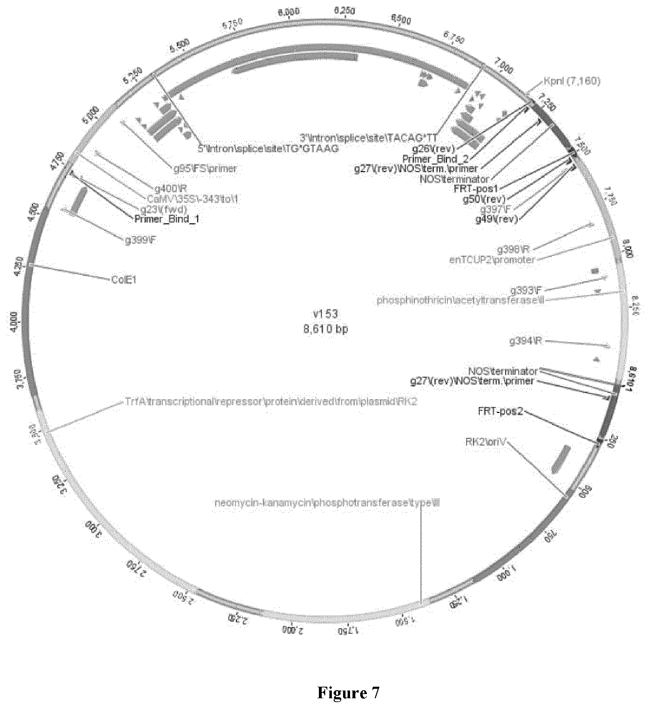

**Figure 7. Plasmid map for v153.**

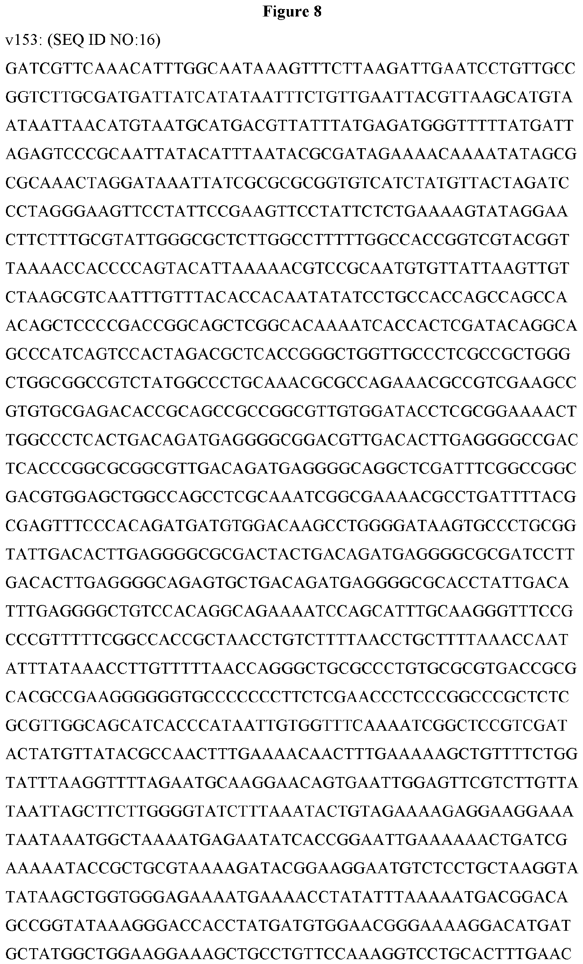

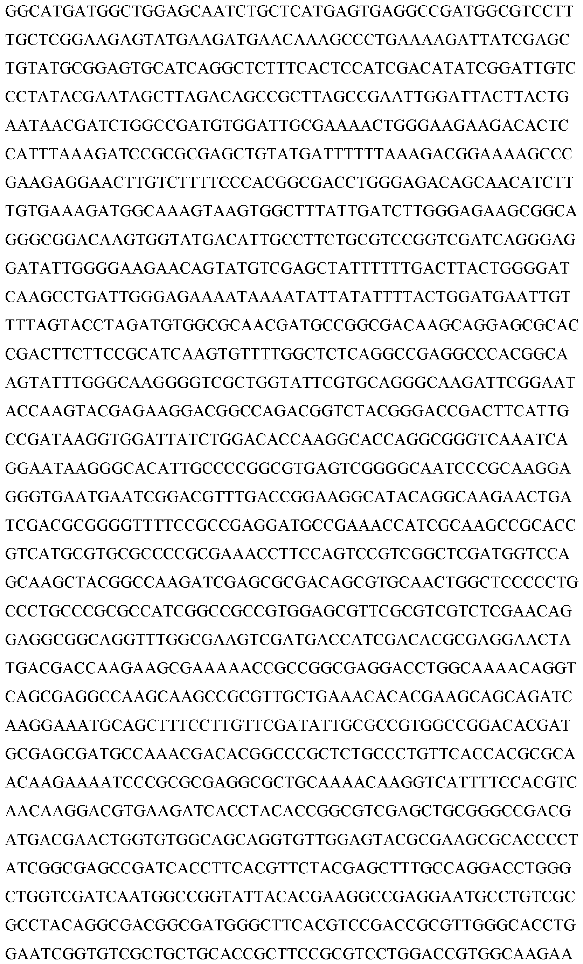

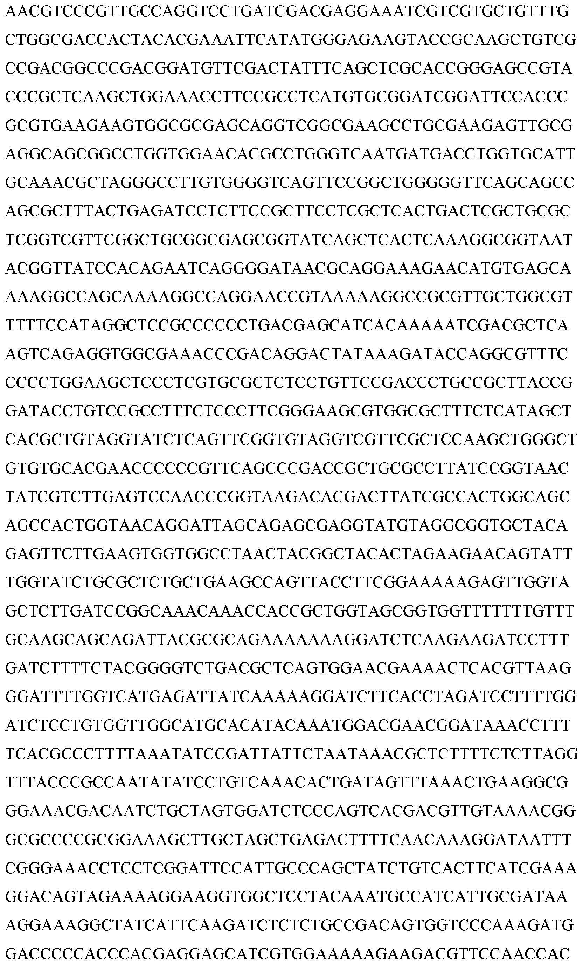

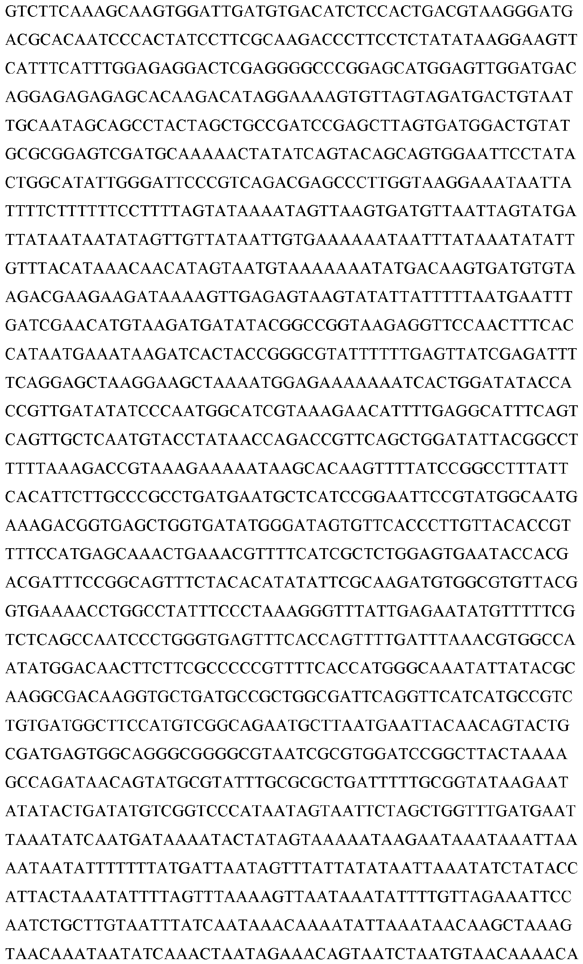

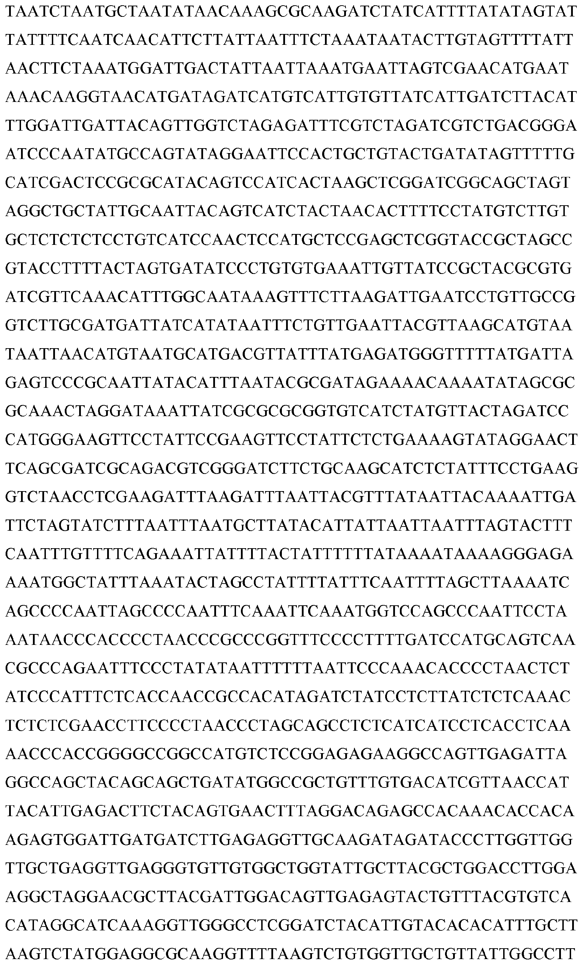

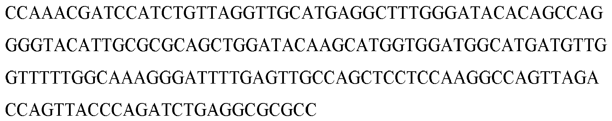

**Figure 8. Nucleotide sequence for v153**

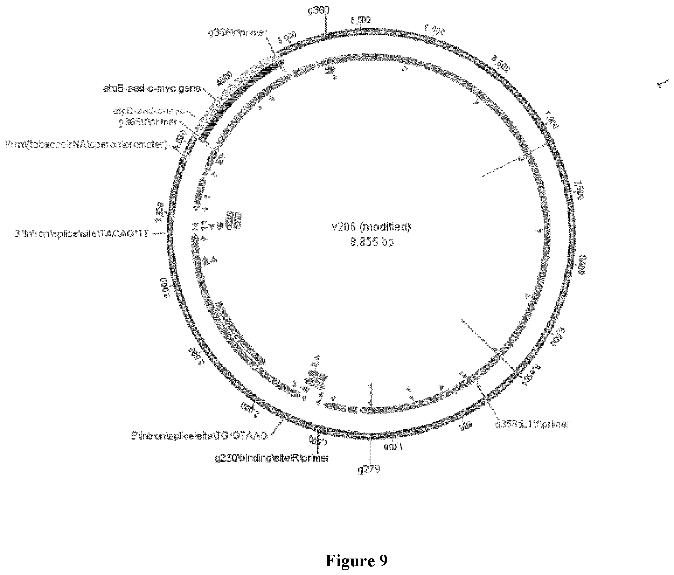

**Figure 9. Plasmid map for v206 modified**

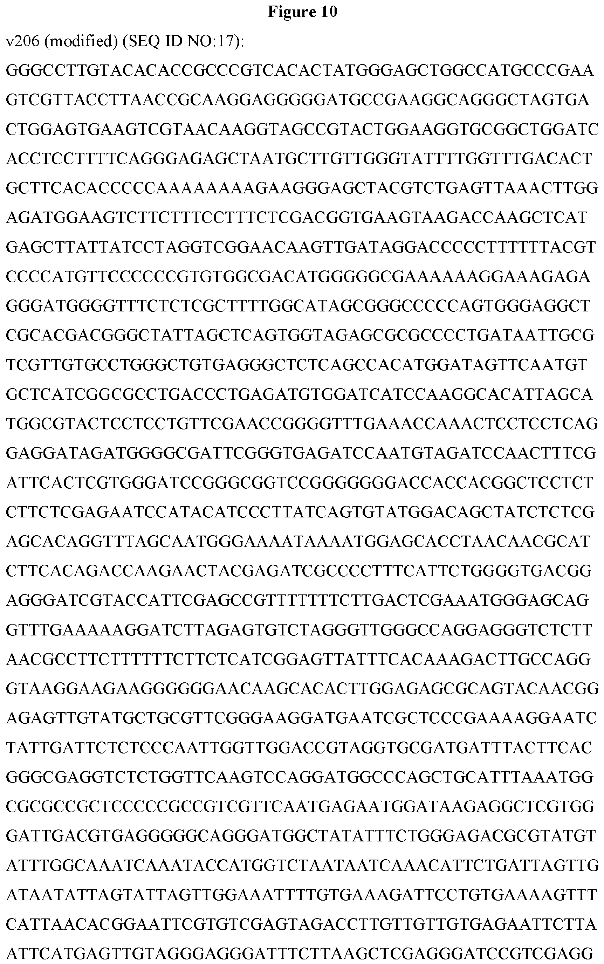

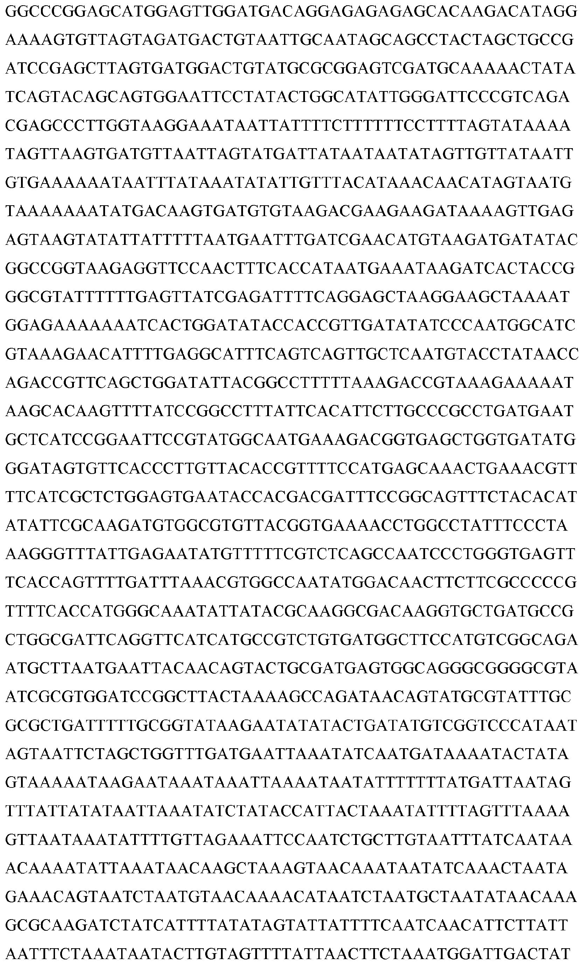

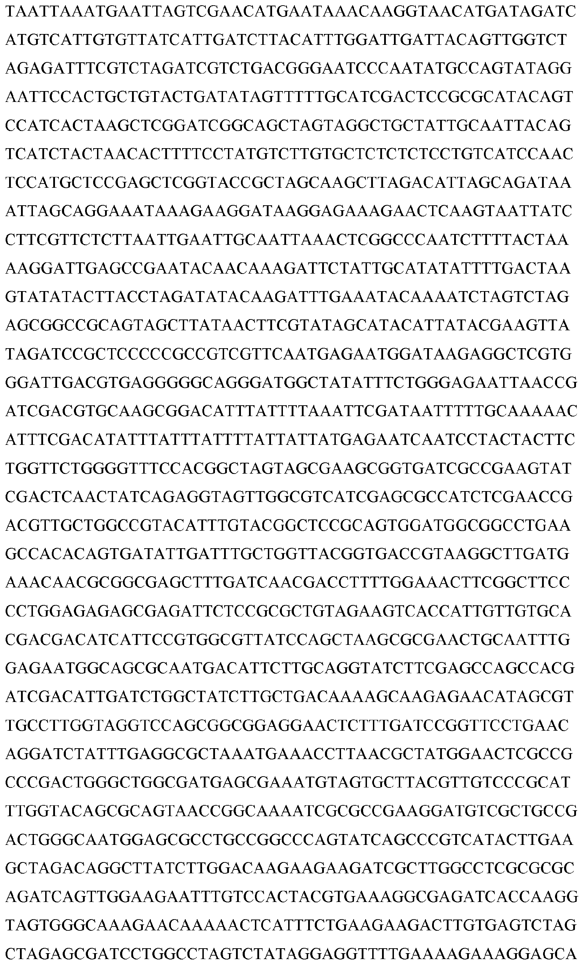

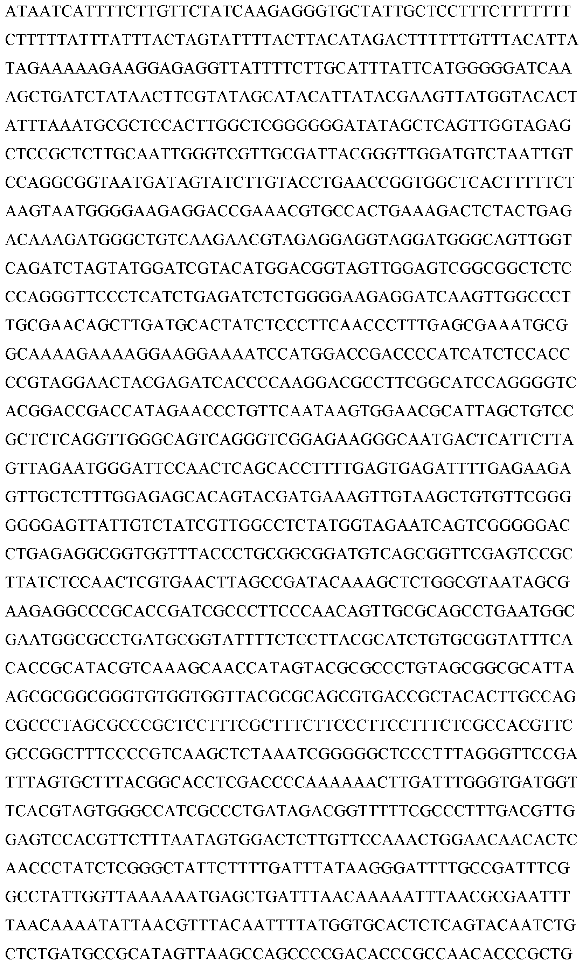

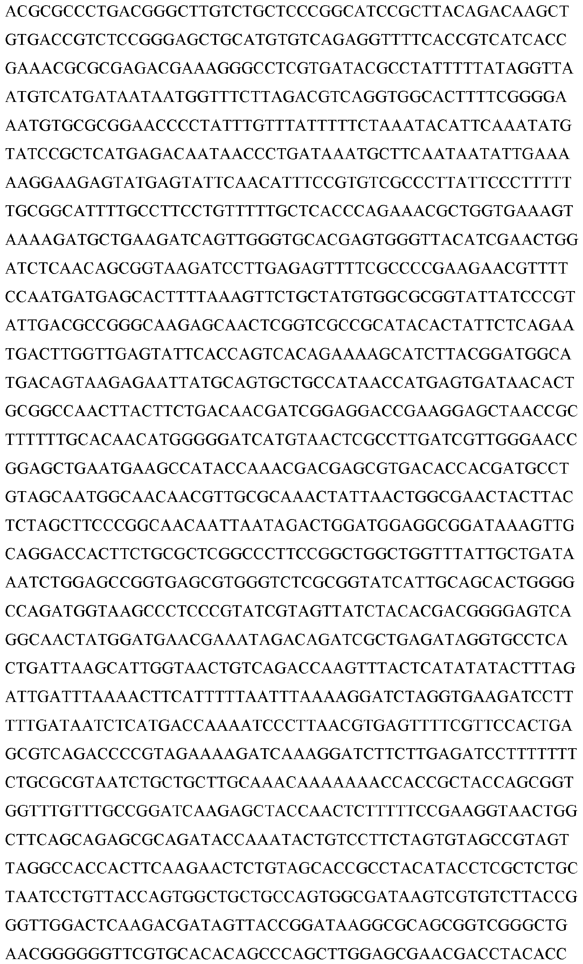

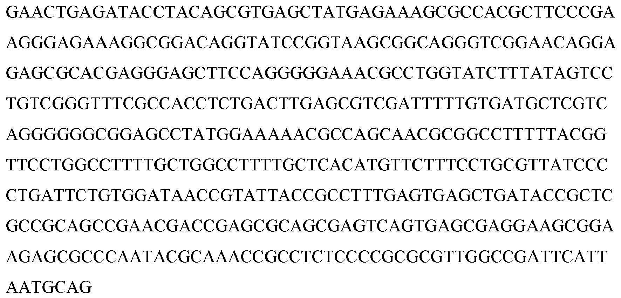

**Figure 10. Nucleotide sequence for v206 modified**

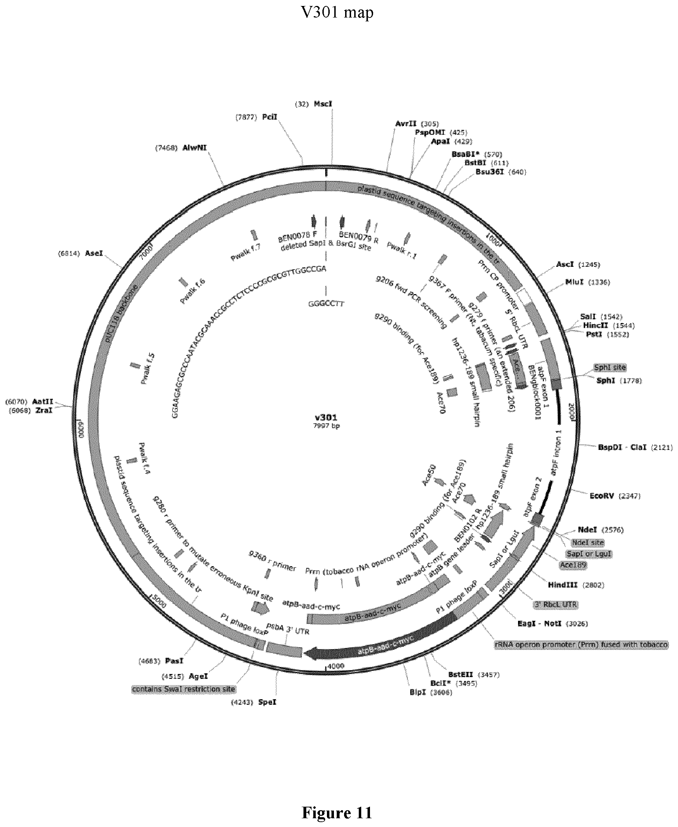

**Figure 11. Plasmid map for v301**

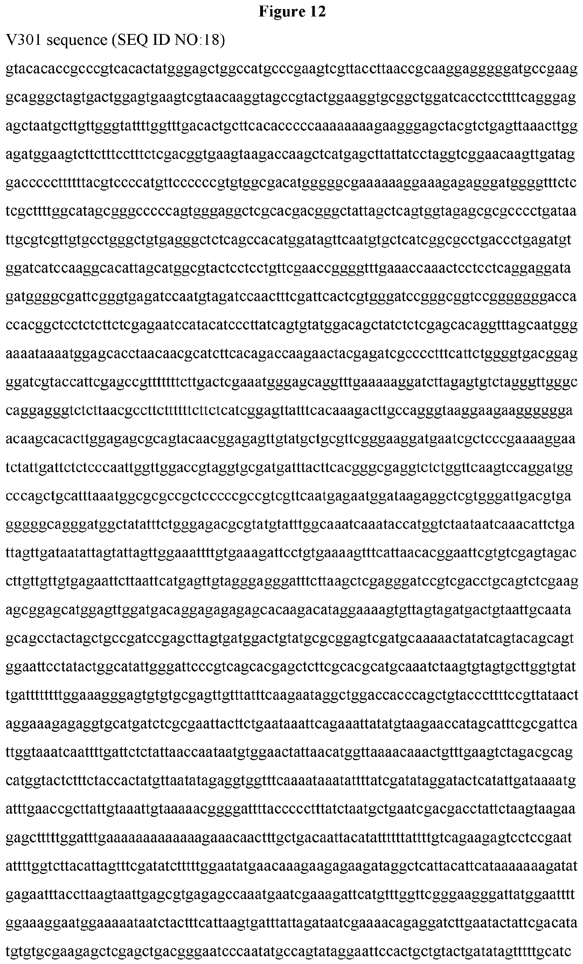

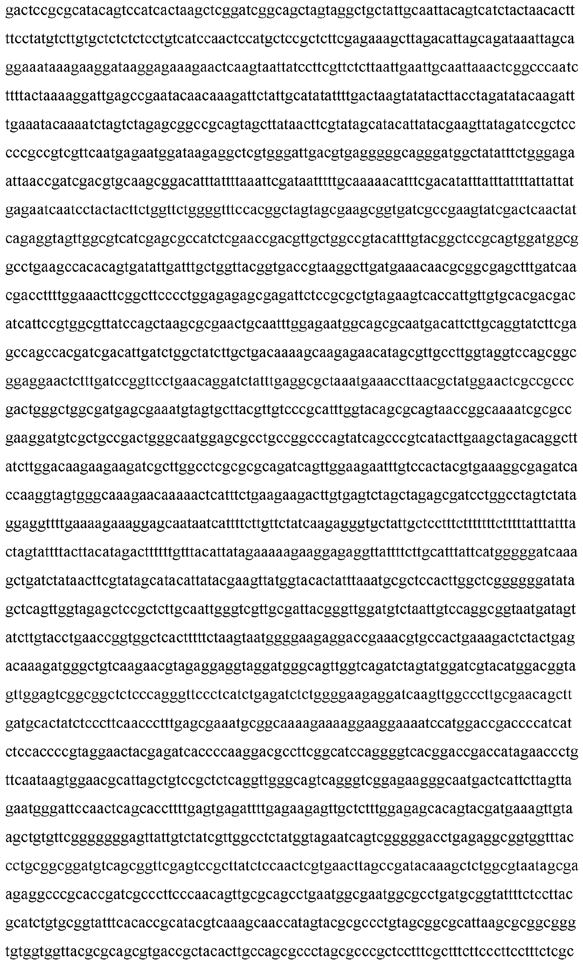

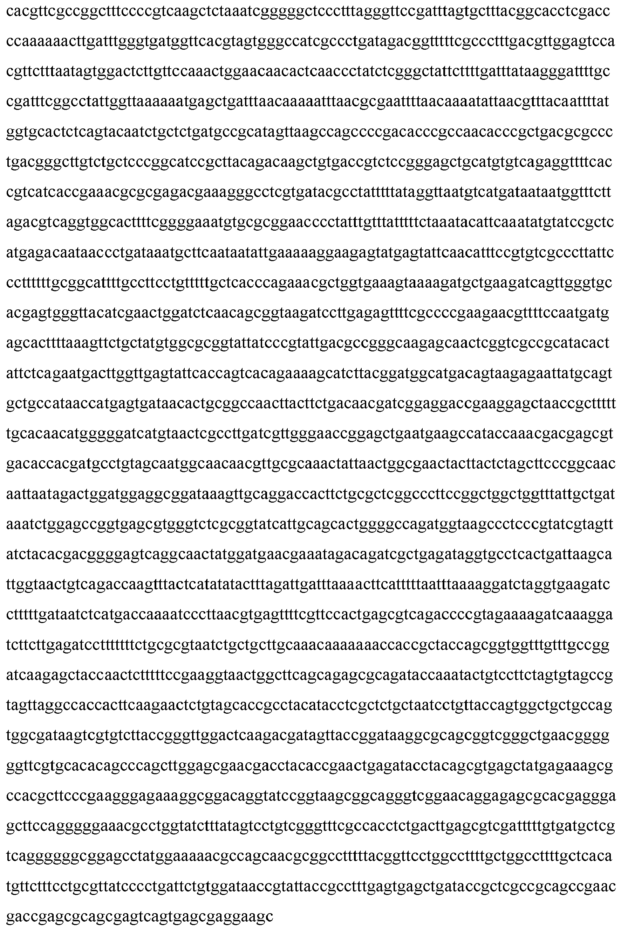

**Figure 12. Nucleotide sequence for v301**

## Detailed description of the embodiments

Transkingdom RNAi has emerged in recent years as an efficient means for inhibiting the growth of pests and pathogens of plants in agriculture. This approach exploits RNA processing pathways present in many eukaryotic organisms whereby exogenous dsRNA is processed into small interfering (siRNA) molecules by cellular RNase III enzymes (for example, Dicer or Dicer-like proteins, such as DCL1, DCL2, DCL3, and DCL4). DICER cleaves long dsRNA molecules into siRNAs which are generally about 19-24 nucleotides in length which are then unwound into two single-stranded RNAs: the passenger strand and the guide strand. The passenger strand is degraded, and the guide strand is incorporated into the RNA-induced silencing complex (RISC). Post-transcriptional gene silencing occurs when the guide strand binds specifically to a complementary sequence of an mRNA molecule and induces cleavage by Argonaut, the catalytic component of the RISC complex.

Transkingdom RNAi exploits this endogenous RNA processing pathway by providing dsRNA molecules to a target organism wherein the dsRNA molecules are not produced in the target organism, but are produced in another organism, for example, an organism that the target organism will consume. Some of the ways to produce dsRNA or hpRNA molecules are depicted in Figure 1A. One example of this approach is the provision of dsRNA molecules in plants wherein the dsRNA molecules are homologous to specific target genes in the target organism. It is understood that when dsRNA produced by the plant is ingested by the pest, the pest RNA interference (RNAi) machinery converts the ingested dsRNA into siRNA, or other RNA form that is capable of inducing essential gene silencing or related events in the pest that impact pest survival. This approach has also been referred to as 'non-cell autonomous RNAi" and distinguishes from the situation where the dsRNA molecule is produced, processed, and exerts its silencing effect, all in the one cell.

One approach to providing dsRNA molecules in a host cell is to introduce a construct into the cell nucleus of the host which, when expressed in the nucleus of the host genome, encodes a dsRNA molecule. It has also been recognised that in providing dsRNA molecules in a host organism, the host organism's own RNA processing machinery may degrade the dsRNA molecules into siRNA. As such, the siRNA that is capable of inducing gene silencing in a pest may be produced in the plant. However, it is now well recognised that in order for transkingdom RNAi to be successful, high levels of dsRNA must be provided to the target organism. Accordingly, recent efforts in transkingdom RNAi methodologies have focused on means for stabilising dsRNA molecules produced in the host cell nucleus, in order to prevent such processing to siRNA. For example, some have suggested the provision of a RNA-binding molecule to prevent degradation. Others have suggested a need to increase the level of dsRNA expression in the host cell nucleus in order to compensate for any loss due to processing into siRNA. Others still have suggested chemical modification of the dsRNA molecules. What is clear is that all efforts at rectifying this situation have been made around modifying existing nuclear-transformation methodologies.

The inventors have taken a different approach to solving the problem of dsRNA degradation, and have surprisingly found that dsRNA produced in a plastid of a vascular plant, for example, in a chloroplast, is not susceptible to the same degradation or processing as dsRNA that is produced in the nucleus of the same cell. Importantly the inventors have found that it is possible to accumulate dsRNA in a vascular plant by selectively transforming the chloroplasts of vascular plants. Further, the inventors have found that chloroplasts generated according to the invention have substantially no siRNA content, which is advantageous for application of the invention to transkingdom RNAi applications. Further, the inventors have found that the dsRNA accumulated in the chloroplasts is otherwise substantially unprocessed by other chloroplast RNA processing pathways. Furthermore, the inventors have found that feeding larvae on transgenic plants expressing hpRNA, in the chloroplast, targeted against an essential larval gene has a strongly inhibitory effect on the growth of such larvae.

The findings of the inventors are surprising because it was hitherto unknown whether plastids degrade dsRNA molecules into siRNAs using Dicer-like enzymes. For example, some have speculated that the presence in plant cells of siRNAs corresponding to chloroplast-replicating viroids is indicative of Dicer-like processing machinery in the chloroplasts of higher plants. The present inventors have found however that the dsRNA molecules produced in plastids of higher plants are not processed into siRNA within the plastid. Accordingly, the inventors have found that the plastid of a vascular plant provides a suitable environment for the production of high levels of dsRNA.

Accordingly, in a first embodiment, the invention provides a plastid of a vascular plant comprising:

- a nucleic acid construct including:
  - a coding region for encoding a double stranded RNA (dsRNA) molecule;
  - a promoter operable in the plastid for enabling production of the dsRNA molecule;
- wherein the dsRNA has a sequence enabling the production of siRNA therefrom.

Generally siRNA is produced from a dsRNA molecule by a cellular RNase III enzyme (for example, Dicer) which cleaves long dsRNA molecules into siRNAs which are generally about 19-24 nucleotides in length. Generally, dsRNA sequences that have a double stranded structure of less than 19 to 24 nucleotides in length do not enable the production of siRNA therefrom.

The dsRNA molecules produced according to the invention therefore retain a substantial proportion of their nascent properties that are generally required for a siRNA to be produced from the dsRNA. In other words, the dsRNA molecule retains properties that enable the RNase III machinery of the target organism to bind to the dsRNA molecule and produce a siRNA molecule therefrom. It is in this context that the dsRNA produced according to the invention has a sequence enabling the production of siRNA therefrom. This is explained further below, although before turning to this, it is useful to discuss the nature of dsRNA further.

Specifically, dsRNA generally includes first and second strands of RNA having sufficient sequence complementarity between the strands to enable the first and second strands to bind to each other by Watson-Crick base pairing to form a *'stem'*. In some embodiments, a stem of dsRNA consists of 100% complementarity between the first and second strands. In some embodiments, there may be one or more mismatches between the first and second strands forming the stem so that the complementarity between strands of the stem may be less than 100%. Generally the level of complementarity between first and second strands of the stem is greater than 80%, preferably 85%, preferably 90%, preferably 95, 96, 97, 98 or 99%. Generally, the mismatches are across a contiguous sequence of no more than about 3 nucleotides, preferably about 2 nucleotides. Preferably a mismatch is limited to between single nucleotides of the first and second strands of the stem at spaced apart locations.

Given above, it will be understood that a stem of dsRNA of defined length may in fact include one or more regions or positions of mismatch. For example, where first and second strands have perfect complementarity across a region of 30 nucleotides but for one or two mismatches within the 30 nucleotide region, the first and second strands would be said to constitute a stem of dsRNA of 30 nucleotides.

A dsRNA may include one or more stems. Where there is more than one stem, these may be arranged in series or clusters to form tandem or overlapping inverted repeats, which form dsRNA structures resembling, for example, a two-stem structure resembling a "hammerhead", "barbell", or "dog bone", or a structure containing 3 or more stems resembling a "cloverleaf", or a structure with a pseudoknot-like shape. Any of these constructs can further include spacer segments found within a double-stranded stem (for example, as a spacer between multiple anti-sense or sense nucleotide sequence segments or as a spacer between a base-pairing anti-sense nucleotide sequence segment and a sense nucleotide sequence segment) or outside of a double-stranded stem (for example, as a loop region of sense or of anti-sense or of unrelated RNA sequence, separating a pair of inverted repeats). In cases where base-pairing anti-sense and sense nucleotide sequence segment are of unequal length, the longer segment can act as a spacer.

The dsRNA may arise from a single strand of RNA having inverted repeat sequences that enable the single strand of RNA to form a stem structure. Alternatively, the dsRNA may arise from 2 or more RNA molecules that align and base pair with each other to form a stem of a dsRNA molecule.

As discussed above, in one embodiment, it is the length of the dsRNA, especially the length of the stem of the dsRNA that enables production of siRNA therefrom. Typically, a stem of dsRNA has a length of at least 30 nucleotides. At lengths that are shorter than 30 nucleotides, there may be limitations in the extent to which RNA polymerase III systems of pest organisms can generate siRNA from dsRNA. In various embodiments, the stem of dsRNA can consist of at least about 50, at least about 75, at least about 100, at least about 150, at least about 200, at least about 250, at least about 300, or even more base pairs. In one preferred embodiment, the dsRNA includes at least about 100 base pairs. In another preferred embodiment, the stem of dsRNA includes at least about 250 base pairs, or even more, for example, 500 or 1000 base pairs.

The dsRNA is encoded by a coding region. The coding region is an exogenous nucleic acid sequence. In some embodiments the coding region may encode a transgene. In some examples, a transgene may be a sequence that encodes one or both strand(s) of a dsRNA molecule that comprises a nucleotide sequence that is complementary to a nucleic acid molecule found in an organism that comprises a target gene sequence. In a further example, a transgene may be a gene sequence (e.g., a herbicide-tolerance gene), a gene encoding a biomarker relevant to a mechanism of action of disease, a pharmaceutically useful compound, or a gene encoding a desirable agricultural trait.

In these and other examples, a transgene may contain regulatory sequences operably linked to a coding sequence of the transgene (e.g., a promoter). Examples are described further below.

In some embodiments, the coding region may encode essentially a single double-stranded RNA and includes multiple serial anti-sense nucleotide sequence segments that are anti-sense to at least one segment of the target gene and multiple serial sense nucleotide sequence segments that are sense to at least one segment of the target gene; the multiple serial anti-sense and multiple serial sense segments can form a single double-stranded RNA stem or multiple double-stranded stems in a serial arrangement (with or without non-base paired spacer DNA separating the multiple double-stranded stems).

In another embodiment, the coding region encodes multiple dsRNA stems of RNA and includes multiple anti-sense nucleotide sequence segments that are anti-sense to at least one segment of the target gene and multiple sense nucleotide sequence segments that are sense to at least one segment of the target gene, and wherein said multiple anti-sense nucleotide sequence segments and the multiple sense nucleotide sequence segments are arranged in a series of double-stranded stems.

In certain embodiments, dsRNA may refer to either or both dsRNA and hpRNA.

The dsRNA molecule encoded by the coding region may have a sequence which enables the production of siRNA molecules which target a particular pest or pathogen of the vascular plant. As such, the nucleotide sequence of the coding region will be complementary to a target gene of a target organism and will not correspond to a nucleotide sequence of the host plant.

Accordingly, in another embodiment there is provided a plastid of a vascular plant, the plastid comprising:

- a nucleic acid construct including:

  - a coding region for encoding a dsRNA molecule;
  - a promoter operable in the plastid for enabling production of the dsRNA molecule;

- wherein the coding region has a nucleotide sequence that is not found in the genome of the plant.

Typically the coding region has a nucleotide sequence of a target gene. Preferred target genes are those genes that are essential to survival of a relevant pest. The pest may be of a plant, animal, bacterial, viral or fungal kingdom. In these embodiments, the dsRNA enables the production of siRNA molecules that either cleave, or repress the translation of, mRNA of the target gene. Examples of pests and target genes are as follows:

- coleopteran pests, including, for example *Diabrotica virgifera virgifera* LeConte (western corn rootworm, "WCR"), *Diabrotica barberi* Smith and Lawrence (northern corn rootworm, "NCR"), *Diabrotica undecimpunctata howardi* Barber (southern corn rootworm, "SCR"), *Diabrotica virgifera zeae* Krysan and Smith (Mexican corn rootworm, "MCR"), *Diabrotica balteata* LeConte; *Diabrotica undecimpunctata tenella*, *D. speciosa* Germar, and *Diabrotica undecimpunctata undecimpunctata* Mannerheim;
- hemipteran pests, including, for example, *Euschistus heros* (Fabr.) (Neotropical Brown Stink Bug, "BSB"), *Nezara viridula* (L.) (Southern Green Stink Bug), *Piezodorus guildinii* (Westwood) (Red-banded Stink Bug), *Halyomorpha halys* (Stål) (Brown Marmorated Stink Bug), *Chinavia hilare* (Say) (Green Stink Bug), *Euschistus servus* (Say) (Brown Stink Bug), *Dichelops melacanthus* (Dallas), *Dichelops furcatus* (F.), *Edessa meditabunda* (F.), *Thyanta perditor* (F.) (Neotropical Red Shouldered Stink Bug), *Chinavia marginatum* (Palisot de Beauvois), *Horcias nobilellus* (Berg) (Cotton Bug), *Taedia stigmosa* (Berg), *Dysdercus peruvianus* (Guérin-Méneville), *Neomegalotomus parvus* (Westwood), *Leptoglossus zonatus* (Dallas), *Niesthrea sidae* (F.), *Lygus hesperus* (Knight) (Western Tarnished Plant Bug), and *Lygus lineolaris* (Palisot de Beauvois);
- lepidopteran pests, including, for example, *Ostrinia nubilalis* (European corn borer), *Spodoptera frugiperda* (Fall army worm), *Spodoptera exigua* (Beet armyworm), *Spodoptera ornithogalli* (Yellowstriped armyworm), *Helicoverpa zea* (Corn earworm), *Agrotis ipsilon* (Black cutworm), *Helicoverpa armigera* (Cotton bollworm), *Diatraea saccharalis* (Sugar cane borer), *Diatraea grandiosella* (Southwestern corn borer), *Elasmopalpus lignosellus* (Lesser cornstalk borer), *Papaipema nebris* (Stalk borer), *Pectinophora gossypiella* (Pink bollworm), *Plathypena scabra* (Green cloverworm), *Colias eurytheme* (Alfalfa caterpillar), *Anticarsia gemmatalis* (Velvet bean caterpillar), *Pseudoplusia includens* (Soybean looper), *Plutella xylostella* (Diamondback moth), *Agromyza parvicornis* (Corn blot leafminer), *Achoroia grisella, Acleris gloverana, Acleris variana, Adoxophyes orana, Alabama argillacea, Alsophila pometaria, Amyelois transitella, Anagasta kuehniella, Anarsia lineatella, Anisota senatoria, Antheraea pernyi, Archips* sp., *Argyrotaenia* sp., *Athetis mindara, Bombyx mori, Bucculatrix thurberiella, Cadra cautella, Choristoneura* sp., *Cochylls hospes, Corcyra cephalonica, Cydia latiferreanus, Cydia pomonella, Datana integerrima, Dendrolimus sibericus, Desmia feneralis, Diaphania hyalinata, Diaphania nitidalis, Ennomos subsignaria, Eoreuma loftini, Esphestia elutella, Erannis tilaria, Estigmene acrea, Eulia salubricola, Eupocoellia ambiguella, Eupoecilia ambiguella, Euproctis chrysorrhoea, Euxoa messoria, Galleria mellonella, Grapholita molesta, Harrisina americana, Helicoverpa subflexa, Hemileuca oliviae, Homoeosoma electellum, Hyphantia cunea, Keiferia lycopersicella, Lambdina fiscellaria fiscellaria, Lambdina fiscellaria lugubrosa, Leucoma salicis, Lobesia botrana, Loxostege sticticalis, Lymantria dispar, Macalla thyrisalis, Malacosoma* sp., *Mamestra brassicae, Mamestra configurata, Manduca quinquemaculata, Manduca sexta, Maruca testulalis, Melanchra picta, Operophtera brumata, Orgyia* sp., *Paleacrita vernata, Papilio cresphontes, Phryganidia californica, Phyllonorycter blancardella, Pieris napi, Pieris rapae, Platynota flouendana, Platynota stultana, Platyptilia carduidactyla, Plodia interpunctella, Pontia protodice, Pseudaletia unipuncta, Sabulodes aegrotata, Schizura concinna, Sitotroga cerealella, Spilonta ocellana, Thaurnstopoea pityocampa, Ensola bisselliella, Trichoplusia hi, Udea rubigalis, Xylomyges curiails, Yponomeuta padella,* and *Heliothis virescens* (Tobacco budworm); and
- nematode pests, including, for example, *Aphelenchoides* spp., *Belonolaimus* spp., *Criconemella* spp., *Ditylenchus* spp., *Globodera* spp., *Heterodera* spp., *Hirschmanniella* spp., *Hoplolaimus* spp., *Meloidogyne* spp., *Pratylenchus* spp., and *Radopholus* spp., a non-exhaustive list of particular sp. includes, but is not limited to, *Dirofilaria immitis, Globodera pallida, Heterodera glycines, Heterodera zeae, Meloidogyne incognita, Meloidogyne javanica, Onchocerca volvulus, Pratylenchus penetrans, Radopholus similis,* and *Rotylenchulus reniformis.*

In particular examples, exemplary nucleic acid molecules are disclosed that may be homologous to at least a portion of one or more native nucleic acid sequences in a coleopteran, hemipteran, and/or lepidopteran pest, or an organism other than an insect pest (e.g., a plant-parasitic nematode), including but not limited to Caf1-180 (U.S. Patent Application Publication No. 2012/0174258), VatpaseC (U.S. Patent Application Publication No. 2012/0174259), Rho1 (U.S. Patent Application Publication No. 2012/0174260), VatpaseH (U.S. Patent Application Publication No.

2012/0198586), PPI-87B (U.S. Patent Application Publication No. 2013/0091600), RPA70 (U.S. Patent Application Publication No. 2013/0091601), RPS6 (U.S. Patent Application Publication No. 2013/0097730), ROP (U.S. Patent Application Publication No. 14/577811), RNAPII (U.S. Patent Application Publication No. 14/577854), and/or as disclosed in U.S. Patent Application Publication Nos. 2012/0164205, 2011/0054007, and 2009/0265818, U.S. Patent No. 6,326,193, European Patent Application Nos. EP1210875 and EP2633048, and PCT International Patent Application Nos. WO2011153418, WO2007080126, WO2012143542, WO2005110068, and WO2006047495; or a transgenic event from which is transcribed a dsRNA molecule targeting a gene in a coleopteran, hemipteran, and/or lepidopteran pest, or an organism other than an insect pest (e.g., a plant-parasitic nematode, such as for example as disclosed in U.S. Patent Application Publication Nos. 2005/0188438, 2006/0080749, 2009/0300796, and PCT International Patent Application Nos. WO2003052110, WO2004066183, WO2004005485, WO2005019408, WO2006046148, WO2007087153, WO2007095496, and WO2009133126).

In certain embodiments, the nucleic acid construct may include more than one coding region for encoding dsRNA. For example the nucleic acid construct may include a first coding region for encoding a first dsRNA for a first insect target gene, and a further coding region for encoding a further dsRNA for an insect target gene.

In further embodiments, a plastid or chloroplast according to the invention may include more than one type of nucleic acid construct. For example, a plastid or chloroplast according to the invention may include:

- a first nucleic acid construct including a first coding region for encoding a first dsRNA for a first insect target gene;

- a further nucleic acid construct including a further coding region for encoding a further dsRNA for an insect target gene.

The first and further coding regions and the dsRNA that are produced from each may be the same or different.

Further, the first and further coding regions may be under the control of the same promoter or regulatory sequence, or different promoters or regulatory sequences. In one embodiment, the first coding region may be under the control of a constitutive promoter and the further coding region may be under the control of an inducible promoter.

In other embodiments, both first and further coding regions are under control of constitutive promoters, or under control of inducible promoters. Examples of particular promoters useful for expression of dsRNA in chloroplasts and plastids are described below.

The minimum degree of recognition expected to have a silencing effect would be between a sequence in the stem of a hairpin and the sequence in the target mRNA that share at least one block of greater than 16 or more nucleotides of perfect identity in a 19 nucleotide contiguous sequence block.

In other embodiments, the target gene is a gene for which gene silencing or RNAi is required. Such a gene may be one that is expressed in pathology of disease. In another example, the gene may be one that is expressed in an industrial process.

It is known that when dsRNA molecules are provided in the cytoplasm of a vascular plant (whether via the expression of DNA constructs in the nucleus or otherwise), the dsRNA molecules are recognised by Dicer enzymes in the cytoplasm and are processed into siRNAs. However, the inventors have found that dsRNA provided in the plastids of these same plants are not subject to the same processing. Accordingly, the dsRNA molecules produced according to the present invention are not processed into siRNA and the plastids do not comprise siRNA molecules derived from the dsRNA molecule sequence.

Accordingly, in another embodiment there is provided a plastid of a vascular plant, the plastid comprising:

- a nucleic acid construct including:
  - a coding region for encoding a double stranded RNA (dsRNA) molecule;
  - a promoter operable in the plastid for enabling production of the dsRNA molecule;

- wherein the plastid does not substantially comprise siRNA molecules including a sequence of the dsRNA.

Generally the plastid does not contain siRNA molecules including a sequence of the dsRNA, although it will be understood that in some embodiments the plastid may contain degradation products arising from RNA breakdown or catabolism, and may otherwise contain certain siRNA in amounts that have no bearing on the production or accumulation of dsRNA in the plastid.

It has been speculated that the plastids of vascular plants may comprise homologs of the known cytoplasmic RNase III ("Dicer") enzymes. Furthermore, it is known that chloroplast RNAs are often subject to significant RNA editing. Accordingly, it was hitherto unknown whether dsRNA provided in a plastid of a vascular plant would retain substantially all of the nucleotide sequence of a target gene which is encoded by a DNA construct. The inventors have surprisingly found that the higher plant chloroplast does not substantially edit the dsRNA transcribed from a coding region for a target gene, nor is there substantial truncation or splicing of the dsRNA transcribed from the coding region.

Accordingly, in another embodiment there is provided a plastid of a vascular plant, the plastid comprising:

- a nucleic acid construct including:
  - a coding region for encoding a double stranded RNA (dsRNA) molecule;
  - a promoter operable in the plastid for enabling production of the dsRNA molecule;
- dsRNA
- wherein the dsRNA contains substantially all of the nucleotide sequence encoded by the coding region.

Generally the dsRNA is unprocessed in the sense that it has not undergone post transcriptional modification that would remove the regions of the dsRNA necessary for formation of a stem structure. In some embodiments, the dsRNA has been modified to remove 5' or 3' untranslated regions, or poly A tail. In some embodiments, the dsRNA has been modified to remove regions of non-complementarity such as those that are observed to form a loop structure, as in a RNA hairpin.

The present invention has particular utility in providing high levels of dsRNA molecules for use in transkingdom RNAi applications. As previously discussed, it is now acknowledged that in order for transkingdom RNAi to be successful, large amounts of dsRNA must be provided to the target organism. One particularly important finding of the invention is that the dsRNA produced in the plastid cannot pass from the plastid into the cytosol. This is important in two respects. First, by remaining in the plastid, the dsRNA is able to accumulate into large amounts in the plastid. Second, by being prevented from interaction with the cytosol by the plastid, the dsRNA is unable to induce cytosolic RNAi that might ultimately impact on the dsRNA produced in the plastid, or otherwise impact plant growth and production. Indeed, the plastid enables dsRNA production in circumstances that would otherwise induce RNAi.

Therefore, according to the invention, the dsRNA will be understood to accumulate in the plastid of the vascular plant and to be retained by the plastid so that the dsRNA is prevented from egress from the plastid into the cytosol, and therefore prevented from inducing RNAi in the cytosol.

In the above described embodiments, the plastid may be a chloroplast, a chromoplast, a leucoplast, or a proplastid. Typically the plastid is a chloroplast. A mitochondrion is a non plastid organelle that may also comprise a nucleic acid construct according to the invention.

In further embodiments there is provided a dsRNA when produced by a plastid or chloroplast as described above.

**B. Nucleic acid constructs**

In one embodiment, the invention provides a nucleic acid construct for transformation of a vascular plant, comprising:

- a coding region for encoding a double stranded RNA (dsRNA) molecule;
- a promoter operable in a plastid for enabling production of the dsRNA molecule in a plastid;
- one or more integration sites selective for integration of the construct into a plastid genome of a vascular plant;
  - wherein the dsRNA has a sequence enabling the production of siRNA therefrom.

Specifically, the construct can be transformed into a plastid of the plant. The construct comprises a coding region that, upon expression to RNA forms or produces a dsRNA molecule which can be used to inhibit target gene expression in another organism. In order to initiate or enhance expression, such nucleic acid constructs may comprise one or more regulatory sequences, which regulatory sequences may be operably linked to the nucleic acid sequence capable of being expressed as a dsRNA.

In specific embodiments, a nucleic acid construct of the invention may comprise a nucleic acid sequence encoding a dsRNA molecule. In many embodiments, a transcribed dsRNA molecule may be provided in a stabilized form; e.g., as a hairpin and stem and loop structure.

A nucleic acid construct encoding a dsRNA molecule may comprise at least two nucleotide sequences with a coding sequence arranged such that one nucleotide sequence is in a sense orientation, and the other nucleotide sequence is in an antisense orientation, relative to at least one promoter, wherein the sense nucleotide sequence and the antisense nucleotide sequence are linked or connected by a spacer sequence of from about five (~5) to about one thousand (1000) nucleotides. The spacer sequence may form a loop between the sense and antisense sequences. The sense nucleotide sequence or the antisense nucleotide sequence may be substantially homologous to the nucleotide sequence of a target gene or fragment thereof. In some embodiments, however, a nucleic acid construct molecule may encode a dsRNA molecule without a spacer sequence. In embodiments, a sense coding sequence and an antisense coding sequence may be different lengths.

Sequences identified as being useful to facilitate transkingdom RNAi may be readily incorporated into expressed dsRNA molecules through the creation of appropriate expression cassettes in a nucleic acid construct of the invention. For example, such sequences may be expressed as a hairpin with stem and loop structure by taking a first segment corresponding to a target gene sequence linking this sequence to a second segment spacer region that is not homologous or complementary to the first segment; and linking this to a third segment, wherein at least a portion of the third segment is substantially complementary to the first segment. Such a construct forms a stem and loop structure by hybridization of the first segment with the third segment, wherein the loop structure forms comprising the second segment. See, e.g., U.S. Patent Publication Nos. US 2002/0048814 and US 2003/0018993; and International PCT Publication Nos. WO94/01550 and WO98/05770.

In one preferred embodiment, a spacer sequence referred to above may have the sequence of an intron, especially the sequence of an intron of a gene of a chloroplast or plastid genome. Examples are shown in Table 1.

*Table 1.* Introns present in protein-coding genes of land plant chloroplasts.

| Gene | Species | Intron size (bp) | Reference |
|---|---|---|---|
| *atp*F | *Triticum aestivum* | 823 | Bird et al. (1985) |
| *atp*F | *Spinacea oleracea* | 764 | Hennig and Herrmann (1986) |
| *atp*F | *Pisum sativum* | 690 | Hudson et al. (1987) |
| *atp*F | *Nicotiana tabacum* | 695 | Shinozaki et al. (1986a) |
| *atp*F | *Marchantia polymorpha* | 588 | Ohyama et al. (1986) |
| *ndh*A | *Nicotiana tabacum* | 1,242 | Shinozaki et al. (1986c) |
| *ndh*A | *Marchantia polymorpha* | 713 | Ohyama et al. (1986) |
| *ndh*B | *Nicotiana tabacum* | 757 | Shinozaki et al. (1986c) |
| *ndh*B | *Marchantia polymorpha* | 537 | Ohyama et al. (1986) |
| *pet*B | *Triticum aestivum* | 744 | S. Hird, unpublished |
| *pet*B | *Pisum sativum* | 810 | C. Eccles, unpublished |
| *pet*B | *Nicotiana tabacum* | 759 | Shinozaki et al. (1986c) |
| *pet*B | *Spinacea oleracea* | 777 | Westhoff et al. (1986) |
| *pet*B | *Marchantia polymorpha* | 495 | Fukuzawa et al. (1987) |
| *pet*D | *Triticum aestivum* | 746 | S. Hird, unpublished |
| *pet*D | *Pisum sativum* | 715 | C. Eccles, unpublished |
| *pet*D | *Nicotiana tabacum* | 742 | Shinozaki et al. (1986c) |
| *pet*D | *Spinacea oleracea* | 743 | Heinemeyer et al. (1984) |
| *pet*D | *Marchantia polymorpha* | 493 | Fukuzawa et al. (1987) |
| *rpl*16 | *Nicotiana tabacum* | 1,020 | Shinozaki et al. (1986c) |
| *rpl*16 | *Marchantia polymorpha* | 536 | Ohyama et al. (1986) |
| *rpl*2 | *Nicotiana tabacum* | 666 | Shinozaki et al. (1986c) |
| *rpl*2 | *Nicotiana debneyi* | 666 | Zurawski et al. (1984) |
| *rpl*2 | *Marchantia polymorpha* | 545 | Ohyama et al. (1986) |
| *rps*12 | *Nicotiana tabacum* | | Fromm et al. (1986) |
| exons I-II | | trans-split | Torazawa et al. (1986) |
| exons II-III | | 540 | |
| *rps*12 | *Marchantia polymorpha* | | Fukuzawa et al. (1986) |
| exons I-II | | trans-split | |
| exons II-III | | 499 | |
| *rps*16 | *Nicotiana tabacum* | 860 | Shinozaki et al. (1986b) |
| *rpo*C | *Marchantia polymorpha* | 597 | Ohyama et al. (1986) |

From Introns in chloroplast protein-coding genes of land plants. AINE L. PLANT & JOHN C. GRAY Photosynthesis Research 16:23-39 (1988)

Chloroplast or plastid introns that are processed by the chloroplast or plastid may have particular advantage in certain embodiments of the invention as they may enable the chloroplast or plastid to produce a hairpin RNA having a smaller loop which may be a better substrate for insect DICER and related enzymes associated with insect RNAi.

In another preferred embodiment, the spacer sequence may have the sequence of consensus splice signals for splicing out of an intron from a primary RNA transcript of a plastid. In this embodiment, the consequence splice signals may flank a spacer sequence that does not itself have the sequence of a chloroplast intron.

Embodiments of the invention include introduction of a construct of the present invention into a plastid (i.e., transformation) to achieve steady-state levels of expression of one or more dsRNA molecules. A nucleic acid construct may, for example, be a vector, such as a linear or a closed circular plasmid. The vector system may be a single vector or plasmid, or two or more vectors or plasmids that together contain the total DNA to be introduced into the plastid genome of a host. In addition, a vector may be an expression vector. Coding regions of the invention can, for example, be suitably inserted into a vector under the control of a suitable promoter that functions in one or more hosts to drive expression of a linked coding sequence or other DNA sequence. Many vectors are available for this purpose, and selection of the appropriate vector will depend mainly on the size of the nucleic acid to be inserted into the vector and the particular plastid to be transformed with the vector. Each vector contains various components depending on its function (e.g., amplification of DNA or expression of DNA) and the particular host cell or subcellular organelle with which it is compatible.

A vector is a nucleic acid molecule introduced into a cell, for example, to produce a transformed cell. A vector may include nucleic acid sequences that permit it to replicate in the host cell, such as an origin of replication. Examples of vectors include, but are not limited to: a plasmid; cosmid; bacteriophage; or virus that carries exogenous DNA into a cell. A vector may also include one or more genes, antisense sequences, and/or selectable marker genes and other genetic elements known in the art. A vector may transduce, transform, or infect a cell, thereby causing the cell to express the nucleic acid molecules and/or proteins encoded by the vector. A vector optionally includes materials to aid in achieving entry of the nucleic acid molecule into the cell (e.g., a liposome, protein coating, etc.).

A vector may include nucleic acid sequences that permit it to replicate in a plastid of a host cell. Preferably the sequences enable the vector to replicate in a plastid of a host cell, but not in the cytosol or nucleus of the host cell.

In one embodiment, the nucleic acid construct includes nucleic acid sequences that permit the integration of the transgene it comprises into the plastid genome of the host cell. Preferably the nucleic acid construct is adapted so that the transgene selectively, or at least, preferentially, integrates into the plastid genome, rather than the nuclear genome. Thus in one embodiment, the nucleic acid construct contains sequences enabling integration into the genome of a plastid of a host cell, but not into the genome of the nucleus of the host cell. Examples of integration regions are trnV-3' - rps12, trnI - trnA, and trnfM - trnG. Examples of specific integration sites are: trnH/pbA, trnG/trnfM, ycf3/trnS, rbcL/accD, petA/psbJ, 5'rps12/clpP, petD/rpoA, ndhB/rps7, 3'rps12/trnV, trnV/rrn16, rrn16/trnI, trnI/trnA, trnN/trnR, and rpl32/trnL.

In order to enable expression (i.e., transcription) of dsRNA molecules in the plastids the nucleic acid construct may comprise a region encoding a dsRNA molecule, operably linked to one or more regulatory sequences, such as a heterologous promoter sequence that functions in a plastid wherein the nucleic acid construct is to be expressed.

A first nucleotide sequence is operably linked with a second nucleic acid sequence when the first nucleic acid sequence is in a functional relationship with the second nucleic acid sequence. When recombinantly produced, operably linked nucleic acid sequences are generally contiguous, and, where necessary to join two protein-coding regions, in the same reading frame (e.g., in a polycistronic ORF). However, nucleic acids need not be contiguous to be operably linked.

The term, "operably linked," when used in reference to a regulatory sequence and a coding sequence, means that the regulatory sequence affects the expression of the linked coding sequence. "Regulatory sequences," or "control elements," refer to nucleotide sequences that influence the timing and level/amount of transcription, RNA processing or stability, or translation of the associated coding sequence. Regulatory sequences may include promoters; translation leader sequences; introns; enhancers; stem-loop structures; repressor binding sequences; termination sequences; polyadenylation recognition sequences; etc. Particular regulatory sequences may be located upstream and/or downstream of a coding sequence operably linked thereto. Also, particular regulatory sequences operably linked to a coding sequence may be located on the associated complementary strand of a double-stranded nucleic acid molecule.

As used herein, the term "promoter" refers to a region of DNA that may be upstream from the start of transcription, and that may be involved in recognition and binding of RNA polymerase and other proteins to initiate transcription. A promoter may be operably linked to a coding sequence for expression in a cell, or a promoter may be operably linked to a nucleotide sequence encoding a signal sequence which may be operably linked to a coding sequence for expression in a cell. A "plant promoter" may be a promoter capable of initiating transcription in plant cells. Examples of promoters under developmental control include promoters that preferentially initiate transcription in certain tissues, such as leaves, roots, seeds, fibers, xylem vessels, tracheids, or sclerenchyma. Such promoters are referred to as "tissue-preferred." Promoters which initiate transcription only in certain tissues are referred to as "tissue-specific."

A "cell type-specific" promoter primarily drives expression in certain cell types in one or more organs, for example, vascular cells in roots or leaves. Promoters which initiate transcription only in certain subcellular organelles (e.g., such as mitochondria or chloroplasts or other plastids) are referred to as "plastid-specific", "chloroplast-specific", or "mitochondrion-specific" promoters, as the case may be. It will be understood that some promoters may initiate transcription in more than one subcellular location.

In one particularly preferred embodiment, the nucleic acid construct contains a promoter or regulatory element that is operable in a plastid and does not contain a promoter or regulatory element that is operable in the nucleus or cytosol or other organelle of the host cell.

An "inducible" promoter may be a promoter which may be under environmental control. Examples of environmental conditions that may initiate transcription by inducible promoters include anaerobic conditions and the presence of light. Tissue-specific, tissue-preferred, cell type specific, and inducible promoters constitute the class of "non-constitutive" promoters. A "constitutive" promoter is a promoter which may be active under most environmental conditions or in most cell or tissue types.

Any inducible promoter can be used in some embodiments of the invention. See Ward et al. (1993) Plant Mol. Biol. 22:361-366. With an inducible promoter, the rate of transcription increases in response to an inducing agent. Exemplary inducible promoters include, but are not limited to: Promoters from the ACEI system that responds to copper; In2 gene from maize that responds to benzenesulfonamide herbicide safeners; Tet repressor from TnIO; and the inducible promoter from a steroid hormone gene, the transcriptional activity of which may be induced by a glucocorticosteroid hormone (Schena et al. (1991) Proc. Natl. Acad. Sci. USA 88:0421).

Exemplary constitutive promoters include, but are not limited to: promoters from plant viruses, such as the 35S promoter from cauliflower mosaic virus (CaMV); promoters from rice actin genes; ubiquitin promoters; pEMU; MAS; maize H3 histone promoter; and the ALS promoter, Xbal/Ncol fragment 5' to the *Brassica napus* ALS3 structural gene (or a nucleotide sequence similarity to said Xbal Ncol fragment) (International PCT Publication No. WO 96/30530).

Additionally, any tissue-specific or tissue-preferred promoter may be utilized in some embodiments of the invention. Plants transformed with a nucleic acid molecule comprising a coding sequence operably linked to a tissue-specific promoter may produce the product of the coding sequence exclusively, or preferentially, in a specific tissue. Exemplary tissue-specific or tissue-preferred promoters include, but are not limited to: a root-preferred promoter, such as that from the phaseolin gene; a leaf-specific and light-induced promoter such as that from cab or rubisco; an anther-specific promoter such as that from LAT52; a pollen-specific promoter such as that from ZmJ3; and a microspore-preferred promoter such as that from apg.

Promoters suitable for use in constructs of the invention include those that are inducible, viral, synthetic, or constitutive, all of which are well known in the art. Non-limiting examples describing such promoters include U.S. Patent Nos. 6,437,217 (maize RS81 promoter); 5,641,876 (rice actin promoter); 6,426,446 (maize RS324 promoter); 6,429,362 (maize PR-1 promoter); 6,232,526 (maize A3 promoter); 6,177,611 (constitutive maize promoters); 5,322,938, 5,352,605, 5,359,142, and 5,530,196 (35S promoter); 6,433,252 (maize L3 oleosin promoter); 6,429,357 (rice actin 2 promoter, and rice actin 2 intron); 6,294,714 (light-inducible promoters); 6,140,078 (salt-inducible promoters); 6,252,138 (pathogen-inducible promoters); 6,175,060 (phosphorous deficiency-inducible promoters); 6,388,170 (bidirectional promoters); 6,635,806 (gamma-coixin promoter); and U.S. Patent Application Serial No. 09/757,089 (maize chloroplast aldolase promoter). Additional promoters include the nopaline synthase (NOS) promoter (Ebert et al. (1987) Proc. Natl. Acad. Sci. USA 84(16):5745-9) and the octopine synthase (OCS) promoter (both of which are carried on tumor-inducing plasmids of *Agrobacterium tumefaciens*); the caulimovirus promoters such as the cauliflower mosaic virus (CaMV) 19S promoter (Lawton et al. (1987) Plant Mol. Biol. 9:315-24); the CaMV 35S promoter (Odell et al. (1985) Nature 313:810-2); the figwort mosaic virus 35S-promoter (Walker et al. (1987) Proc. Natl. Acad. Sci. USA 84(19):6624-8); the sucrose synthase promoter (Yang and Russell (1990) Proc. Natl. Acad. Sci. USA 87:4144-8); the R gene complex promoter (Chandler et al. (1989) Plant Cell 1:1175-83); the chlorophyll a/b binding protein gene promoter; CaMV35S (U.S. Patent Nos. 5,322,938, 5,352,605, 5,359,142, and 5,530,196); FMV35S (U.S. Patent Nos. 6,051,753, and 5,378,619); a PC1SV promoter (U.S. Patent No. 5,850,019); the SCP1 promoter (U.S. Patent No. 6,677,503); and AGRtu.nos promoters (GenBank Accession No. V00087; Depicker et al. (1982) J. Mol. Appl. Genet. 1:561-73; Bevan et al. (1983) Nature 304: 184-7).

In particular embodiments, nucleic acid constructs of the invention comprise a tissue-specific promoter, such as a root-specific promoter. Root-specific promoters drive expression of operably-linked sequences exclusively or preferentially in root tissue. Examples of root-specific promoters are known in the art. See, e.g., U.S. Patents 5,110,732; 5,459,252 and 5,837,848; and Opperman et al. (1994) Science 263:221-3; and Hirel et al. (1992) Plant Mol. Biol. 20:207-18. In some embodiments, a region encoding a dsRNA molecule may be cloned between two root-specific promoters, which are operable in a transgenic plant cell and expressed therein to produce dsRNA molecules in the transgenic plant cell and/or plastid.

Additional regulatory sequences that may optionally be operably linked to a nucleic acid molecule of interest include 5' UTRs located between a promoter sequence and a coding sequence that function as a translation leader sequence. The translation leader sequence is present in the fully-processed mRNA, and it may affect processing of the primary transcript, and/or RNA stability. Examples of translation leader sequences include maize and petunia heat shock protein leaders (U.S. Patent No. 5,362,865), plant virus coat protein leaders, plant rubisco leaders, and others. See, e.g., Turner and Foster (1995) Molecular Biotech. 3(3):225-36. Non-limiting examples of 5' UTRs include GmHsp (U.S. Patent No. 5,659,122); PhDnaK (U.S. Patent No. 5,362,865); AtAntl; TEV (Carrington and Freed (1990) J. Virol. 64:1590-7); and AGRtunos (GenBank Accession No. V00087; and Bevan et al. (1983) Nature 304:184-7).

In some embodiments, the promoter region is derived from chloroplast genes, such as the psbA gene from spinach or pea, the rbcL and atpB promoter region from maize and rRNA promoters (from the plastid rrn operon). Examples of promoters are described in Verma & Daniell (2007) Plant Physiol. 145: 1129-43; Rasala et al. (2011) Plant Biotech. J. 9: 674-83, Hanley-Bowdoin and Chua, TIBS (1987) 12:67-70; Mullet et al., Plant Molec. Biol. (1985) 4:39-54; Hanley-Bowdoin (1986) PhD. Dissertation, The Rockefeller University; Krebbers et al., Nucleic Acids Res. (1982) 10:4985-5002; Zurawski et al., Nucleic Acids Res. (1981) 9:3251-3270; and Zurawski et al., Proc. Nat'l Acad Sci. U.S.A. (1982) 79:7699-7703. Other promoters may be identified and the relative strength of promoters so identified evaluated, by placing a promoter of interest 5' to a promoterless marker gene and observing its effectiveness relative to transcription obtained from, for example, the promoter from the psbA gene, the strongest chloroplast promoter identified to date. The efficiency of coding region expression additionally may be enhanced by a variety of techniques. These include the use of multiple promoters inserted in tandem 5' to the DNA sequence of interest, for example a double psbA promoter, the addition of enhancer sequences and the like. In one embodiment, the promoter region is derived from a chloroplast gene as shown in Table 2 below.

| Plastids Gene | Promoter | Product | |
|---|---|---|---|
| rps12 | Prps12 | **Translation** | ribosomal protein S12 |
| psbA | PpsbA | **Photosynthesis** | photosystem II protein D1 |
| rps16 | Prps16 | **Translation** | ribosomal protein S16 |
| psbK | PpsbK | **Photosynthesis** | photosystem II protein K |
| psbI | PpsbI | **Photosynthesis** | photosystem II protein I |
| atpA | PatpA | **Energy Metabolism** | ATP synthase CF1 alpha subunit |
| atpF | atpF | **Energy Metabolism** | ATP synthase CF0 B subunit |
| atpH | atpH | **Energy Metabolism** | ATP synthase CF0 C subunit |
| atpI | PatpI | **Energy Metabolism** | ATP synthase CF0 A subunit |
| rps2 | Prps2 | **Translation** | ribosomal protein S2 |
| rpoC2 | PrpoC2 | **Transcription** | RNA polymerase beta" subunit |
| rpoC1 | PrpoC1 | **Transcription** | RNA polymerase beta' subunit |
| rpoB | PrpoB | **Transcription** | RNA polymerase beta subunit |
| petN | PpetN | **Energy Metabolism** | cytochrome b6/f complex subunit VIII |
| psbM | PpsbM | **Photosynthesis** | photosystem II protein M |
| psbD | PpsbD | **Photosynthesis** | photosystem II protein D2 |
| psbC | psbC | **Photosynthesis** | photosystem II 44 kDa protein |
| psbZ | psbZ | **Photosynthesis** | photosystem II protein Z |
| rps14 | rps14 | **Translation** | ribosomal protein S14 |
| psaB | psaB | **Photosynthesis** | photosystem I P700 chlorophyll a apoprotein A2 |
| psaA | psaA | **Photosynthesis** | photosystem I P700 chlorophyll a apoprotein A1 |
| ycf3 | ycf3 | **Conserved Hypothetical Plastid** | photosystem I assembly protein Ycf3 |
| rps4 | Prps4 | **Translation** | ribosomal protein S4 |
| ndhJ | ndhJ | **Energy Metabolism** | NADH dehydrogenase subunit J |
| ndhK | ndhK | **Energy Metabolism** | NADH dehydrogenase subunit K |
| ndhC | ndhC | **Energy Metabolism** | NADH dehydrogenase subunit 3 |

(continued)

| Plastids Gene | Promoter | Product | |
|---|---|---|---|
| atpE | atpE | **Energy Metabolism** | ATP synthase CF1 epsilon subunit |
| atpB | PatpB | **Energy Metabolism** | ATP synthase CF1 beta subunit |
| rbcL | PrbcL | **Photosynthesis** | ribulose-1,5-bisphosphate carboxylase/oxygenase large subunit |
| accD | PaccD | **Fatty Acid Metabolism** | acetyl-CoA carboxylase beta subunit |
| psaI | psaI | **Photosynthesis** | photosystem I subunit VIII |
| ycf4 | ycf4 | **Conserved Hypothetical** | photosystem I assembly protein Ycf4 |
| cemA | cemA | **Transporters** | envelope membrane protein |
| petA | petA | **Energy Metabolism** | cytochrome f |
| psbJ | psbJ | **Photosynthesis** | photosystem II protein J |
| psbL | psbL | **Photosynthesis** | photosystem II protein L |
| psbF | psbF | **Photosynthesis** | photosystem II protein VI |
| psbE | psbE | **Photosynthesis** | photosystem II protein V |
| petL | petL | **Energy Metabolism** | cytochrome b6/f complex VI |
| petG | petG | **Energy Metabolism** | cytochrome b6/f complex V |
| psaJ | psaJ | **Photosynthesis** | photosystem I subunit IX |
| rpl33 | rpl33 | **Translation** | ribosomal protein L33 |
| rps18 | rps18 | **Translation** | ribosomal protein S18 |
| rpl20 | rpl20 | **Translation** | ribosomal protein L20 |
| clpP | PclpP | **Cellular Processes** | ATP-dependent Clp protease proteolytic subunit |
| psbB | psbB | **Photosynthesis** | photosystem II 47 kDa protein |
| psbT | psbT | **Photosynthesis** | photosystem II protein T |
| psbN | psbN | **Photosynthesis** | photosystem II protein N |
| psbH | psbH | **Photosynthesis** | photosystem II protein H |
| petB | petB | **Energy Metabolism** | cytochrome b6 |
| petD | petD | **Energy Metabolism** | cytochrome b6/f complex subunit IV |
| rpoA | rpoA | **Transcription** | RNA polymerase alpha subunit |
| rps11 | rps11 | **Translation** | ribosomal protein S11 |
| rpl36 | Prpl36 | **Translation** | ribosomal protein L36 |
| rps8 | Prps8 | **Translation** | ribosomal protein S8 |
| rpl14 | Prpl14 | **Translation** | ribosomal protein L14 |
| rpl16 | rpl16 | **Translation** | ribosomal protein L16 |
| rps3 | rps3 | **Translation** | ribosomal protein S3 |
| rpl22 | rpl22 | **Translation** | ribosomal protein L22 |
| rps19 | rps19 | **Translation** | ribosomal protein S19 |
| rpl2 | rpl2 | **Translation** | ribosomal protein L2 |
| rpl23 | rpl23 | **Translation** | ribosomal protein L23 |

(continued)

| Plastids Gene | Promoter | Product | |
|---|---|---|---|
| ycf2 | ycf2 | **Conserved Hypothetical Plastid** | Ycf2 |
| ndhB | ndhB | **Energy Metabolism** | NADH dehydrogenase subunit 2 |
| rps7 | rps7 | **Translation** | ribosomal protein S7 |
| ndhF | ndhF | **Energy Metabolism** | NADH dehydrogenase subunit 5 |
| rpl32 | rpl32 | **Translation** | ribosomal protein L32 |
| ccsA | PccsA | **Biosynthesis of Cofactors** | cytochrome c biogenesis protein |
| ndhD | PndhD | **Energy Metabolism** | NADH dehydrogenase subunit 4 |
| psaC | psaC | **Photosynthesis** | photosystem I subunit VII |
| ndhE | PndhE | **Energy Metabolism** | NADH dehydrogenase subunit 4L |
| ndhG | ndhG | **Energy Metabolism** | NADH dehydrogenase subunit 6 |
| ndhI | ndhI | **Energy Metabolism** | NADH dehydrogenase subunit I |
| ndhA | ndhA | **Energy Metabolism** | NADH dehydrogenase subunit 1 |
| ndhH | ndhH | **Energy Metabolism** | NADH dehydrogenase subunit 7 |
| rps15 | rps15 | **Translation** | ribosomal protein S15 |
| ycf1 | Pycf1 | **Conserved Hypothetical Plastid** | Ycf1 |
| rpl23 | rpl23 | **Translation** | ribosomal protein L23 |
| rpl2 | rpl2 | **Translation** | ribosomal protein L2 |
| tRNA genes | PtrnY<br>PtrnM<br>PtrnE<br>PtrnV | | |
| rRNA operon | Prrn | | |

For the most part, promoters functional in the chloroplast are constitutive rather than inducible. However, where it is desired to provide for inducible expression of the dsRNA molecule of interest, a regulatable promoter and/or a 5' untranslated region (5' UTR) containing sequences which provide for regulation at the level of transcription and/or translation (at 3' end) may be provided. Transcription and RNA stability appear to be important determinants of chloroplast gene expression. For example, the 5' untranslated region may be used from a gene wherein expression is regulatable by light. Similarly, 3' inverted repeat regions could be used to stabilize RNA of foreign genes. Regulatable genes may be identified by enhanced expression in response to a particular stimulus of interest and low or absent expression in the absence of the stimulus. For example, a light regulatable gene may be identified where enhanced expression occurs during irradiation with light, while substantially reduced expression or no expression occurs in the negligible of light. 5' UTRs which may be used in accordance with the invention include GGAGG, psbA, rbcL, atpB, Cry2A, and T7gene10.

Additional regulatory sequences that may optionally be operably linked to a nucleic acid molecule of interest also include 3' un-translated region, (3'UTR) 3' transcription termination regions, or poly-adenylation regions. These are genetic elements located downstream of a nucleotide sequence, and include polynucleotides that provide polyadenylation signal, and/or other regulatory signals capable of affecting transcription or mRNA processing. The polyadenylation signal functions in plants to cause the addition of polyadenylate nucleotides to the 3' end of the mRNA precursor. The polyadenylation sequence can be derived from a variety of plant genes, or from T-DNA genes. A non-limiting example of a 3' transcription termination region is the nopaline synthase 3' region (nos 3'; Fraley et al. (1983) Proc. Natl. Acad. Sci. USA 80:4803-7). An example of the use of different 3' nontranslated regions is provided in Ingelbrecht et al, (1989) Plant Cell 1:671-80. Non-limiting examples of polyadenylation signals include one from a Pisum sativum RbcS2 gene (Ps.RbcS2-E9; Coruzzi et al. (1984) EMBO J. 3:1671-9) and AGRtu.nos (GenBank Accession No. E01312). 3' UTRs which may be used in accordance with the invention include rps16, rbcL, psbA, and petD.

In some embodiments, the transformation vector may contain sequences specifically complementary to more than one target sequence, thus allowing production of more than one dsRNA for inhibiting expression of two or more genes in cells of one or more populations or species of target organisms. Segments of nucleotide sequence specifically complementary to nucleotide sequences present in different genes can be combined into a single composite nucleic acid molecule for expression in a transgenic plant. Such segments may be contiguous or separated by a spacer sequence.

A nucleic acid construct or vector of the present invention may comprise a selectable marker that confers a selectable phenotype on a transformed cell. The marker may encode biocide resistance, antibiotic resistance (e.g., kanamycin, Geneticin (G418), bleomycin, hygromycin, etc.), or herbicide tolerance (e.g., glyphosate, etc.).

Examples of selectable markers include, but are not limited to: a neo gene which codes for kanamycin resistance and can be selected for using kanamycin, G418, etc.; a bar gene which codes for bialaphos resistance; a mutant EPSP synthase gene which encodes glyphosate tolerance; a nitrilase gene which confers resistance to bromoxynil; a mutant acetolactate synthase gene (ALS) which confers imidazolinone or sulfonylurea resistance; and a methotrexate resistant DHFR gene. Multiple selectable markers are available that confer resistance to ampicillin, bleomycin, chloramphenicol, gentamycin, hygromycin, kanamycin, lincomycin, methotrexate, phosphinothricin, puromycin, spectinomycin, rifampicin, streptomycin and tetracycline, and the like. Examples of such selectable markers are illustrated in, e.g., U.S. Patent Nos. 5,550,318; 5,633,435; 5,780,708; and 6,118,047.

A recombinant nucleic acid construct or vector of the present invention may also include a screenable marker. Screenable markers may be used to monitor expression. Exemplary screenable markers include a β-glucuronidase or uidA gene (GUS) which encodes an enzyme for which various chromogenic substrates are known (Jefferson et al. (1987) Plant Mol. Biol. Rep. 5:387-405); an R-locus gene, which encodes a product that regulates the production of anthocyanin pigments (red color) in plant tissues (Dellaporta et al. (1988) "Molecular cloning of the maize R-nj allele by transposon tagging with Ac." In 18th Stadler Genetics Symposium. P. Gustafson and R. Appels, eds. (New York: Plenum), pp. 263-82); a β-lactamase gene (Sutcliffe et al. (1978) Proc. Natl. Acad. Sci. USA 75:3737-41); a gene which encodes an enzyme for which various chromogenic substrates are known {e.g., PADAC, a chromogenic cephalosporin); a luciferase gene (Ow et al. (1986) Science 234:856-9); a xylE gene that encodes a catechol dioxygenase that can convert chromogenic catechols (Zukowski et al. (1983) Gene 46(2-3):247-55); an amylase gene (Ikatu et al. (1990) Bio Technol. 8:241-2); a tyrosinase gene which encodes an enzyme capable of oxidizing tyrosine to DOPA and dopaquinone which in turn condenses to melanin (Katz et al. (1983) J. Gen. Microbiol. 129:2703-14); and an a-galactosidase.

In one embodiment, the nucleic acid construct may also include a coding region for production of an insecticidal polypeptide.

The insecticidal polypeptide may be insecticidal for a coleopteran, lepidopteran or hemipteran pest.

The insecticidal polypeptide may be one having a sequence of a polypeptide found in *Bacillus thuringiensis.*

An insecticidal *B. thuringiensis* polypeptide may be selected from the group consisting of: Cry1 (*e.g.,* Cry1Ab, Cry1Ac, Cry1A.105, Cry1Ca, Cry1Da), Cry2 (*e.g*., Cry2Ab), Cry3 (*e.g*., Cry3Bb; Cry3A), Cry6, Vip3 (*e.g.*, Vip3Ab1), Cry34, Cry35, and modifications thereof including, but not limited to additions and deletions. An expansive list of *Bts* is maintained and regularly updated at http://www.lifesci.sussex.ac.uk/home/Neil_Crickmore/Bt/intro.html. There are currently over 73 main groups of "Cry" toxins (Cry1-Cry73), with additional Cyt toxins and Vegetative Insecticidal Protein (VIP) toxins and the like. Many of each numeric group have capital-letter subgroups, and the capital letter subgroups have lower-cased letter sub-subgroups. (Cry1 has A-L, and Cry1A has a-i, for example).

The insecticidal polypeptide may be a PIP-1 polypeptide.

In one embodiment, a *B. thuringiensis* insecticidal polypeptide may be used with a construct containing dsRNA that is insecticidal for an essential gene in CPB (Colorado potato beetle), corn rootworm, or other insect pest. Such target genes include, for example, ATPase encoding genes in CPB. Other such target genes include, for example, vacuolar ATPase, ARF-1, Act42A, CHD3, EF-1α, ROP, RNAPII, and TFIIB in corn rootworm. An example of a suitable target gene is vacuolar ATPase, as disclosed in WO2007035650.

In some embodiments, recombinant nucleic acid constructs, as described, *supra,* may be used in methods for the creation of transgenic plants and expression of heterologous nucleic acids specifically in plant plastids to enable production of steady state levels of dsRNA molecules. Plant transformation vectors can be prepared, for example, by inserting nucleic acid constructs encoding dsRNA molecules into plant transformation vectors, specifically, plastid transformation vectors and introducing these into plastids (for example, chloroplasts).

Where it is desired to obtain replication of a plasmid containing the expression cassette in the chloroplast any chloroplast origin of replication may be used in the expression construct.

In one embodiment, the nucleic acid construct is adapted to prevent the expression of dsRNA produced therefrom in the host cell nucleus or other host cell compartment where siRNA could be produced from the dsRNA. This enables the production of plant material containing insecticidal dsRNA that is not contaminated by plant derived siRNA or associated Argonaute proteins, and hence has enhanced insecticidal potency. According to this embodiment there is provided a nucleic acid construct for transformation of a vascular plant, comprising:

- a coding region for encoding a double stranded RNA (dsRNA) molecule;
- a promoter operable in a plastid for enabling production of the dsRNA molecule in a plastid, preferably selected from a promoter or regulatory element shown in Table 2;
- one or more integration sites selective for integration of the construct into a plastid genome of a vascular plant; preferably selected from the following group of integration sites: trnH/pbA, trnG/trnfM, ycf3/trnS, rbcL/accD, petA/psbJ, 5'rps12/clpP, petD/rpoA, ndhB/rps7, 3'rps12/trnV, trnV/rrn16, rrn16/trnI, trnI/trnA, trnN/trnR, and rpl32/trnL.

According to this embodiment, the use of the promoter that is only activated in the chloroplast and use of the integration sites that enable integration into the chloroplast genome only, prevent the formation of plants that express dsRNA for the relevant transgene outside of the chloroplast and prevent the formation of plants that express siRNA for the relevant transgene. One advantage, is that one is able to select for transformants on the basis of the presence of a chloroplast transformation without having to further select for cells that are both plastid and nuclear transformants because this latter population of cells is not created by use of the construct.

In a particularly preferred embodiment there is provided: a nucleic acid construct for transformation of a vascular plant, comprising:

- a coding region for encoding an RNA molecule having first and second regions spaced apart by a spacer region, wherein the first region has partial or complete sequence complimentarity to the second region, and wherein the spacer is identical or homologous to a sequence of an intron shown in Table 1;
- a promoter operable in a plastid for enabling production of the RNA molecule in a plastid, preferably selected from a promoter or regulatory element shown in Table 2;
- one or more integration sites selective for integration of the construct into a plastid genome of a vascular plant; preferably selected from the following group of integration sites: trnH/pbA, trnG/trnfM, ycf3/trnS, rbcL/accD, petA/psbJ, 5'rps12/clpP, petD/rpoA, ndhB/rps7, 3'rps12/trnV, trnV/rrn16, rrn16/trnI, trnI/trnA, trnN/trnR, and rpl32/trnL.

**C. Plastid transformation**

The nucleic acid constructs of the invention may be transformed into a plant cell of interest by any of a number of methods. These methods include, for example, biolistic devices (See, for example, Sanford, Trends In Biotech. (1988) 6:299-302; U.S. Pat. No. 4,945,050); electroporation (Fromm et al., Proc. Nat'l. Acad. Sci. (USA) (1985) 82:5824-5828); use of a laser beam, electroporation, microinjection or any other method capable of introducing DNA into a chloroplast. The use of these techniques permits the application of the invention described herein in a wide variety of both monocotyledonous and dicotyledonous plant cells.

As used herein, the term "transformation" or "transduction" refers to the transfer of one or more nucleic acid molecule(s) into a cell. A cell is "transformed" by a nucleic acid molecule transduced into the cell when the nucleic acid molecule becomes stably replicated by the cell, either by incorporation of the nucleic acid molecule into the cellular genome, or by episomal replication. As used herein, the term "transformation" encompasses all techniques by which a nucleic acid molecule can be introduced into such a cell. Examples include, but are not limited to: transfection with viral vectors; transformation with plasmid vectors; electroporation (Fromm et al. (1986) Nature 319:791-3); lipofection (Feigner et al. (1987) Proc. Natl. Acad. Sci. USA 84:7413-7); microinjection (Mueller et al. (1978) Cell 15:579-85); Agrobacterium-mediated transfer (Fraley et al. (1983) Proc. Natl. Acad. Sci. USA 80:4803-7); direct DNA uptake; and microprojectile bombardment (Klein et al. (1987) Nature 327:70).

For use in the bombardment transformation technique, a nucleic acid construct is adsorbed to a bombardment particle, typically consisting of tungsten particle having an average size of about 0.7 $\mu$m. Particles consisting of other metals having a density similar to tungsten may also find use, such as gold, platinum and the like. Typically, about 2-5 $\mu$g of DNA is adsorbed per $\mu$g of tungsten, usually about 2 $\mu$g of DNA per $\mu$g of tungsten. Following adsorption of the DNA to the particles, any clumps of particles are dispersed, for example, by sonication. Any method to fix the DNA on the outside surface of the metal bombardment particles is acceptable and are known to those skilled in the art. (See, for example, Sanford et al. (1988) *supra* and Klein et al. *supra*). The DNA must be secured to the particles for delivery, but not fixed in such a manner as to impede release of the DNA into the cell.

For transformation, about 100-500 $\mu$g, generally approximately 200 $\mu$g of bombardment particles to which the expression cassette has been adsorbed are loaded into a particle gun (such as those available from Biolistics, Inc. and DuPont) according to the manufacturer's directions. Isolated cells generally 100-300 $\mu$g fresh weight per petri plate (5 cm diameter) are placed on a growth surface, such as a petri dish or tissue culture dish containing for example Whatman #1 filter paper with growth medium to adhere the cells to a solid surface. The cells do not have to be in a single cell layer, but may be a few layers thick. The immobilized cells are placed in the bombardment chamber of the particle gun and placed under as high a vacuum as feasible, generally about 0.07 to 0.3 atmospheres, preferably 0.07. The pressure in the sample chamber is then reduced to about 0.07 atmospheres prior to bombardment. The cells preferably are bombarded about 10 cm from the end of the barrel of the particle gun (at the fourth level in the DuPont gene gun). The firing mechanism of the particle gun is activated and the cells are bombarded from 1-3 times, generally twice to increase the number of transformed cells. The vacuum is then released, and the bombarded cells placed in fresh growth medium at about 26° C., preferably in the light in growth chambers. Other bolistic devices include the use of "flying discs" such as those made from plastic membranes or discs made of, for example nylon mesh (94 μm) ("helium entrainment" method).

A plant containing transgenic plastids (eg, chloroplasts) may be generated when the host cell used in the transformation process possesses totipotency. Procedures for regeneration of transgenic plants from transformed cells or tissues are, in general, similar, with suitable modifications within the capability of one skilled in the art. Regeneration of dicots such as sugar beets, Freytag et al. Plant Cell Rep. (1988) 7:30-34; tobacco, Svab et al. Proc. Nat'l Acad. Sci. U.S.A. (1990) 8526-8530, or monocots such as wheat from anthers or embryos (see below) routinely has been successful.

If it is desired to transform plastids in other plant materials, for example anther culture derived plants or embryo derived callus, the plant material is placed in a convenient container, for example a petri dish as described above for isolated cells. (See also US 6,680,426; Hanson et al (2012) Journal of Experimental Botany, 64: 753-768; and Golds et al., (1993) Nature Biotechnology 11, 95-97).

Once the plastid has been shown to have been transformed, the cells of the plant may be used repeatedly for tissue culture, followed by a growth of callus tissue where desired or regeneration of a plant. Thus, the modified plant cell may be repetitively regenerated by use of cell and tissue culture. In some instances, proper propagation may be maintained from seed. In order to improve the ability to identify transformed cells, one may desire to employ a selectable or screenable marker gene, as previously set forth, with the transformation vector used to generate the transformant. In the case where a selectable marker is used, transformed cells are identified within the potentially transformed cell population by exposing the cells to a selective agent or agents. In the case where a screenable marker is used, cells may be screened for the desired marker gene trait.

Cells that survive the exposure to the selective agent, or cells that have been scored positive in a screening assay, may be cultured in media that supports regeneration of plants. In some embodiments, any suitable plant tissue culture media (e.g., MS and N6 media) may be modified by including further substances, such as growth regulators. Tissue may be maintained on a basic medium with growth regulators until sufficient tissue is available to begin plant regeneration efforts, or following repeated rounds of manual selection, until the morphology of the tissue is suitable for regeneration (e.g., at least 2 weeks), then transferred to media conducive to shoot formation. Cultures are transferred periodically until sufficient shoot formation has occurred. Once shoots are formed, they are transferred to media conducive to root formation. Once sufficient roots are formed, plants can be transferred to soil for further growth and maturation.

To confirm the presence of a dsRNA molecule in the regenerating plants, a variety of assays may be performed. Such assays include, for example: molecular biological assays, such as Southern and Northern blotting, PCR, and nucleic acid sequencing; plant part assays, such as leaf or root assays.

Depending on the transformation approach, the transformants may include cells that are both plastid and nuclear transformants so that selection for a marker linked to plastid transformation might also result in the selection of cells that are both plastid and nuclear transformants, potentially resulting in cells that could express dsRNA and/or form siRNA outside of the chloroplast which might impact insecticidal potency of a plant generated by these cells. This problem can be addressed by utilising a preferred construct of the invention described above that contains homologous recombination regions (also described herein as integration sites) that enable integration in the plastid genome, but not the nuclear genome, and use of a promoter that is active in the chloroplasts only. This enables the selection method to select cells or plants that produce dsRNA in plastids and that do not produce dsRNA outside of plastids, or siRNA.

Integration events may be analyzed, for example, by PCR amplification using, e.g., oligonucleotide primers specific for a nucleic acid molecule or dsRNA of interest. PCR genotyping is understood to include, but not be limited to, polymerase-chain reaction (PCR) amplification of genomic DNA derived from isolated host plant callus tissue predicted to contain a nucleic acid molecule of interest integrated into the genome, followed by standard cloning and sequence analysis of PCR amplification products. Methods of PCR genotyping have been well described (for example, Rios, Q. et al. (2002) Plant J. 32:243-53) and may be applied to genomic DNA derived from any plant species (e.g., *Z. mays* or *G. max*) or tissue type, including cell cultures.

In particular embodiments, at least 2, 3, 4, 5, 6, 7, 8, 9 or 10 or more different dsRNA molecules are produced in a plant cell. The dsRNA molecules may be expressed from multiple nucleic acid constructs introduced in different transformation events, or from a single nucleic acid construct introduced in a single transformation event. In some embodiments, a plurality of dsRNA molecules are expressed under the control of a single promoter. In other embodiments, a plurality of dsRNA molecules are expressed under the control of multiple promoters. Single dsRNA molecules may be expressed that comprise multiple nucleic acid sequences that are each homologous to different loci within one or more organisms having target gene sequences.

In addition to direct transformation of a plant with a nucleic acid construct, transgenic plants can be prepared by crossing a first plant having at least one transgenic event with a second plant lacking such an event. For example, a nucleic acid construct comprising a nucleotide sequence that encodes a dsRNA molecule may be introduced into a first plant line that is amenable to transformation to produce a transgenic plant, which transgenic plant may be crossed with a second plant line to introgress the nucleotide sequence that encodes the dsRNA molecule into the genetic background of the second plant line.

Any of a number of vascular plants may be transformed in accordance with the above methods.

Plastids may also be transformed with insecticidal constructs targeting the same or different biological pathways of action in the pest as the dsRNA produced by the plastids and nucleic acid constructs of the invention. Examples of these constructs include those encoding insecticidal proteins as mentioned *supra,* especially *B. thuringiensis* insecticidal proteins, preferably Cry protiens. The relevant coding region of these constructs may be provided in the nucleic acid constructs of the invention (*i.e.*, those that produce dsRNA), or they may be in separate constructs. Regarding the latter, for example, in one embodiment, the plastid may include a first nucleic acid construct including:

- a coding region for encoding a double stranded RNA (dsRNA) molecule;
- a promoter operable in a plastid for enabling production of the dsRNA molecule in a plastid;
- one or more integration sites selective for integration of the construct into a plastid genome of a vascular plant;
- wherein the dsRNA has a sequence enabling the production of siRNA therefrom; and

a further nucleic acid construct including:
a coding region for encoding an insecticidal agent, preferably an insecticidal protein.

In one embodiment the plastid has more than 2 different nucleic acid constructs, one of which is the nucleic acid construct for encoding or producing insecticidal dsRNA as disclosed herein. In other embodiments, the plastid has more than 3, 4, 5, 6, 7, 8, 9, 10 or more different constructs, at least one of which is the nucleic acid for encoding or producing insecticidal dsRNA as disclosed herein.

**D. Plants**

Vascular plants are also known as tracheophytes and also as higher plants. Vascular plants are land plants that have lignified tissues (the xylem) for conducting water and minerals throughout the plant. They also have a specialized non-lignified tissue (the phloem) to conduct products of photosynthesis. Accordingly, the plant of the present invention can be a plant selected from the group consisting of clubmosses, horsetails, ferns, gymnosperms (including conifers) and angiosperms (flowering plants), and plants from the group Tracheophyta and Tracheobionta.

In a preferred embodiment the plant is a dicot selected from the group consisting of: canola, cotton, potato, quinoa, amaranth, buckwheat, safflower, soybean, sugarbeet, sunflower, rape, tobacco, *Arabidopsis, Brassica* species (e.g., *B. napus, B. rapa, B. juncea, B. carinata, B. nigra, B. oleracea, B. alba,* etc.), cotton, alfalfa, and clover.

In a preferred embodiment the plant is a monocot selected from the group consisting of: corn, rice, rye, sorghum, millet, wheat, sugarcane, oats, barley, pineapple, banana, palm, ornamentals, and grasses (e.g., *Brachiaria, Lolium,* and fescue).

Thus, in a further embodiment, the present invention provides a plant containing a plastid comprising:

- a nucleic acid construct including:
    - a coding region for encoding a double stranded RNA (dsRNA) molecule;
    - a promoter operable in the plastid for enabling production of the dsRNA molecule;
- wherein the dsRNA has a sequence enabling the production of siRNA therefrom.

In yet a further embodiment, the present invention provides a plant containing a nucleic acid construct for transformation of a vascular plant, comprising:

- a coding region for encoding a double stranded RNA (dsRNA) molecule;
    - a promoter operable in a plastid for enabling production of the dsRNA molecule in a plastid;
    - one or more integration sites selective for integration of the construct into a plastid genome of a vascular plant;
- wherein the dsRNA has a sequence enabling the production of siRNA therefrom.

In a preferred embodiment a plant may not contain siRNA for hybridising to the dsRNA encoded by the nucleic acid construct. In another preferred embodiment, a plant may not contain dsRNA encoded by the nucleic acid construct in a compartment other than a plastid. In these embodiments, the plant may contain dsRNA in a plastid of the plant but does not contain siRNA derived from or otherwise having sequence homology or complimentarity to the dsRNA produced by the nucleic acid construct in another organelle, cytosol, nucleus or elsewhere in the plant.

It will be understood that while the plants of the invention generally do not contain siRNA derived from or otherwise having sequence homology or complimentarity to the dsRNA produced by the nucleic acid construct, nor dsRNA outside of the chloroplast that has sequence homology or complimentarity to the dsRNA produced by the nucleic acid construct inside the chloroplast, in one embodiment, the plants of the invention may have dsRNA or siRNA that does not have sequence homology or complimentarity to the dsRNA produced by the nucleic acid construct inside the chloroplast. In this embodiment, a plant according to the invention may further include a nuclear transformation event that enables the production or expression of dsRNA or siRNA in the nucleus of the plant, provided that the dsRNA or siRNA does not have a sequence homology or complimentarity with that of the dsRNA produced inside the chloroplast. In one example a plant may be further engineered to provide for gene silencing thereby forming a desirable agronomic trait or an insecticidal property. The latter may arise from the formation of a siRNA that results in the production of an insecticidal compound by the plant.

In one embodiment, the plant may include cellular organelles, other than a chloroplast or plastid, that contain a nucleic acid construct for encoding an insecticidal agent, such as RNA or protein, or the insecticidal agent itself. In one embodiment the nucleus or cytoplasm contains at least one nucleic acid construct for encoding an insecticidal agent, such as RNA or protein, or the insecticidal agent itself, preferably more than 3, 4, 5, 6, 7, 8, 9, 10 or more different constructs or gene products encoded by the constructs..

A plant of the invention may be heteroplasmic for the nucleic acid construct of embodiments of the invention, where only a fraction of each cell's total number of chloroplasts are transformed, or homoplasmic, where most or all of the chloroplasts in each cell are transformed. Preferably the plant is homoplasmic. More preferably the plant is of a generation post T1 and is homoplasmic.

The plants of the invention may include a mutation in the gene encoding a gene product required for function of the RNAi pathway. One example of such a gene product is DICER-like 4. Others include DRB4 and its orthologs. These plants may be generated by providing plant cells or vegetative tissue that contains the mutation (for example a mutation resulting from knock out or homologous recombination) and transforming those cells with a nucleic acid construct of embodiments of the invention described herein. Alternatively, a plant transformed with a nucleic acid construct of embodiments of the invention described herein may be crossed with a plant having mutation in gene encoding a gene product required for function of the RNAi pathway.

The plants of the invention may also include a gene encoding tolerance to a herbicide, such as a carboxylic acid -containing herbicide including a phenoxy auxin.

In one embodiment, the invention provides a seed, cutting or other vegetative material derived or otherwise obtained from a plant according to the invention, especially a plant containing a plastid comprising:

- a nucleic acid construct including:
    - a coding region for encoding a double stranded RNA (dsRNA) molecule;
    - a promoter operable in the plastid for enabling production of the dsRNA molecule;
- wherein the dsRNA has a sequence enabling the production of siRNA therefrom.

A seed as described above may contain insecticidal dsRNA, and may contain the nucleic acid construct containing the coding region and promoter described above. Alternatively, the seed may not contain insecticidal dsRNA, (for example because the promoter is not activated in the seed) and may simply contain the nucleic acid construct containing the coding region and promoter described above, which is then able to be activated to form insecticidal dsRNA in the plastid or chloroplast, for example when the seed germinates and/or produces foliage.

The seed may be coated, and in one form, the seed may be coated with insecticidal RNA, including any form of silencing RNA, preferably dsRNA. Other coatings may include other insecticides, including insecticidal proteins or chemicals as known in the art.

In other embodiments, there is provided a commodity product produced by a plant according to the above described embodiments.

## E. Insecticidal compositions

The invention further relates to utilising material derived from plants according to the invention, preferably plants that are homoplasmic for production of insecticidal dsRNA in chloroplasts or related plastids. These materials may be provided in particular grade forms of purity with respect to the insecticidal dsRNA produced in the plastids or chloroplasts of the plants of the invention.

In some embodiments, the material may be processed plant material that has undergone simple physical treatment (for example chopping, cutting, grinding) rather than chemical treatment.

In other embodiments, the material may have undergone a chemical treatment, for example to extract and/or separate and/or preserve one or more components of the material.

Further, the material may be provided in the form of a living cell culture.

In certain embodiments, these materials may be utilised as an insecticidal composition in one or more applications, including as seed coating for coating seeds, including seeds which have not been transformed with insecticidal dsRNA.

In another embodiment, the material may be used as an insecticidal bait. A bait may attract a pest or prevent a pest from ingress to a particular location.

The insecticidal compositions may take the form of a sprayable liquid for spraying seeds, plants or crops, or a solid such as a powder, granule, pellet or the like.

**F. Methods**

In one embodiment there is provided a method of controlling insect pests comprising presenting or providing to said pests or to the environment of said pests a plant including a plastid or nucleic acid construct described above. Typically the dsRNA contained in the chloroplasts or plastids of the plant functions upon contact with the pest or insect, preferably on contacting with the GI tract of the pest or insect, to inhibit a biological function in the pest or insect, or to invoke a RNAi pathway in the pest or insect resulting in the interference in the function of an essential insect or pest gene. Preferably the environment in which control of insect pests is required is a crop or field of plants.

Typically the control of the insect pests results in the pest population being reduced relative to a pest population in an environment which does not contain plants having the nucleic acid or plastids of the invention.

In one embodiment the control of pests results in inhibiting or minising the growth of the insect pests, relative to a pest population that has not consumed a plant having the nucleic acid construct or plastid of the invention. In this embodiment, the pests may or may not be killed by their consumption of the plant having the nucleic acid construct or plastid of the invention. In this embodiment the inhibition or minimisation of growth of insect pests may result in minimised crop damage, and may result in improved yield.

In certain embodiments, the plants and plastids of the invention and dsRNA containing same are insecticidal to the extent that they induce death, and/or reduce growth rate and/or reduce fecundity.

The crop or field of plants may consist of plants including a plastid or nucleic acid construct as described above, or may include these plants and contain non chloroplast transformant refuge plants in the form of plants that do not contain a plastid or nucleic acid construct as described above. Where the crop or field contains non chloroplast transformant refuge plants, preferably the crop or field contains less than 30%, preferably less than 20%, preferably less than 10%, more preferable less than 5% of plants that are non chloroplast transformant refuge plants.

Preferably the control of the pests results in minimisation or reduction of damage to leaf, roots, reproductive tissues, or foliage of the plants. Preferably the reduction of damage is at least 50%, preferably, 60%, 70%, 80%, 90%, 95%, or greater than 95% of that observed in a non transformed control. Thus the invention provides a method for improving a yield of a crop or field of plants comprising presenting to pests or pathogens in the crop or field a plant including a plastid or nucleic acid construct described above, preferably to reduce damage of a plant or part thereof by the pest or pathogen.

In another embodiment there is provided a method for improving the yield of a crop including:

providing a plant or propagative material therefrom including a nucleic acid construct or plastid according to the invention;

cultivating the plant to allow the expression of the nucleic acid construct in the cultivated plant, wherein the expression of the nucleic acid product, thereby forming dsRNA, inhibits pest viability or growth in or on the plant.

In one embodiment there is provided a method of controlling insect pests, or preventing infestation or damage caused by insect pests, or killing insect pests in maize comprising presenting to said pests a maize plant including a plastid or nucleic acid construct described above. Preferably the insect pest is selected from the table below.

**Maize:**

| Lepidopteran | Coleopteran |
|---|---|
| *Agromyza parvicornis,* corn blot leafminer | *Chaetocnema pulicaria*, corn flea beetle |
| *Agrotis ipsilon,* black cutworm | *Diabrotica longicornis barberi,* northern corn rootworm |
| *Diatraea grandiosella,* southwestern corn borer | *Diabrotica undecimpunctata howardi,* southern corn rootworm |
| *Diatraea saccharalis,* sugarcane borer | *Diabrotica virgifera virgifera* LeConte, western corn rootworm |
| *Elasmopalpus lignosellus,* lesser cornstalk borer | *Cyclocephala borealis,* northern masked chafer (white grub) |
| *Helicoverpa zea,* corn earworm | *Cyclocephala immaculata,* southern masked chafer (white grub) |
| *Ostrinia nubilalis,* European corn borer | *Melanotus* spp., wireworms<br>*Agroites mancus,* heat wireworm<br>*Limonius agonus*, eastern field wireworm |
| *Spodoptera frugiperda,* fall armyworm | *Popillia japonica,* Japanese beetle |
| *Papaipema nebris,* stalk borer | *Sphenophorus maidis,* maize billbug<br>*Sphenophorus aequalis aequalis,* (billbug)<br>curlew-bug (bill bug) |
| | Southern corn leaf beetle |
| | Seedcorn beetles |
| **Other** | |
| *Melanoplus sanguinipes,* migratory grasshopper | *Anaphothrips obscrurus,* grass thrips |
| *Melanoplus femurrubrum,* redlegged grasshopper | *Anuraphis maidiradicis*, corn root aphid |
| *Rhopalosiphum maidis,* corn leaf aphid | *Blissus leucopterus,* chinch bug |
| *Solenopsis milesta,* thief ant | *Hylemya platura, Delia platura,* seed corn maggot |
| *Tetranychus urticae,* twospotted spider mite | *Colaspis brunnea,* Grape colaspis |
| *Crambus caliginosellus* corn root webworm | |
| *Crambus teterrellus,* bluegrass webworm | |

In another embodiment there is provided a method of controlling insect pests, or preventing infestation or damage caused by insect pests, or killing insect pests in sorghum comprising presenting to said pests a sorghum plant including a plastid or nucleic acid construct described above. Preferably the insect pest is selected from the table below.

**Sorghum:**

| Lepidopteran | Coleopteran |
|---|---|
| *Chilo partellus,* sorghum borer | *Phyllophaga crinita,* white grub |
| *Helicoverpa zea,* corn earworm | *Eleodes, Conoderus,* and *Aeolus* spp., wireworms |
| *Feltia subterranea,* granulate cutworm | *Oulema melanopus,* cereal leaf beetle |
| *Elasmopalpus lignosellus,* lesser cornstalk borer | *Sphenophorus maidis,* maize billbug |
| *Spodoptera frugiperda,* fall armyworm | *Chaetocnema pulicaria,* corn flea beetle |
| **Other** | |
| *Blissus leucopterus leucopterus,* chinch bug | *Contarinia sorghicola,* sorghum midge |
| *Tetranychus cinnabarinus,* carmine spider mite | *Tetranychus urticae,* twospotted spider mite |
| *Rhopalosiphum maidis;* corn leaf aphid | *Sipha flava,* yellow sugarcane aphid |

In one embodiment there is provided a method of controlling insect pests, or preventing infestation or damage caused by insect pests, or killing insect pests in sunflower comprising presenting to said pests a sunflower plant including a plastid or nucleic acid construct described above. Preferably the insect pest is selected from the table below.

**Sunflower:**

| Lepidopteran | Coleopteran |
|---|---|
| *Suleima helianthana,* sunflower bud moth | *Zygogramma exclamationis,* sunflower beetle |
| *Homoeosoma electellum,* sunflower moth | *Bothyrus gibbosus,* carrot beetle |
| **Other** | |
| *Neolasioptera murtfeldtiana,* sunflower seed midge | |

In one embodiment there is provided a method of controlling insect pests, or preventing infestation or damage caused by insect pests, or killing insect pests in rice comprising presenting to said pests a rice plant including a plastid or nucleic acid construct described above. Preferably the insect pest is selected from the table below.

**Rice:**

| Lepidopteran | Coleopteran |
|---|---|
| *Diatraea saccharalis,* sugarcane borer | *Lissorhoptrus oryzophilus* rice water weevil |
| *Helicoverpa zea,* corn earworm | *Sitophilus oryzae,* rice weevil |
| *Spodoptera frugiperda,* fall armyworm | |
| **Other** | |
| *Nephotettix nigropictus*, rice leafhopper | *Blissus leucopterus leucopterus* chinch bug; |
| *Acrosternum hilare,* green stink bug | *Colaspis brunnea,* grape *colaspis* |

In one embodiment there is provided a method of controlling insect pests, or preventing infestation or damage caused by insect pests, or killing insect pests in wheat comprising presenting to said pests a wheat plant including a plastid or nucleic acid construct described above. Preferably the insect pest is selected from the table below.

**Wheat:**

| Lepidopteran | Coleopteran |
|---|---|
| *Spodoptera frugiperda,* fall armyworm | *Oulema melanopus,* cereal leaf beetle |
| *Pseudaletia unipunctata,* army worm | *Hypera punctata,* clover leaf weevil |
| *Elasmopalpus lignosellus,* lesser cornstalk borer | *Diabrotica undecimpunctata howardi,* southern corn rootworm |
| *Elasmopalpus lignosellus,* lesser cornstalk borer | |
| *Agrotis orthogonia,* western cutworm | |
| **Other** | |
| *Melanoplus diferentialis,* differential grasshopper | *Melanoplus femurrubrum,* redlegged grasshopper |
| *Mayetiola destructor,* Hessian fly | *Melanoplus sanguinipes,* migratory grasshopper |
| *Meromyza americana,* wheat stem maggot | *Sitodiplosis mosellana,* wheat midge |
| *Frankliniella fusca,* tobacco thrips | *Hylemya coarctata,* wheat bulb fly |
| *Aceria tulipae,* wheat curl mite | *Cephus cinctus,* wheat stem sawfly |
| *Macrosiphum avenae,* English grain aphid | *Schizaphis graminum,* greenbug |
| | Russian wheat aphid |

In one embodiment there is provided a method of controlling insect pests, or preventing infestation or damage caused by insect pests, or killing insect pests in cotton comprising presenting to said pests a cotton plant including a plastid or nucleic acid construct described above. Preferably the insect pest is selected from the table below.

**Cotton:**

| Lepidopteran | Coleopteran |
|---|---|
| *Helicoverpa zea,* cotton bollworm | *Anthonomus grandis,* boll weevil |
| *Helicoverpa armigera,* cotton bollworm | |
| *Heliothis virescens,* cotton budworm | |
| *Spodoptera exigua,* beet armyworm | |
| *Pectinophora gossypiella,* pink bollworm | |
| **Other** | |
| *Pseudatomoscelis seriatus,* cotton fleahopper | *Aphis gossypii,* cotton aphid |
| *Lygus lineolaris,* tarnished plant bug | *Trialeurodes abutilonea,* bandedwinged whitefly, |
| *Melanoplus diferentialis,* differential grasshopper | *Melanoplus femurrubrum,* redlegged grasshopper |
| *Frankliniella fusca,* tobacco thrips | *Thrips tabaci,* onion thrips |
| *Tetranychus urticae,* twospotted spider mite | *Tetranychus cinnabari nus,* carmine spider mite |

In one embodiment there is provided a method of controlling insect pests, or preventing infestation or damage caused by insect pests, or killing insect pests in barley comprising presenting to said pests a barley plant including a plastid or nucleic acid construct described above. Preferably the insect pest is selected from the table below.

**Barley:**

| Lepidopteran | Coleopteran |
|---|---|
| *Ostrinia nubilalis,* European corn borer | |
| *Agrotis ipsilon,* black cutworm | |
| Other | |
| *Acrosternum hilare,* green stink bus | *Euschistus servos,* brown stink bug |
| *Delia platura,* seedcorn maggot | *Mayetiola destructor,* Hessian fly |
| *Petrobia latens,* brown wheat mite | *Schizaphis gram inum,* greenbug |
| *Blissus leucopterus leucopterus,* chinch bug | |

In one embodiment there is provided a method of controlling insect pests, or preventing infestation or damage caused by insect pests, or killing insect pests in oil seed rape comprising presenting to said pests an oil seed plant including a plastid or nucleic acid construct described above. Preferably the insect pest is selected from the table below.

**Oil Seed Rape**

| Lepidopteran | Coleopteran |
|---|---|
| *Plutella xylostella,* diamondback moth | *Phyllotreta cruciferae,* Flea beetle |
| *Mamestra configurata,* Bertha armyworm | |
| **Other** | |
| *Delia* spp., Root maggots *Brevicoryne brassicae,* cabbage aphid | |

In one embodiment there is provided a method of controlling insect pests, or preventing infestation or damage caused by insect pests, or killing insect pests in soybean comprising presenting to said pests a soybean plant including a plastid or nucleic acid construct described above. Preferably the insect pest is selected from the table below.

**Soybean:**

| Lepidopteran | Coleopteran |
|---|---|
| *Pseudoplusia includens,* soybean looper | *Epilachna varivestis,* Mexican bean beetle |
| *Plathypena scabra,* green cloverworm | *Cerotoma trifurcate,* Bean leaf beetle |
| *Agrotis ipsilon,* black cutworm | *Diabrotica undecimpunctata howardi,* Spotted cucumber beetle |
| *Heliothis virescens,* cotton budworm | *Epicauta pestifera, Epicauta lemniscata,* Blister beetles |
| *Ostrinia nubilalis,* European corn borer | *Popillia japonica,* Japanese beetle |
| *Spodoptera exigua,* beet armyworm | *Dectes texanus texanus*, Soybean stem borer |
| *Helicoverpa zea,* cotton bollworm | *Colaspis brunnea,* Grape colaspis |
| *Anticarsia gemmatalis,* velvetbean caterpillar | |
| *Heliothis zea,* Corn earworm | |
| *Spodoptera ornithogalli,* Yellowstriped armvworm | |
| *Colias eurytheme,* alfalfa caterpillar | |
| *Elasmopalpus lignosellus,* Lesser cornstalk borer | |
| | |
| **Other** | |
| *Empoasca fabae,* potato leafhopper | *Tetranychus turkestani,* strawberry spider mite |
| *Melanoplus femurrubrum,* redlegged grasshopper | *Hylemya platura,* seedcorn maggot |
| *Tetranychus urticae,* twospotted spider mite | *Thrips tabaci,* onion thrips |
| *Myzus persicae,* green peach aphid | *Acrosternum hilare, Nezara viridula,* green stinkbugs |
| *Melanoplus diferentialis,* differential grasshopper | *Sericothrips variabilis,* soybean thrips |
| *Estigmene acrea,* Saltmarsh caterpillar | *Euschistus servus,* brown stink bug |
| *Sericothrips variabilis,* Soybean thrips | southern green stinkbug |
| *Epargvreus clarus,* Silverspotted skipper | Margined blister beetle |
| *Spissistilus festinus,* Threecornered alfalfa hopper | Striped blister beetle |
| | Bahia grass borer |

Plants comprising a plastid or nucleic acid construct as described herein may be used in Insect Resistance Management (IRM) strategies.

IRM strategies, generally involve planting transformed and non transformed (so called "refuge" plants) in particular structures. In these structures, the refuge plants provide a reservoir for insects that are not under insecticidal pressure and that therefore, are less likely to be selected for an insecticidal resistance allele. Crossing of these insects in the field with those insects that have be selected for a mutation forming an insecticidal resistance allele is more likely to generate heterozygote genotypes, thus decreasing the likelihood of development of insecticidal resistance, or otherwise, the time to development of resistance. Exemplary approaches for IRM are shown in www.epa.gov/oppbppd1/biopesticides/pips/bt corn refuge 2006.htm.

IRM strategies may be enhanced by the "stacking" of constructs into plants so as to target multiple pathways of the insect, thereby further decreasing the likelihood of inheritance of an insecticidal resistance haplotype. See for example Roush *et al.,* for example, outlines two-toxin strategies, also called "pyramiding" or "stacking," for management of insecticidal transgenic crops. (The Royal Society. Phil. Trans. R. Soc. Lond. B. (1998) 353, 1777-1786). Specifically, stacking or pyramiding of two or more similar (e.g. two or more different dsRNA targets) or different insecticidal actives (e.g. *Bt* or plastid RNAi) each effective against the target pests and with little or no cross-resistance can allow for use of a smaller refuge. Roush suggests that for a successful stack, a refuge size of less than 10% refuge, can provide comparable resistance management to about 50% refuge for a single (non-pyramided) trait. For currently available pyramided *Bt* corn products, the U.S. Environmental Protection Agency requires significantly less (generally 5%) structured refuge of non-*Bt* corn be planted than for single trait products (generally 20%).

There are various ways of providing the IRM effects of a refuge, including various geometric planting patterns in the fields (as mentioned above) and in-bag seed mixtures, as discussed further by Roush *et al.* (*supra*), and U.S. Patent No. 6,551,962.

The above percentages, or similar refuge ratios, can be used for the subject stacks or pyramids containing 2, 3, or more insecticidal actives. For triple stacks with three sites of action against a single target pest, one objective may be to maintain a refuge of susceptible individuals, for example 5% refuge. This is particularly true for commercial acreage - of over 10 acres for example.

Dose impacts the efficacy and reliability for resistance management ensuring that a lethal dose is available to the pest over time. In one embodiment of the invention the expression of available dsRNA is greater when located in the plastid leading to a higher dose available to the target pest. The dsRNA within the plastid remains stable throughout the growth and development of the transgenic plant maintaining the availability of the dose over time to the target pest.

The compositions and methods disclosed herein may be used together in combinations with other methods and compositions for controlling damage by pests. For example, plants, plastids, and nucleic acid constructs described herein may be used in a method comprising the additional use of one or more chemical agents effective against a coleopteran, lepidopteran, and/or hemipteran pest, biopesticides effective against a coleopteran, lepidopteran, and/or hemipteran pest, crop rotation, or recombinant genetic techniques that exhibit features different from the features of the RNAi-mediated methods and RNAi compositions of the invention (e.g., recombinant production of proteins in plants that are harmful to a coleopteran, lepidopteran, and/or hemipteran pest (e.g., *Bt* toxins)).

As used herein, except where the context requires otherwise, the term "comprise" and variations of the term, such as "comprising", "comprises" and "comprised", are not intended to exclude further additives, components, integers or steps.

Unless specifically indicated or implied, the terms "a", "an", and "the" signify "at least one" as used herein.

It will be understood that the invention disclosed and defined in this specification extends to all alternative combinations of two or more of the individual features mentioned or evident from the text or drawings. All of these different combinations constitute various alternative aspects of the invention.

## Examples

### Example 1. Nucleic acid constructs

*Construction of chloroplast transformation vector pR1*

Chloroplast transformation constructs were derivatives of the chloroplast transformation vector pPRV323Clox (formerly pPRV312L; NCBI accession DQ489715.1) (Chakrabarti et al., 2006 and Lutz et al., 2007). This vector was designed for transformation of *N. tabacum,* recombining with the trnI and trnA sequences of the *N. tabacum* chloroplast genome. It has also been used to successfully transform the related *N. benthamiana,* as the *N. tabacum* and *N. benthamiana* recombination regions are 99.52 % similar (only 10 of 2076 bp difference) (Davarpanah et al., 2009). The vector was originally designed for read-through expression (of a sequence of interest) from the upstream ribosomal RNA promoter (rrn16), or for insertion of a complete expression cassette upstream of the aadA selectable marker. The latter context was used in the present example.

pPRV323Clox was first modified by using 'mutating PCR' to correct an unmapped Kpn I restriction enzyme recognition site erroneously present 5' and adjacent to the 3' homologous recombination arm, which would have interfered with the conceived assembly plan described below. The sequence was changed as indicated: ...ATG(G>C)(TACCdel)GCT... to create the vector pPRV323Clox(k-fix), wherein the original sequence of the vector, ATGGTACCGCT was modified to ATGCGCT. A synthetic dsDNA insert was synthesized to include the sequences encoding Asc I, the rrn promoter (Prrn), the 5' untranslated region of the transcript for the *N. benthamiana* chloroplast rubisco larger subunit gene (rbcL 5' UTR), a multiple cloning site (MCS), and the rbcL 3' UTR and Not I (Genscript). The insert was delivered in a pUC57 vector, excised with Asc I and Not I and sub-cloned into pPRV323Clox(k-fix) already digested with Asc I and Not I, to create the new vector, pR1.

*Construction of the 'golden gate' dsRNA assembly vector, p32c-GG*

Large dsRNA expression units were first assembled in a secondary 'assembly' vector before being transferred as complete units into pR1. dsRNA expression units were made by a further modified version of the 'Golden Gate' (GG) method previously adapted for inverted repeat (aka hairpin) assembly (Yan et al., 2012). To set up the components for this method of assembly, the GG cloning cassette was amplified from its original vector, pRNAi-GG (NCBI accession JQ085427.1; available from the Arabidopsis Biological Resource Center (ARBC), #CD3-1786), using primers g187f and g188r (see Table 3). The PCR product was digested with Xho I and Kpn I restriction enzymes and inserted into the binary vector, p32c, already similarly digested. p32c is an in-house derived version of the pORE-03 binary vector, pre-modified to include a CaMV 35s promoter (the -343 to +1 version) and an altered multiple cloning site (MCS). p32c was also pre-modified by mutating PCR to remove a BsaI RE recognition site, GGTCTCN|NNNN (originally present in the phosphinothricin acetyltransferase II, PPT, selectable marker gene), by converting the sequence as indicated: ..GAGAG(G>A)AG(A>G)CC..., as this BsaI site would have interfered with ensuing GG hairpin assemblies. In other words, the sequence GAGAGGAGACC in the vector was modified to GAGAGAAGGCC. These changes resulted in a silent mutation, such that the nucleotide sequence was altered but the translated PPT sequence would remain unchanged. The resulting GG vector, p32c-GG, was maintained in *E. coli* DB3.1 cells, as the GG expression vector encodes the cddB gene which otherwise causes bacterial cell death in other *E. coli* lab strains (e.g. DH5alpha).

*Large dsRNA expression unit assembly in p32c-GG vector using the Golden Gate method*

Large dsRNA expression units typically encoding hairpins with stem lengths up to many 100s of base pairs were made in the p32c-GG assembly vector following the method as originally described by Yan et. al. for their similar vector pRNAi-GG, with the following exceptions (Yan et al., 2012). Ligations were performed in a PEG-supplemented ligation buffer (e.g. a 2x rapid ligation buffer) for 2 hours, the total ligation volume was increased accordingly, the final 80 °C incubation was omitted (it is incompatible with ligation buffers with PEG), and GT116 cells (Invivogen) were used in place of DH5alpha (DH5alpha cells worked, but fewer colonies were recovered compared with GT116 cells). We found the PEG-based buffer to be a vital modification, as very few or no colonies were recovered without it. Briefly, the assembly process included the following steps. The target stem was amplified by PCR using KOD polymerase (Toyobo) as per the manufacturer's protocol, with primers designed to have the following layout:
Forward primer layout = ACCA_GGTCTCA(GGAG)_bind, wherein: the sequence 'ACCA' is the 5' terminal restriction enzyme binding seat. (This is an extra 4 bases added to the terminal 5' end so as to provide some additional 'purchase' for the restriction enzyme to 'grab' onto the sequence when attempting to cut at the adjacent downstream position); '_' is simply a divider symbol to separate different functional units in the shorthand sequence when written out and not have a physical representation in the actual sequence; 'GGTCTCA(GGAG)' is the Bsa I restriction enzyme (RE) site. Unlike most RE sites, it will recognise GGTCTCA, but cut outside of this, in the 3' adjacent 4 nucleotides (NNNN). These 4 nucleotides can be any sequence, and in this example, the sequence "(GGAG)" was used and in combination with the preceding Bsa I recognition sequence is termed a type 'blue' Bsa I sequence (e.g. GGTCTCAGGAG); and 'bind' is a generic placeholder to represent whatever specific sequence is necessary to be added to the 3' end of each primer to 'bind' to the specific target sequence being amplified in the subsequent PCR, i.e. this sequence varies for every target (see for example, SEQ ID No:3 in Table 3).

Reverse primer layout = ACCA_GGTCTCA(TCGT)_bind (see for example, SEQ ID No:4 in Table 3).

Following PCR, the reactions were column-purified (Qiagen), and eluted in 30 μl Elution Buffer. The GG reaction was then set up with 50 ng of purified PCR reaction, 200 ng of vector, 5 U Bsa I, 10 U T4 ligase, 2x RAPID LIG. BUFFER (New England Biolabs), and $H_2O$ to 20 μl. The GG reaction was incubated at 37 °C for 2 hours + 5 min. at 50 °C. 3 μl of the reaction was electroporated into ~ 50 μl of a GT116 electro-competent cell mix prepared according to standard procedures. The entire transformation mix was spread onto appropriate selection medium, including kanamycin (to select for the vector) and chloramphenicol (to select for intron inclusion). Typically ~ 50 - 100's of colonies were recovered.

*Construction of the nuclear dsRNA expression control vector v153*

The nuclear dsRNA expression control vector shown in Figure 1, v153, derived from the p32c binary vector, was made as an intermediate vector on the way to assembling the corresponding chloroplast transformation vector, v206. Both vectors encoded a dsRNA expression unit, Ha-AceHp1236-189, which was designed to have a self-complementary 189 nt sense (SE) / antisense (AS) stem configuration with the stems separated by a nuclear / cytoplasmic intron-based spacer or 'loop' of ~ 1599 nt. The 189 nt stem sequence was selected to be homologous to the acetylcholinesterase gene (Ace; NCBI accession AF369793) from the lepidopteran moth, *Helicoverpa armigera* (Ha; cotton bollworm), starting at nt position 1236 (relative to the first nt of the CDS) and continuing for a total length of 189 nt in the 5' direction. The first step in the assembly was to construct the dsRNA expression unit for hp1236-189 in the vector p32c-GG, using the method as already described. For the specific assembly in example 1, the sequence corresponding to Ace1236-189 was amplified by PCR using *H. armigera* cDNA, and the primers g216f/r and g217f/r. The resultant vector, p32c-Ha-AceHp1236-189, was renamed v153, and served as the nuclear dsRNA expression control vector. This was transformed into *N. benthamiana* using standard *Agrobacterium* transformation methods and selection processes commonly known to those skilled in the art. v153 is depicted in Figure 1.A, shown in the general linearised context as it would be expected to form following integration into the nuclear genome of *N. benthamiana.* A simplified schematic of the expected dsRNA production and RNAi-type processing from this vector is shown, along with a schematic representation of the primers used for subsequent PCR assays to determine integration or expression of this vector construct *in planta.*

*Construction of the dsRNA chloroplast transformation and expression vector v206*

The chloroplast transformation vector shown in Figure 1, v206, was made from v153 using the additional vectors and methods outlined above. v206, also referred to as pR1-Ha-AceHp1236-189, was assembled from v153 by using v153 as a donor to move the dsRNA expression unit into pR1. This transfer was achieved by excising the dsRNA expression unit with Xho I and Kpn I, and sub-cloning it into pR1 already digested with Sal I (compatible cohesive ends with Xho I) and Kpn I. The resultant vector, v206, is depicted in Figure 1.CB in the context of the surrounding chloroplast genomic sequence following its precise integration into the chloroplast genome of N. benthamiana. A simplified schematic of the expected dsRNA production and RNAi-processing from this vector is shown, along with a schematic representation of the primers used for subsequent PCR assays with this vector.

*Construction of the dsRNA chloroplast transformation and expression vector v301*

The chloroplast transformation vector shown in Figure 1, v301, was made from v206 using the additional vectors and methods outlined above. v301 was assembled from v206 by replacing the PDK intron+CMr region with an 795bp chloroplastic intron sequence (atpF intron) and small flanking regions. The replacement sequence was amplified from Nicotiana benthamiana using primers BEN0002F and BEN0003R. The resultant vector, v301, is depicted in Figure 1.D in the context of the surrounding chloroplast genomic sequence following its precise integration into the chloroplast genome of N. benthamiana. A simplified schematic of the expected dsRNA production and RNAi-processing from this vector is shown, along with a schematic representation of the primers used for subsequent PCR assays with this vector.

**Example 2. Transformation of host cells and chloroplasts**

*N. benthamiana tissue culture methods*

*N. benthamiana* plants were introduced into tissue culture by first sterilizing a small volume of seed (~ 50 μl volume equivalent) with Cl- gas. An open tube of the seed was freshly gassed for ~ 1 - 4 hours in a sealed glass chamber with an open dish of 3 ml 1 N HCL added to 100 ml of hypochlorite (4 % available). The seed was spread onto Murashige-Skoog medium with 3 % (w / v) sucrose and 5 % (w / v) noble agar (MSN) (Murashige et al., 1962). Plants were maintained in 15 x 6 cm pots and transferred onto new medium every 2 - 3 weeks or as required.

*Gold micro-carrier preparation (for bombardment)*

0.6 micron gold micro-carriers (Biorad) were prepared according to the manufacturer's protocol for the Biolistic PDS-1000/He Particle Delivery System, which follows the method from Sanford et. al. (Sanford et al., 1993). Briefly, 30 mg of micro-carriers were weighed into a 1.5 ml microfuge tube, 1 ml of 70% ethanol was added, and the mixture vortexed for 3 - 5 minutes. The gold was left to settle for 15 minutes before centrifuging for 5 sec (up to ~ 20,000 rcf). The supernatant was discarded. 1 ml of sterile water was added to the gold, vortexed for 1 minute and allowed to settle for 1 minute. The gold was pelleted by spinning for 5 sec. and the supernatant removed. The water washing steps were repeated twice for 3 times in total. 500 μl of sterile 50 % glycerol was added to the final pellet (the preparation was stored at 4 °C and can be used for up to ~ 2 weeks). This volume made 10 standard preparations.

*DNA-coating of gold micro-carriers*

The gold micro-carriers were coated with DNA on the day of bombardment. The micro-carriers prepared above were vortexed for >= 5 min. to re-suspend the gold. For each construct, 50 μl (~ 3 mg) of the gold suspension was removed to a new tube whilst vortexing to avoid settling. 5 μl DNA (ideally at 1ug / μl), 50 μl 2.5 M $CaCl_2$ and 20 μl of 0.1 M spermadine were added to each aliquot of gold. If the DNA concentration was not high enough, a sufficient volume of DNA (> 5 ul) was added up to 5 ug, and the volumes of the remaining components was scaled up accordingly. Each sample was vortexed for 3 min., allowed to settle for 10 min. (longer than the manufactures stated 1 min.), and centrifuged for 5 sec. The supernatant was removed, 140 μl of 70 % ethanol was added to the pellet, and the mixture vortexed until resuspended (~ 1 min.). The micro-carriers were left to settle for 1 min., centrifuged for a further 5 sec. and the supernatant was discarded. The step was repeated, but with 140 μl of 100 % ethanol. The final pellet of micro-carriers was resuspended in 48 μl of 100 % ethanol, and stored on ice until bombardment (on the same day). This volume made >6 'shots' for use in transformation of chloroplasts.

*Transformation of chloroplasts using the particle bombardment method*

The Biolistic PDS-1000/He machine (Biorad) was moved into a laminar flow cabinet, rigorously surface sterilized with 70 % ethanol, and made ready in accordance with the manufacturer's protocol. As per the recommendations the helium was 'High Grade' (4.5; > 99.995 % purity) as lesser grades may block the machine and or contaminate the samples. Stopping screens, macro-carrier holders, and macro-carrier discs were sterilized by a brief dip in 100 % ethanol before air-drying on sterile filter paper lined dishes. 1100 psi rupture discs were dipped in 70 % Isopropanol and whilst still moist (but not overly wet) were loaded into the firing nozzle (some isopropanol is necessary for a suitable seal). The first 'shot' of each session was with a rupture disc only (i.e. no sample) so as to purge air from the lines and charge them with He. Each gold micro-carrier preparation was sonicated for > 1 min., and 6 ul / shot was evenly spread over the centre of a macro-carrier, first placed in the macro-carrier holder and allowed to air-dry (~ 1 - 5 min.). Bombardments were at 6 cm (from the nozzle) in a vacuum of ~ 25 - 28 in Hg. Leaves were bombarded abaxial side up (the bottom side), on MSN plates. Leaves were prepared up to 24 hours in advance, with either a single leaf of ~ 2 - 3 $cm^2$ (or several smaller leaves) from a tissue culture plant placed centrally on a standard 100 x 20 mm culture dish.

*Explant, callus, and plantlet selection (heteroplasmic)*

Following bombardment, the plates were immediately sealed with 2 wraps of micropore tape (3M) and kept in the dark for ~ 48 - 72 hours. After this, each leaf was cut into small segments of ~ 0.5 - 1 $cm^2$ and aseptically transferred adaxial side up (upside up) to MSN selection medium containing 500 mg / L spectinomycin, 2 mg / L BAP and 0.5 mg / L NAA (to induce callus and shoot formation). The tissue was moved onto fresh medium every 2 - 3 weeks. Untransformed tissue goes a pale yellow colour after ~ 2 - 4 weeks, and calli start to appear from ~ 4 - 8 weeks onward. Calli were detached from the the explant material when ~ 0.5 - 1 $cm^3$ and moved onto separate medium. Emergent shoots were detached from calli as early as possible and were maintained on the same medium. When the shoots had developed 1 - 2 leaves of ~ 0.5 - 1 $cm^2$, they were moved to MSN medium containing 500 mg / L spectinomycin (but without hormones) to induce rooting. After suitable root development the plantlets were moved to soil.

Transformed cells may be heteroplasmic, where only a fraction of each cell's total number of chloroplasts are transformed, or homoplasmic, where most or all of the chloroplasts in each cell are transformed. Nicotiana shoots regenerated from a bombarded leaf after a single round of selection, e.g. callus formation, shoot formation and isolation, and root induction, are thought to always be chimeric (heteroplasmic) (Maliga et al., 2012 and Maliga et al., 2004). Homoplasmy, or at least genetically stable plants, are generally considered to be achieved by 2 or more successive rounds of selections by repeatedly dividing the newly formed shoots into small pieces and re-inducing callus formation. 'Plasmy' level can be determined by a suitable PCR reaction across the insertion junction.

*DNA extraction from calli or plantlets*

Individual calli were sampled for DNA extraction by removing a small section of ~ 2 - 3 $mm^3$. Emerging plantlets destined for DNA extractions were grown to the stage of having at least 2 leaves of ~ 0.5 - 1 $cm^2$ each before a single leaf was harvested for extraction. DNA extractions were performed with a basic 'salting out' method. Each tissue sample was placed in a 200 μl PCR strip tube with a small sterile stainless steel ball and ~ 180 - 200 μl of DNA extraction buffer (0.5 M NaCl and 1 % SDS). The tubes were shaken vigorously in a mixer-mill at ~ 30 revs. / sec. for ~ 2 min. The sample plate was re-oriented by reversing and flipping and the plate was shaken again. The tubes were centrifuged using a plate centrifuge at 3800 rpm for 1 hour. 100 μl of supernatant was transferred to a fresh plate with 200 μl of 100 % ethanol. The sample plate was re-centrifuged as before. The supernatant was discarded by firmly inverting the sample plate. The sample plate was centrifuged upside down for ~ 20 sec. at ~ 300 rpm. 200 μl of 70% ethanol was added to each well (a quick wash), and the supernatant was again discarded by firmly inverting the sample plate. The sample plate was again centrifuged upside down for ~ 20 sec. at ~ 300 rpm. The sample plate was air-dried at room temperature for ~ 20 min. and the pellet was resuspended in 100 μl of T.E. buffer and stored at 4 °C.

*Generation of chloroplast lines*

Nine *N. benthamiana* plant lines were generated using the v206 chloroplast transformation (Sample #s 1 to 9). Each selected line was from an independent transformation event. Each line was resistant to spectinomycin, and the incorporation of v206 into the chloroplast genome was confirmed by PCR. Untransformed *N. benthamiana* was maintained as a negative, or untransformed control (sample #24), and a nuclear transformed line of v153 was created as indicated previously, and maintained as a positive nuclear transformed control (sample #17).

**Example 3. Confirmation of transgene expression using RT-PCR**

*RNA extraction and DNase treatment*

Total RNA was extracted using Trizol (Life Technologies) according to the manufacturer's protocol. Tissue for RNA extraction was similarly harvested as that previously described for DNA extraction. Tissue was snap frozen and ground with a mortar and pestle in liquid $N_2$. Approximately 1.5 ml of Trizol was added to ~ 0.5 - 1 ml equivalent volume of finely powdered tissue. All other volumes in the standard method were scaled accordingly (to the 1.5 ml of Trizol used). The optional isolation step was included, wherein the initial homogenate (immediately following homogenization) is centrifuged at 12,000 x g for 10 min. at ~ 4°C. The pellet was discarded and the cleared homogenate further processed as per the protocol. Each sample was re-suspended in ~ 30 - 50 μl of RNase-free water (e.g. DEPC treated). Following extraction, the RNA samples were DNase treated in accordance with the manufactures protocol (New England Biolabs), to reduce the likelihood of false-positive amplifications in subsequent reverse transcription tests. Briefly, 8 μl of RNA sample was combined with 1 μl of DNase buffer and 1 μl of RQ1 DNase enzyme (NEB). The reaction was incubated at 37 °C for ~ 30 min. at which point 1 μl of stop solution was added and the mix further incubated at 65 °C for ~ 10 min. to fully inactive the DNase.

*RT-PCR*

Each sample was subjected to reverse-transcription PCR (RT-PCR) to test for the presence of the long dsRNA species. The RT-PCR used was a 2 step RT-PCR, where a single cDNA synthesis step was conducted first, followed by separate PCR reactions for different RNA species. Each cDNA synthesis reaction was conducted with the Superscript III First Strand Synthesis kit (Invitrogen), according to the manufacturer's protocol. The specific user included components were 5 μl of mixed primers (1 μl each of g280r, g276r, g216f/r, g301r and oligo dT), 1 μl of dNTPs, and 7 μl of DNase treated RNA sample to a total user input volume of 13 μl. Following cDNA synthesis, 1 μl of each sample was used as template in standard 20 ul PCR reactions. PCRs were conducted on samples v206 chloroplast samples #1-9, the v153 nuclear control #17, and the untransformed *N. benthamiana* control #24. Each sample was amplified with 3 different primer sets, using 2 different template conditions: 1) the extracted RNA before cDNA synthesis as the template (to test for the possibility of DNA contamination in the samples), and 2) the corresponding cDNA sample sets. The first PCR primer set was designed to amplify across the intron and rear stem junction to test for the presence of the large RNA species containing both the intron loop at the AS stem (primers g25f and g216f/r). The second set was internal to the dsRNA stem region, but binding at the extremities of the stem to test for the presence of the full length SE or AS stem independent of the loop sequence (primers g216f/r and g217f/r). The third set was a control reaction to test for the presence of the ubiquitous house-keeping sequence for eIF4E (primers g165f and g166r).

As shown in Figure 2, all 9 chloroplast cDNA samples were positive for the intron to rear stem PCR, confirming the expression of RNA species containing at least both sections (Figures 2A and B). Since the intron and AS stem sections are downstream of the SE stem, this result indicates the presence of the full-length dsRNA forming region. There was no amplicon from the PCR reaction containing cDNA from *N. benthamiana* transformed with the v153 nuclear expression control vector. This would be expected if the intron is efficiently spliced out of the RNA during or immediately following transcription in the plant. There was no amplicon from the PCR reaction comprising cDNA from control, untransformed *N. benthamiana*. This was also as expected since *N. benthamiana* does not express the Ha-AceHp1236-189 dsRNA sequence. The 'no-RT' PCR reactions (i.e. reactions where only RNA was used as template prior to cDNA synthesis) showed no amplicons confirming that there was no detectable level of DNA contamination influencing the formation of specific product seen in the cDNA sample sets.

All 9 chloroplast cDNA samples were also positive for the dsRNA / hp PCR specific for the stem region (Figures 2C and D), further confirming that at least the SE and or AS stem is present in its entirety for each sample. Chloroplast transformation sample v206 #8, exhibited a small amount of product in the no RT control reaction, though this was much lower than that seen in the corresponding cDNA reaction. There was no amplicon from PCR reactions containing cDNA from the v153 nuclear expression control, as would be expected if the nuclear expressed dsRNA region / hairpin was efficiently processed down into smaller siRNA-sized species of ~19 - 24 bp immediately following transcription in the plant, and in so doing reducing amount of expressed long dsRNA to below detectable levels. There was no amplicon from the PCR reaction containing cDNA from the *N. benthamiana* untransformed control, as expected.

The chloroplast cDNA samples and the 2 control cDNA samples were all positive for the eIF4E house-keeping sequence (Figures 2E and F). This result confirmed that all cDNA samples were suitable templates for PCR, and thus supporting the interpretation that v153 sample has no detectable long dsRNA as it is mostly likely efficiently processed down by RNAi pathways, as is expected. The 'no-RT' control reactions had no detectable product in any sample, further showing that the samples were free of significant DNA contamination.

### Example 4. Quantification of dsRNA in chloroplast transformants and controls using Northern blotting

The chloroplast transformation samples were further subjected to high molecular weight (MW) or 'large RNA' Northern blot analysis to detect and quantify the relative amounts of large dsRNA present in the chloroplast transformed samples relative to the corresponding nuclear-expressed control sample (and the untransformed control). RNA extractions were re-prepared for each of the samples #1-9, 17 and 24 according to the method described previously, with the exception that each sample was re-suspended in formamide rather than RNase-free water. (Sample #6 was omitted in this example set as there was insufficient material remaining for additional RNA extractions.) Approximately 10 μl of each sample + 6 μl of formamide RNA loading dye was loaded per lane (except for sample 7, where only 4 μl of sample was available) onto a 1.4 % TBE agarose gel and run at 100 V until the 2 lower dye fronts were suitably separated. The gel was stained with ethidium bromide (EtBr) and visualized to gauge relative loading amounts (Figure 3.A). The gel was capillary transferred overnight onto Hybond N+ (Amersham), and the samples were UV cross-linked to the membrane. The membrane was pre-hybridized and hybridized in with PerfectHyb at 65 c. The probe was a partial length SE strand UTPp32 riboprobe run off SP6 from a reverse inserted HaAce1236 pGEM vector (v158); in other words, the probe was to detect the anti-sense of the target sequence, or the AS stem of the dsRNA. The probe length was 538 nt with ~ 357 nt homologous to HaAce which spanned the entire 189 nt of the target incorporated into the chloroplast and nuclear transformed plants. The membrane was exposed for 1 hour.

As can be seen in Figure 3.B, many of the chloroplast transformed samples possessed large amounts of specific high molecular weight RNA corresponding in size to that expected for the HaAce1236-189 dsRNA sequences if they remained unprocessed by the RNAi-type machinery. The corresponding nuclear expression control, sample #17, showed no evidence of unprocessed long dsRNA, with a background signal level similar to the untransformed control. The RNA amounts were quantified using ImageJ, and were normalised to the amounts loaded (using the EtBr image). The relative expression levels were calculated as normalised fold changes in band intensity relative to the *N. benthamiana* untransformed control (set as 1). As can be seen in Figure 3.C, all v206 chloroplast samples had detectable amounts of specific dsRNA. There was some variation between the lines, which was not unexpected given the independent nature of each line. Many independent lines in this example, notably 1, 2, 4, 5, 8 and 9, exhibited quite high levels of dsRNA production and accumulation. When normalised, it was apparent that the v153 nuclear expression control sample retained no detectable amount of dsRNA, a result in-line with the previous RT-PCR data.

### Example 5. Quantification of siRNA in chloroplast transformants and controls using Northern blotting

The chloroplast transformation samples were further subjected to low molecular weight (MW) or 'small RNA' Northern analysis to detect and quantify the relative amounts of siRNA-sized processed products as would be expected to be present if the dsRNA in the samples were exposed to the RNAi-type processing machinery. The same RNA extractions as used in example 4 were used (Samples #6 and 7 were omitted in this example set as there was insufficient material remaining.). Approximately 25 μl of each sample + 25 μl of formamide RNA loading dye was loaded per lane onto a 17 % PAGE gel. This resulted in an estimated range of amount loaded of ~ 18 - 42 μg. Prior to loading, the PAGE gel was pre-run at 150 V / 40 min. Once loaded the gel was run at 150V / ~30 min. + 200 V /~4-5 hours (until the dye front was suitably migrated). The gel was stained with ethidium bromide (EtBr) and visualized to gauge relative loading amounts (Figure 4.A). The gel was electro-transferred at 45 V / 60 min. onto Hybond N+ (Amersham), and the samples were UV cross-linked to the membrane. The membrane was pre-hybridized and hybridized in with PerfectHyb at 42 °C. The probe was prepared that same as described previously, except it was also 'carbonated' by standard methods to fractionate it into ~ 50 nt fragments. The membrane was exposed for 1 hour and 60 hours, with no difference in the detection limit between the 2 time points (the 1 hour time point showed herein).

Figure 4.B, shows that none of the chloroplast transformed samples showed any sign of small RNA species specific for the probe. The v153 nuclear expression control sample (#17), however, showed strong and clear products corresponding to the expected size range of ~ 19 - 24 nt. The untransformed *N. benthamiana* control showed no specific-binding in the small RNA size range, as expected. The RNA amounts were quantified using ImageJ, Figure 4.C, and were normalised to the amounts loaded (using the EtBr image). The relative expression levels were calculated as normalised fold changes in band intensity relative to the *N. benthamiana* untransformed control (set as 1). Quantification further confirms that the interpretation of the Northern image, showing that no chloroplast sample shows evidence of small RNA species being produced, whereas the v153 nuclear expression control sample possesses high levels of small RNAs.

### Example 6. Chloroplast Transformation of Crop Species

Maize is transformed substantially as generally described herein and in U.S. Patent Application No. 20080289063A1. Tobacco, carrot, cotton, soybean and edible leafy crops (*e.g.* lettuce) transformations are conducted as generally described herein and in Verma and Danielle (2007) Plant Physiol. 145(4): 1129-1143. Rice, wheat, sorghum, sunflower, canola, sugar beet, and barley can be transformed following protocols as found in Maliga (2014) Chloroplast Biotechnology, Humana Press. Those skilled in the art of plant transformation may make modifications to the protocols based on the crop species.

**Example 7a. Insecticidal Activity**

Transplastomic plants with chloroplasts expressing dsRNA were tested for insecticidal activity in bioassays conducted with neonate Helicoverpa armigera larvae on seedlings of transgenic and wildtype Nicotiana benthamiana. Helicoverpa larvae were hatched from eggs (obtained from a colony maintained by a commercial insectary; AgBiTech, Toowoomba, QLD) on artificial diet and within a few hours of eclosion, individual larvae are picked up with a moistened camel hair brush and deposited (20 individuals/petridish) onto washed 6 day-old seedlings in plastic petri dishes. The petridishes containing seedling infested with the larvae were then sealed with a lid allowing gas exchange. The petridishes were held under controlled environmental conditions (28 °C, ~60% Relative Humidity, 16:8 [Light:Dark]) for 4 days, after which the weight of surviving larvae were recorded (Figure 5A). Fresh seedlings of the appropriate genotype were added each day to the petridishes during the 4 day feeding period. This data shows that in all the four independent lines transformed with v206 (expressing hpRNA in the chloroplast), the larvae were significantly reduced ($p<0.005$ n= 7 - 13) in their growth rate compared to those fed on wild type N.benthamiana seedlings. The growth rate of larvae fed on two lines transformed with v153 (expressing hpRNA in the nucleus/cytoplasm) were not significantly different ($p>0.9$; n =4-7) to those fed on wt.

**Example 7b. Insecticidal Activity**

Insecticidal activity can be measured by various bioassays as known to those skilled in the art including but not limited to those described below. Lepidopteran insect pests include but are not limited to *Helicoverpa* sp., *Agromyza parvicornis, Agrotis ipsilon, Diatraea* sp., *Elasmopalpus lignosellus, Ostrinia nubilalis, Spodoptera* sp., *Papaipema nebris, Heliothis* sp., *Pectinophora gossypiella, Plutella xylostella, Mamestra configurata, Pseudoplusia includens, Plathypena scabra, Anticarsia gemmatalis, Colias eurytheme, Achoroia grisella, Acleris gloverana, Acleris variana, Adoxophyes orana, Alabama argillacea, Alsophila pometaria, Amyelois transitella, Anagasta kuehniella, Anarsia lineatella, Anisota senatoria, Antheraea pernyi, Archips* sp., *Argyrotaenia* sp., *Athetis mindara, Bombyx mori, Bucculatrix thurberiella, Cadra cautella, Choristoneura* sp., *Cochylls hospes, Corcyra cephalonica, Cydia latiferreanus, Cydia pomonella, Datana integerrima, Dendrolimus sibericus, Desmia feneralis, Diaphania hyalinata, Diaphania nitidalis, Ennomos subsignaria, Eoreuma loftini, Esphestia elutella, Erannis tilaria, Estigmene acrea, Eulia salubricola, Eupocoellia ambiguella, Eupoecilia ambiguella, Euproctis chrysorrhoea, Euxoa messoria, Galleria mellonella, Grapholita molesta, Harrisina americana, Hemileuca oliviae, Homoeosoma electellum, Hyphantia cunea, Keiferia lycopersicella, Lambdina fiscellaria fiscellaria, Lambdina fiscellaria lugubrosa, Leucoma salicis, Lobesia botrana, Loxostege sticticalis, Lymantria dispar, Macalla thyrisalis, Malacosoma* sp., *Mamestra brassicae, Manduca quinquemaculata, Manduca sexta, Maruca testulalis, Melanchra picta, Operophtera brumata, Orgyia* sp., *Paleacrita vernata, Papilio cresphontes, Phryganidia californica, Phyllonorycter blancardella, Pieris napi, Pieris rapae, Platynota flouendana, Platynota stultana, Platyptilia carduidactyla, Plodia interpunctella, Pontia protodice, Pseudaletia unipuncta, Sabulodes aegrotata, Schizura concinna, Sitotroga cerealella, Spilonta ocellana, Thaurnstopoea pityocampa, Ensola bisselliella, Trichoplusia hi, Udea rubigalis, Xylomyges curiails,* and *Yponomeuta padella.*

Transplastomic plants with chloroplasts expressing dsRNA are tested for insecticidal activity in bioassays conducted with neonate lepidopteran larvae on artificial insect diet. Larvae of lepidopteran are hatched from eggs obtained from a colony maintained by a commercial insectary (Benzon Research Inc., Carlisle, PA).

These bioassays are conducted in 128-well plastic trays specifically designed for insect bioassays (C-D International, Pitman, NJ). Each well contains multi-species Lepidoptera diet (Southland Products, Lake Village, AR). Macerated plant material from transplastomic events is incorporated into the diet.

Within a few hours of eclosion, individual larvae are picked up with a moistened camel hair brush and deposited on the treated diet, one larva per well. The infested wells are then sealed with adhesive sheets of clear plastic, vented to allow gas exchange (C-D International, Pitman, NJ). Bioassay trays are held under controlled environmental conditions (28 °C, ~60% Relative Humidity, 16:8 [Light:Dark]) for 5 days, after which the total number of insects exposed to each sample, the number of dead insects, and the weight of surviving insects are recorded. Percent mortality and percent growth inhibition are calculated for each treatment. Growth inhibition (GI) is calculated as follows:

$$GI = [1 - (TWIT/TNIT)/(TWIBC/TNIBC)]$$

where TWIT is the Total Weight of Insects in the Treatment,

TNIT is the Total Number of Insects in the Treatment

TWIBC is the Total Weight of Insects in the Background Check (Buffer control), and

TNIBC is the Total Number of Insects in the Background Check (Buffer control).

The $GI_{50}$ (Growth Inhibition) is defined as the dosage at which the mean growth (*e.g.*, live weight) of the test insects is 50% of the mean value seen in Background Check samples. The statistical analysis is done using JMP™ software (SAS, Cary, NC). The $LC_{50}$ (Lethal Concentration) is defined as the dosage at which 50% of the test insects are killed.

For some species bioassays are conducted in 32-well test trays. Approximately 5mL of a 2% water-agar solution is applied to each well and the agar is allowed to solidify completely.

Plants are approximately 3 weeks old and tested at T1 generation. Three replicates of T1 leaf material are completed. One leaf is cut (2.54 cm X 1.27 cm rectangular) and placed in a single well of the tray. Each well is infested with 10 individual insect larvae.

The infested wells are sealed with adhesive sheets of clear plastic, vented to allow gas exchange (C-D International, Pitman, NJ). Trays are placed in a conviron incubator and maintained at 28 °C (16:8h light:dark, 60% RH) for 3 days, after which the total amount of damage to each leaf piece (0, 5, 10, 15, 25, 50, 75% damage, etc., up to 100%) is recorded.

**Example 8: Bioassay of transgenic plants.**

Bioactivity of the dsRNA produced in transplastomic plant cells is demonstrated by methods known to those skilled in the art (see, for example Huang et al., 2006 Econ. Entomol. 99: 194-202). Efficacy may be demonstrated by feeding various plant tissues or tissue pieces derived from a plant producing the dsRNA to target insects in a controlled feeding environment. Alternatively, dsRNA extracts may be prepared from various plant tissues derived from a plant producing the dsRNA and incorporated in an artificial diet bioassay as previously described herein. It is to be understood that the results of such feeding assays are to be compared to similarly conducted bioassays that employ appropriate control tissues from host plants that do not produce the dsRNA or to other control samples.

The biological activity of various events produced in plants from a construct is tested for preventing leaf damage caused by the feeding activity of lepidopteran insects.

A 1.0 X 0.5 inch square leaf cutting is taken in duplicate from the V3 or V4 leaf of V5 (in the case of maize) $T_0$ transgenic plants. For each test, wild type plant leaf tissues are sampled first to prevent cross contamination on the leaf edges, followed by the transformed plants. In each bioassay tray well (32-well trays and lids, CD International, Pitman, NJ), 1 leaf cutting is placed on 2% water-agar (Fisher Scientific, Fair Lawn, NJ) and this is replicated 2 times per insect species, per event and per construct. Each well is infested with ten lepidoptera neonates and sealed with a plastic perforated lid to allow for air exchange. The trays are placed at 28 °C (16:8 hr light:dark, 40% RH) and after 3 days they are graded for percent leaf damage. The percent leaf damage data is analysed with ANOVA and mean separations with the Tukey-Kramer test when variances are homogenous by using JMP® Pro 9.0.1 (2010 SAS Institute Inc., Cary, NC).

Table 3: Vector constructs used in this work

| Vector name: | Description |
|---|---|
| pPRV323Clox (formerly pPRV312L), | A basic chloroplast transformation vector, (NCBI accession DQ489715.1), designed for read-through expression of recombinant sequences. |
| pPRV32 3Clox (k-fix) | A modification of pPRV323Clox in which an unmapped additional Kpn I site was removed. |
| pR1 | A modification of pPRV323Clox (k-fix) that includes additional sequences (chloroplast promoter, 5' UTR, MCS, and 3' UTR) for discrete / self-contained transgene expression in the chloroplast. |
| pORE-03 | A basic plant 'binary' vector for agrobacterium mediated transformation. |
| p32c | A modification of pORE-03 incorporating a CaMV 35s promoter and MCS for transgene expression. A Bsa I RE site was also removed from the backbone without altering the function of any part of the vector. |
| pRNAi-GG | A basic Golden Gate (GG) reaction assembly vector for GG-based assembly of hairpins. |

(continued)

| Vector name: | Description |
|---|---|
| p32c-GG | A modification of p32c incorporating the GG assembly section from pRNAi-GG so as to allow GG-based assembly of hairpins within the p32c backbone. |
| v153 (p32c-HaAceH p1236-189) | A modification of p32c-GG wherein the hairpin targeting Ha-Ace1236-189 has been assembled. |
| v206 (pR1-HaAceH p1236-189) | A modification of pR1 wherein the GG-assembled hairpin targeting Ha-Ace1236-189 was introduced. |
| v301 | A modification of v206 wherein the assembled hairpin targeting HaAce1236-189 was modified to replace the nuclear PDK intron (+CMr) with the chloroplastic atpF intron. |
| v158 | A pGEM-based vector containing the Ha-1236-189 sequence to act as a template for making probes for Northern analysis of Ha-1236-189 related sequences. |

Table 4: Oligonucleotides sequences used in this work

| SEQ ID NO: | Name | Sequence (5'-3') | Description |
|---|---|---|---|
| SEQ ID NO: 1 | g187f | CGCTCGAGCGGAGTGAGACCAAT TCTCG | forward primer to move the GG cassette into p32c |
| SEQ ID NO: 2 | g188r | CGGGTACCCGGAGTGAGACCCTG TGTAT | reverse primer to move the GG cassette into p32c |
| SEQ ID NO: 3 | g216f/r | ACCAGGTCTCAGGAGCATGGAGT TGGATGACAGGA | hp1236-189 forward primer, Bsa I ends. |
| SEQ ID NO: 4 | g217f/r | ACCAGGTCTCCTCGAGCTGACGGG AATCCCAATATG | hp1236-189 reverse primer, Bsa I ends. |
| SEQ ID NO: 5 | g25f | TGATAGATCATGTCATTGTG | Ft. PDK intron reverse primer. |
| SEQ ID NO: 6 | g165f | TGAAGTAGAGAAACCGGCGT | N.b. eIF4E forward primer. |
| SEQ ID NO: 7 | g166r | CCAAGCAGCCTGTTTCGATT | N.b. eIF4E reverse primer. |
| SEQ ID NO: 8 | g280r | AACGACCCAATTGCAAGAGCGGA GCTCTACCAACTGAGCTATATCCC CCCGAGCCAAGTGGAGCGCATTT AAATAGTGTACCATAAC | 3' homologous recombination region reverse primer |
| SEQ ID NO: 9 | g276r | CGAAGGATAATTACTTGAGTTC | rbcL 3' UTR reverse primer. |
| SEQ ID NO: 10 | g301r | TACTCCTACTCATAATGTTTTGTTA CTACTCATAACTGTTTCTTACTCTT TGATATTATTTGTTACTTTAGC | Ft. PDK intron forward primer. |
| SEQ ID NO: 11 | BEN0002F | GCATGCAAATCTAAGTGTAGTGCT | atpF intron and small flanking sequences. Forward primer |
| SEQ ID NO: 12 | BEN0003R | CATATGTCGAATAGTATTCAAGAT CCT | atpF intron and small flanking sequences. Reverse primer. |

The invention also pertains to the following:

1. A plastid of a vascular plant, the plastid comprising:

- a nucleic acid construct including:

  - a coding region for encoding a double stranded RNA (dsRNA) molecule;
  - a promoter operable in the plastid for enabling production of the dsRNA molecule;

- wherein the dsRNA has a sequence enabling the production of siRNA therefrom.

2. The plastid of item 1, further comprising dsRNA having a sequence enabling the production of siRNA therefrom, wherein said dsRNA is encoded by the coding region.
3. The plastid of item 1 or 2 wherein the coding region has a nucleotide sequence that is not found in the genome of the plant.
4. The plastid of item 3 wherein the coding region has a nucleotide sequence of a pest or pathogen of the plant.
5. The plastid of item 1 wherein siRNA molecules are not formed inside the plastid.
6. The plastid of item 1 wherein the dsRNA contains substantially all of the nucleotide sequence encoded by the coding region.
7. The plastid of item 6 wherein the dsRNA is subjected to editing, splicing, or capping by the plastid.
8. The plastid of any one of the preceding items, further including at least one further coding region for encoding at least one insecticidal agent.
9. The plastid of item 8 wherein the insecticidal agent is an insecticidal polypeptide.
10. A nucleic acid construct for transformation of a vascular plant, comprising:

- a coding region for encoding a double stranded RNA (dsRNA) molecule;
- a promoter operable in the plastid for enabling production of the dsRNA molecule;
- one or more integration sites selective for integration of the construct into a plastid genome of a vascular plant;

    - wherein the dsRNA has a sequence enabling the production of siRNA therefrom.

11. The construct of item 10 wherein the coding region has a nucleotide sequence that is not found in the genome of the plant.
12. The construct of item 10 wherein the coding region has a nucleotide sequence of a pest or pathogen of the plant.
13. The construct of any one of items 10 to 12 wherein the construct includes at least one further coding region for encoding at least one insecticidal agent.
14. The construct of item 13 wherien the insecticidal agent is an insecticidal polypeptide.
15. A plant containing a plastid or construct of any one of the preceding items.
16. The plant of item 15 wherein the plant is substantially homoplasmic for the plastid of item 1.
17. The plant of item 15 or 16 wherein the plant further includes:

- at least one further nucleic acid construct for encoding an insecticidal polypeptide; and/or
- at least one further nucleic acid construct for encoding an insecticidal RNA.

18. The plant of item 17 wherein the at least one further nucleic acid construct is located in a nucleus of a cell of the plant.
19. Propagating material derived from a plant of item 18.
20. A method of accumulating dsRNA in a cell of a vascular plant, the method including:

- transforming a plastid of a vascular plant with a nucleic acid construct of any one of the preceding items;
- providing conditions to the cell for production of dsRNA in the plastid from the nucleic acid construct.

21. The method of item 20 wherein the dsRNA molecule is stably integrated into the genome of a chloroplast of the plant cell.
22. The method of item 20 wherein the nucleic acid construct includes a selectable marker.
23. The method of item 20 further comprising using the selectable marker of the nucleic acid construct to select for plant cells or a plant being substantially homoplasmic for a nucleic acid construct encoding a dsRNA molecule.
24. A method of controlling insect pests comprising presenting to said pests or to the environment of said pests a plant including a plastid or nucleic acid construct according to any one of the preceding items.
25. The method of item 24 wherein the environment in which control of insect pests is required is a crop or field of plants.
26. A crop or field including:

- plants including a plastid or nucleic acid construct according to any one of the preceding items; or
- plants including a plastid or nucleic acid construct according to any one of the preceding items and non chloroplast transformant refuge plants in the form of plants that do not include a plastid or nucleic acid construct according to any one of the preceding items.

## Claims

**1.** A vascular plant containing a plastid, the plastid comprising:

- a nucleic acid construct including:
  - a coding region for encoding a double stranded RNA (dsRNA) molecule;
  - a promoter operable in the plastid for enabling production of the dsRNA molecule;
- wherein the dsRNA has a sequence enabling the production of siRNA therefrom.

**2.** The plant of claim 1, further comprising dsRNA having a sequence enabling the production of siRNA therefrom, wherein said dsRNA is encoded by the coding region.

**3.** The plant of claim 1 or 2, wherein the coding region of the construct has a nucleotide sequence that is not found in the genome of the plant.

**4.** The plant of claim 3, wherein the coding region of the construct has a nucleotide sequence of a pest or pathogen of the plant.

5. The plant of claim 1, wherein siRNA molecules are not formed inside the plastid.

6. The plant of claim 1, wherein the dsRNA contains substantially all of the nucleotide sequence encoded by the coding region of the construct.

7. The plant of claim 6, wherein the dsRNA is subjected to editing, splicing, or capping by the plastid.

8. The plant of any one of the preceding claims, further including at least one further coding region for encoding at least one insecticidal agent, wherein the insecticidal agent is an insecticidal polypeptide or polynucleotide.

9. A vascular plant containing a nucleic acid construct, the construct comprising:

- a coding region for encoding a double stranded RNA (dsRNA) molecule;
- a promoter operable in a plastid for enabling production of the dsRNA molecule in a plastid;
- one or more integration sites selective for integration of the construct into a plastid genome of a vascular plant;
- wherein the dsRNA has a sequence enabling the production of siRNA therefrom.

10. The plant of claim 9, wherein the coding region of the construct has a nucleotide sequence that is not found in the genome of the plant.

11. The plant of claim 9, wherein the coding region of the construct has a nucleotide sequence of a pest or pathogen of the plant.

12. The plant of any one of claims 9 to 11, wherein the construct includes at least one further coding region for encoding at least one insecticidal agent, wherein the insecticidal agent is an insecticidal polypeptide or polynucleotide.

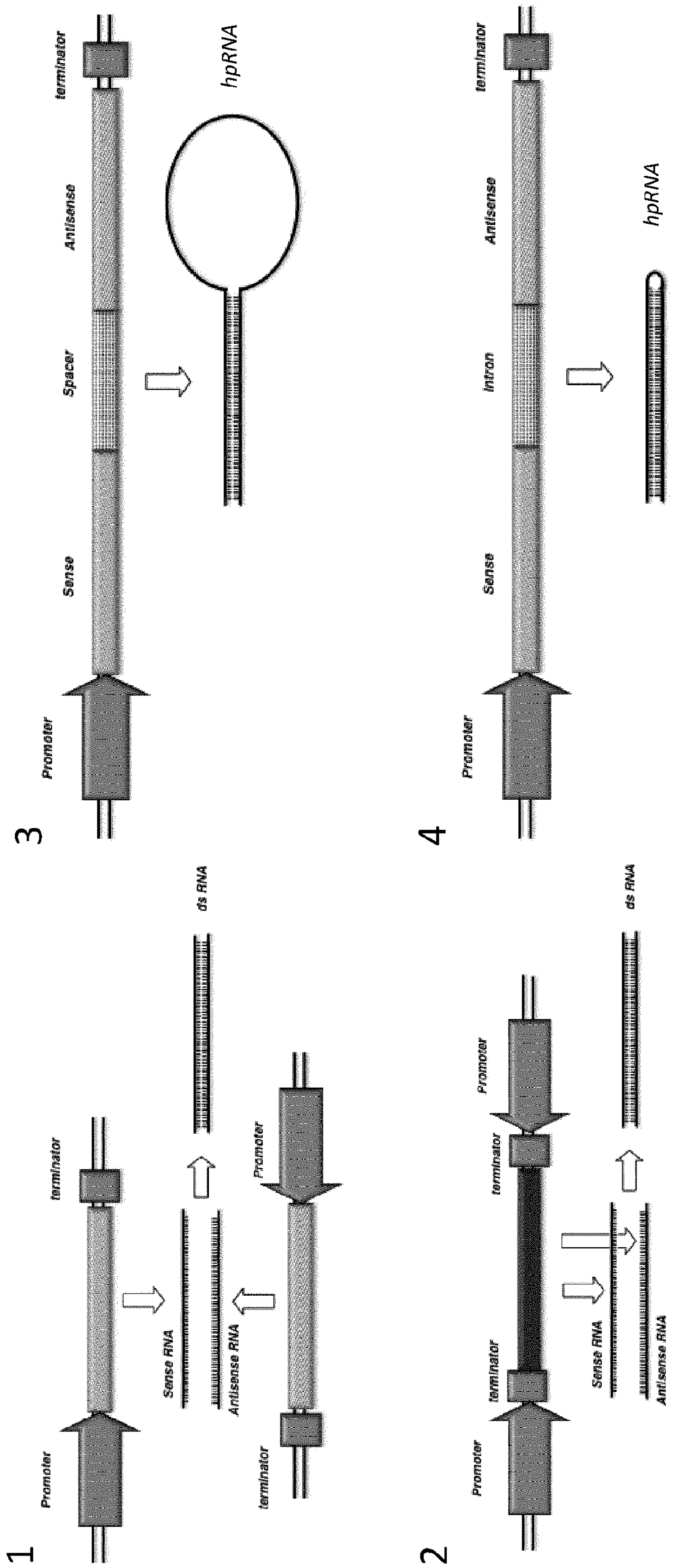
1
Promoter
terminator
Sense RNA
Antisense RNA
ds RNA
Promoter
terminator
2
Promoter
terminator
Sense RNA
Antisense RNA
terminator
Promoter
ds RNA
3
Promoter
Sense
Spacer
Antisense
terminator
hpRNA
4
Promoter
Sense
Intron
Antisense
terminator
hpRNA

Figure 1A

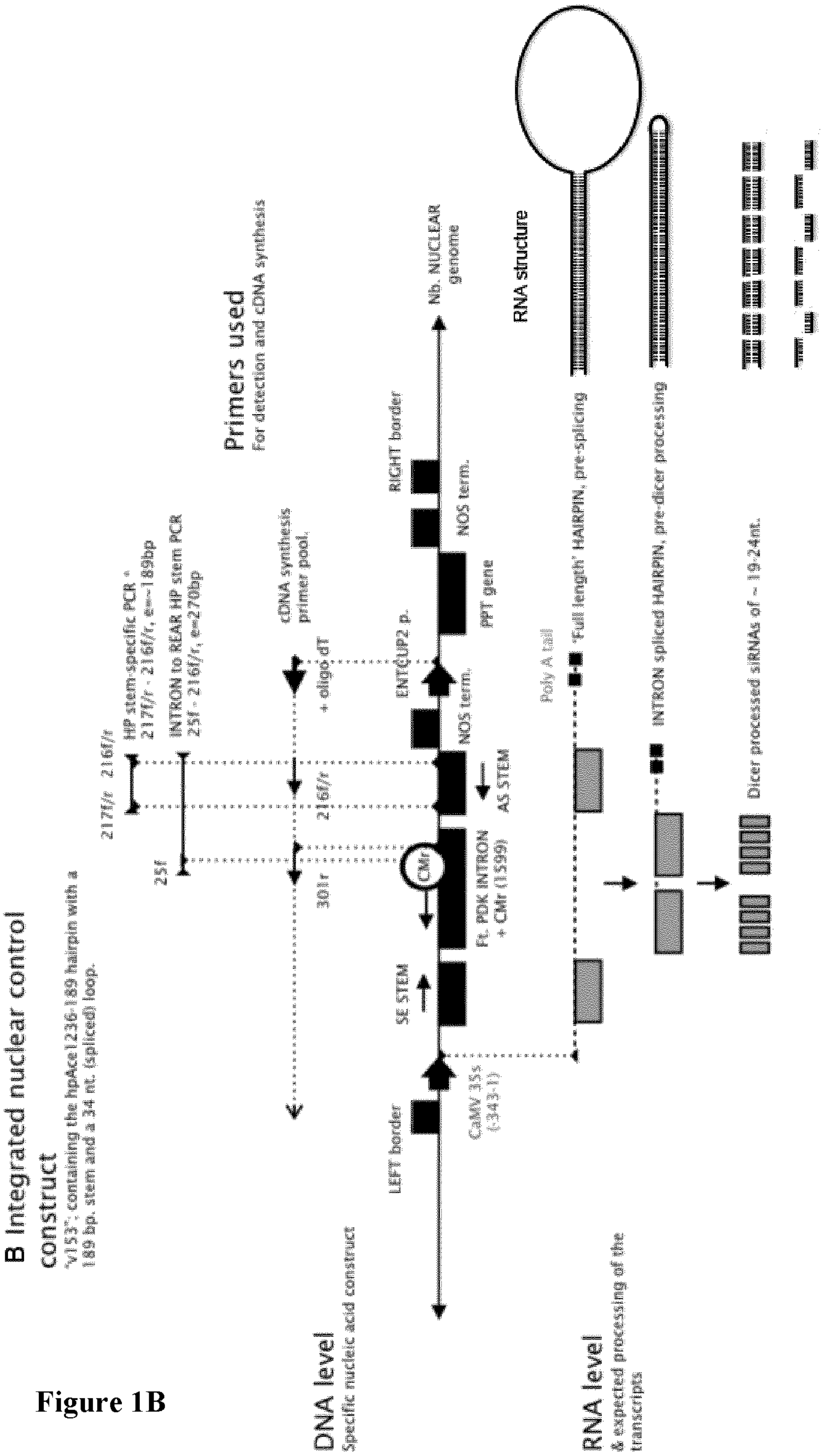
B Integrated nuclear control construct
"v153": containing the hpAce1236-189 hairpin with a 189 bp. stem and a 34 nt. (spliced) loop.
Primers used
For detection and cDNA synthesis
217f/r
216f/r
HP stem-specific PCR *
217f/r - 216f/r, e=~189bp
INTRON to REAR HP stem PCR
25f - 216f/r, e=270bp
25f
cDNA synthesis
primer pool.
30f/r
216f/r
+ oligo dT
DNA level
Specific nucleic acid construct
LEFT border
CaMV 35s
(-343-1)
SE STEM
Ft. PDK INTRON
+ CMr (1599)
CMr
AS STEM
NOS term.
ENTCUP2 p.
PPT gene
NOS term.
RIGHT border
Nb. NUCLEAR
genome
RNA level
& expected processing of the transcripts
Poly A tail
'Full length' HAIRPIN, pre-splicing
INTRON spliced HAIRPIN, pre-dicer processing
Dicer processed siRNAs of ~ 19-24nt.
RNA structure

Figure 1B

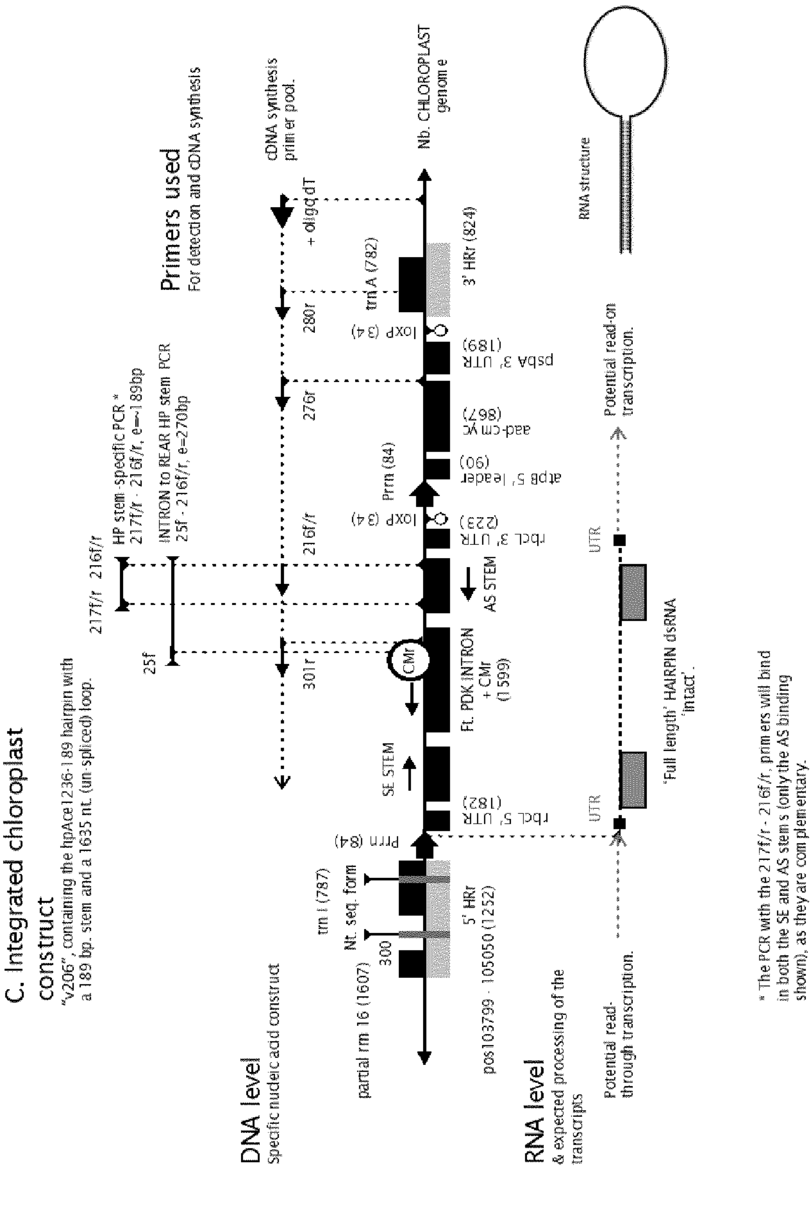
C. Integrated chloroplast construct
"v206", containing the hpAce1236-189 hairpin with a 189 bp. stem and a 1635 nt. (un-spliced) loop.
Primers used
For detection and cDNA synthesis
HP stem-specific PCR * 217f/r - 216f/r, e=~189bp
INTRON to REAR HP stem PCR 25f - 216f/r, e=270bp
217f/r 216f/r
25f
301r
216f/r
276r
280r
+ oligo dT
cDNA synthesis primer pool.
DNA level
Specific nucleic acid construct
partial rrn 16 (1607)
300
Nt. seq. form
trn I (787)
Prrn (84)
SE STEM
CMr
AS STEM
loxP (34)
Prrn (84)
loxP (34)
trnA (782)
Nb. CHLOROPLAST genome
pos103799 - 105050 (1252)
5' HRr
rbcL 5' UTR (182)
Ft. PDK INTRON + CMr (1599)
rbcL 3' UTR (223)
atpB 5' leader (90)
aad-cmyc (867)
psbA 3' UTR (189)
3' HRr (824)
RNA level
& expected processing of the transcripts
Potential read-through transcription.
UTR
'Full length' HAIRPIN dsRNA 'intact'.
UTR
Potential read-on transcription.
RNA structure
* The PCR with the 217f/r - 216f/r, primers will bind in both the SE and AS stems (only the AS binding shown), as they are complementary.

**Figure 1C**

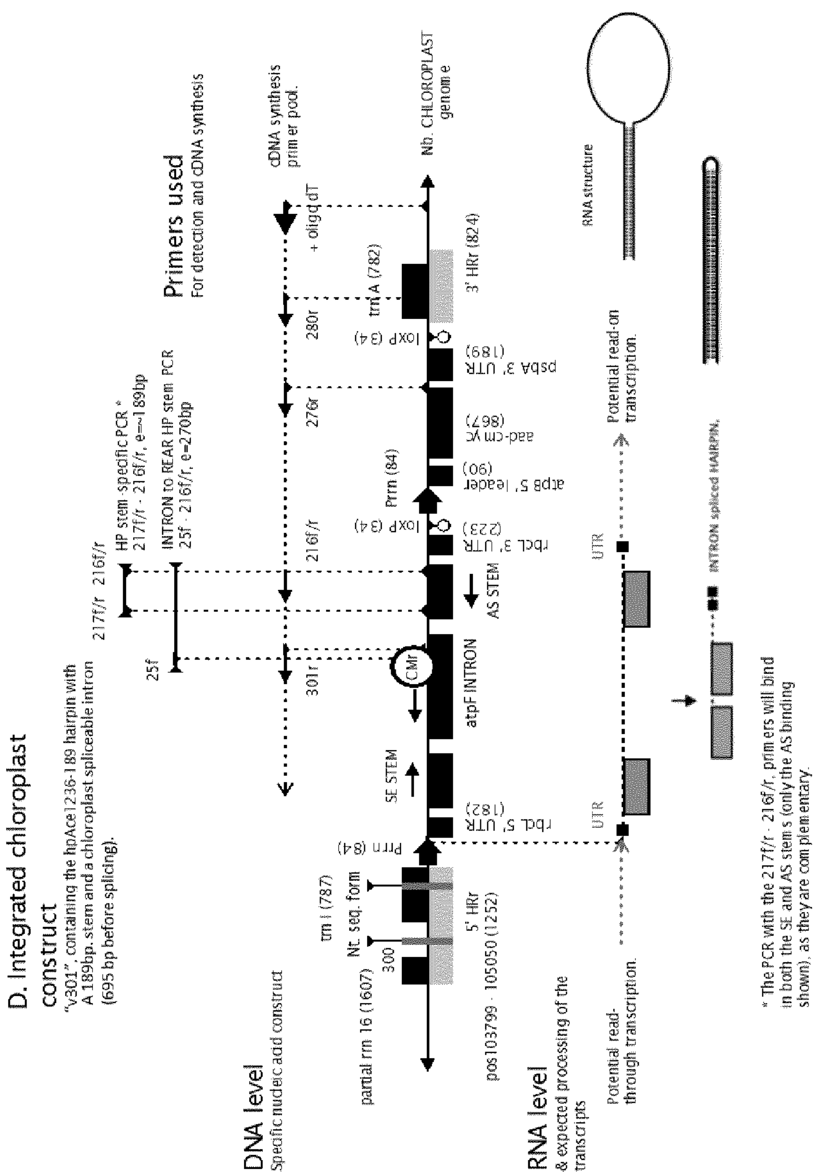
D. Integrated chloroplast construct
"v301", containing the hpAce1236-189 hairpin with A 189bp. stem and a chloroplast spliceable intron (695 bp before splicing).
Primers used
For detection and cDNA synthesis
217f/r 216f/r
HP stem specific PCR *
217f/r - 216f/r, e=~189bp
25f
INTRON to REAR HP stem PCR
25f - 216f/r, e=270bp
cDNA synthesis primer pool.
301r
216f/r
276r
280r
+ oligo dT
DNA level
Specific nucleic acid construct
partial rrn 16 (1607)
300
Nt. seq. form
trn I (787)
Prrn (84)
SE STEM
CMr
atpF INTRON
AS STEM
loxP (34)
Prrn (84)
loxP (34)
trnA (782)
Nb. CHLOROPLAST genome
pos103799 - 105050 (1252)
5' HRr
rbcL 5' UTR (182)
rbcL 3' UTR (223)
atpB 5' leader (90)
aad-cm yC (867)
psbA 3' UTR (189)
3' HRr (824)
RNA level
& expected processing of the transcripts
Potential read-through transcription.
UTR
UTR
Potential read-on transcription.
INTRON spliced HAIRPIN.
RNA structure
* The PCR with the 217f/r - 216f/r, primers will bind in both the SE and AS stems (only the AS binding shown), as they are complementary.

**Figure 1D**

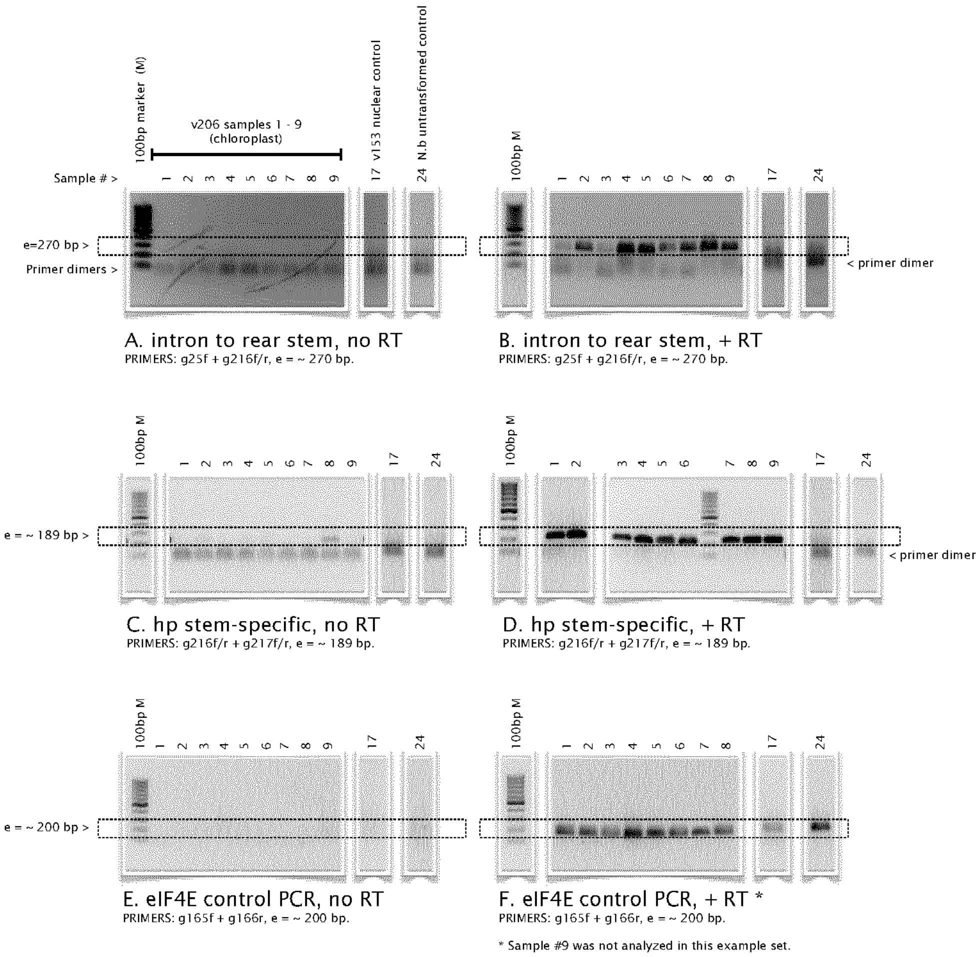
100bp marker (M)
v206 samples 1 - 9
(chloroplast)
17 v153 nuclear control
24 N.b untransformed control
Sample # >
1 2 3 4 5 6 7 8 9
e=270 bp >
Primer dimers >
100bp M
17
24
< primer dimer
A. intron to rear stem, no RT
PRIMERS: g25f + g216f/r, e = ~ 270 bp.
B. intron to rear stem, + RT
PRIMERS: g25f + g216f/r, e = ~ 270 bp.
e = ~ 189 bp >
< primer dimer
C. hp stem-specific, no RT
PRIMERS: g216f/r + g217f/r, e = ~ 189 bp.
D. hp stem-specific, + RT
PRIMERS: g216f/r + g217f/r, e = ~ 189 bp.
e = ~ 200 bp >
E. eIF4E control PCR, no RT
PRIMERS: g165f + g166r, e = ~ 200 bp.
F. eIF4E control PCR, + RT *
PRIMERS: g165f + g166r, e = ~ 200 bp.
* Sample #9 was not analyzed in this example set.

**Figure 2**

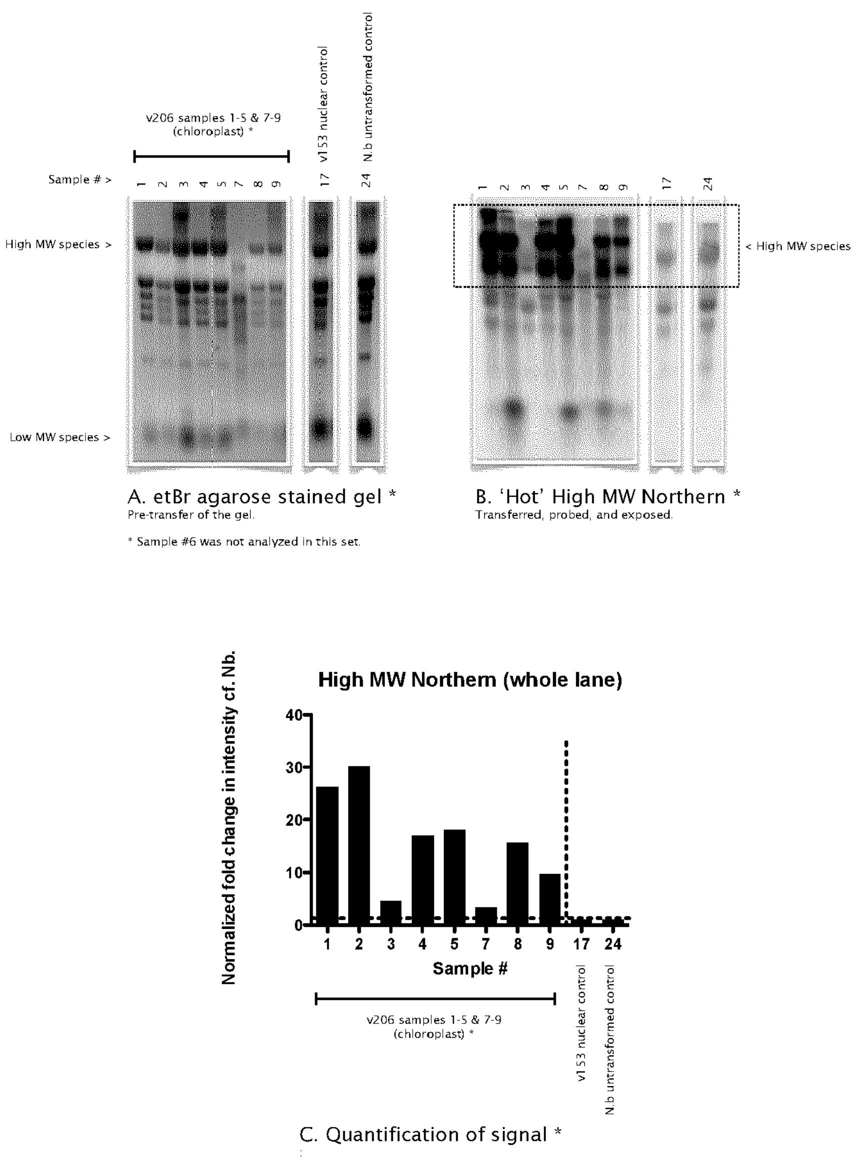

v206 samples 1-5 & 7-9
(chloroplast) *
v153 nuclear control
N b untransformed control
Sample # >
1 2 3 4 5 7 8 9 17 24
High MW species >
Low MW species >
< High MW species
A. etBr agarose stained gel *
Pre-transfer of the gel.
B. 'Hot' High MW Northern *
Transferred, probed, and exposed.
* Sample #6 was not analyzed in this set.
High MW Northern (whole lane)
Normalized fold change in intensity cf. Nb.
0
10
20
30
40
1 2 3 4 5 7 8 9 17 24
Sample #
v206 samples 1-5 & 7-9
(chloroplast) *
v153 nuclear control
N b untransformed control
C. Quantification of signal *


**Figure 3**

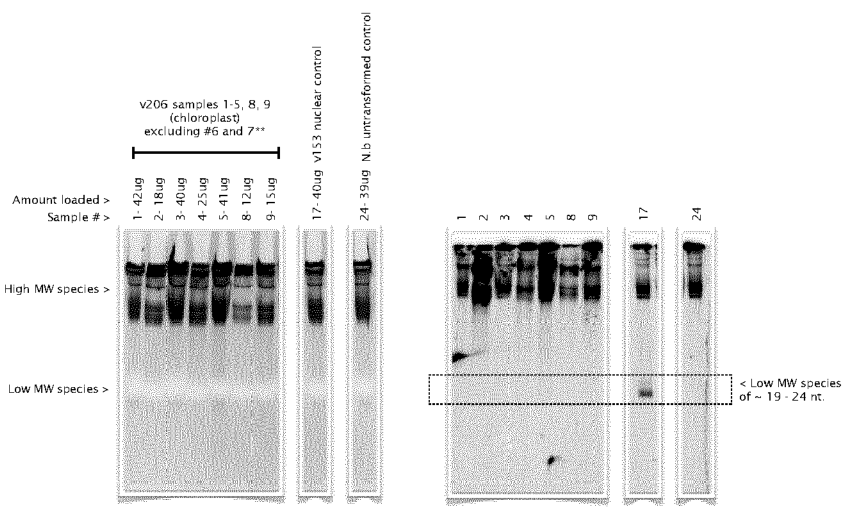


A. etBr agarose stained gel **
Pre-transfer of the gel.

B. 'Hot' low MW Northern **
Transferred, probed, and exposed.

** Samples #6 & 7 were not analyzed in this set.

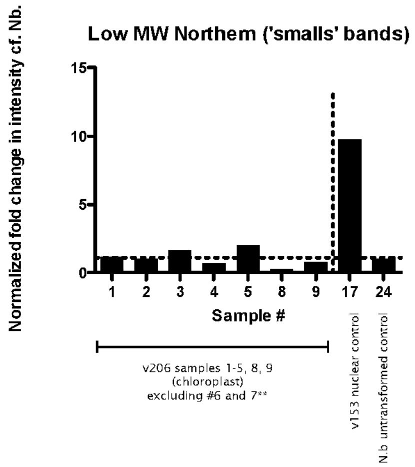


C. Quantification of signal *

**Figure 4**

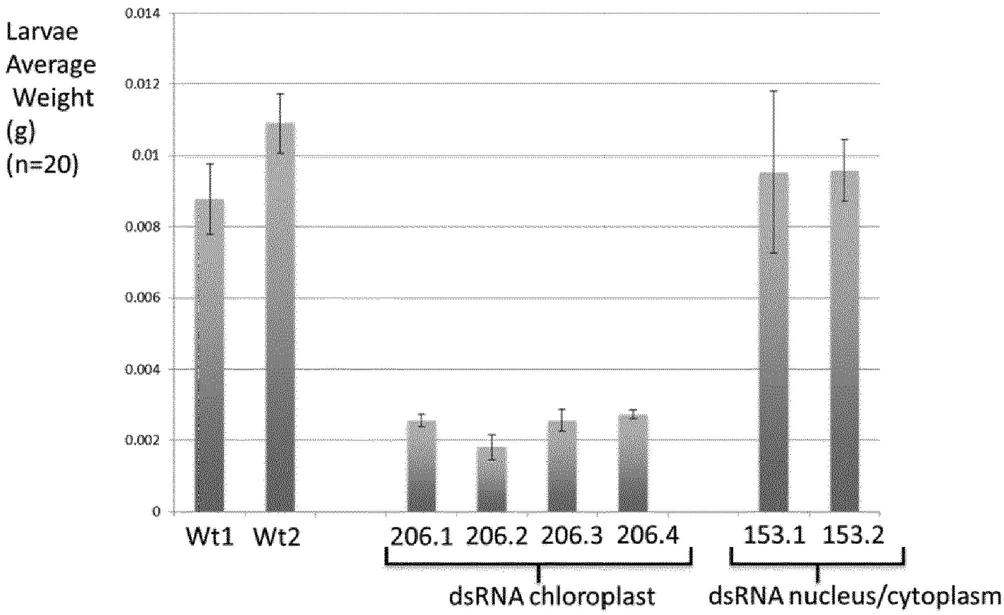
Larvae
Average
Weight
(g)
(n=20)
0.014
0.012
0.01
0.008
0.006
0.004
0.002
0
Wt1
Wt2
206.1
206.2
206.3
206.4
153.1
153.2
dsRNA chloroplast
dsRNA nucleus/cytoplasm

Figure 5A

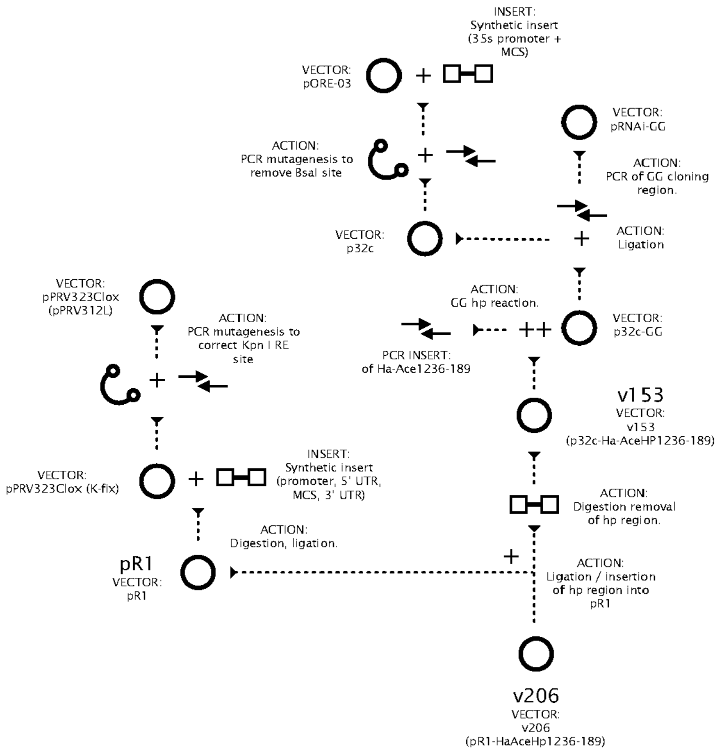
INSERT:
Synthetic insert
(35s promoter +
MCS)
VECTOR:
pORE-03
ACTION:
PCR mutagenesis to
remove BsaI site
VECTOR:
p32c
VECTOR:
pRNAi-GG
ACTION:
PCR of GG cloning
region.
ACTION:
Ligation
VECTOR:
pPRV323Clox
(pPRV312L)
ACTION:
PCR mutagenesis to
correct Kpn I RE
site
ACTION:
GG hp reaction.
PCR INSERT:
of Ha-Ace1236-189
VECTOR:
p32c-GG
v153
VECTOR:
v153
(p32c-Ha-AceHP1236-189)
VECTOR:
pPRV323Clox (K-fix)
INSERT:
Synthetic insert
(promoter, 5' UTR,
MCS, 3' UTR)
ACTION:
Digestion removal
of hp region.
ACTION:
Digestion, ligation.
pR1
VECTOR:
pR1
ACTION:
Ligation / insertion
of hp region into
pR1
v206
VECTOR:
v206
(pR1-HaAceHp1236-189)

Figure 5B

**Figure 6.**

**A. Helicoverpa armigera (Ha) Acetylcholinesterase (Acc. no. AF369793) (SEQ ID NO:13)**

acgcggggagtctactctgtcacgtcgatggagacgcatcgcggtcggcctctctctcaatattattactttattaccatacaa
aacgtgagtgagtgtctgtgaacttatttcaacttgagtgacggttgctccggaccatacagcgagctgacagttcagccaa
ggtcatcgatcagcaatttgacaccagttgtgtttatatactatatgtacgaccacaacagaataccgtgtgcccctagaaatg
tgttttaacaagtatctcattaagctggcggattacttcagatcagtttctgatacgtctcgttaatgcccgtgcgaacaacaac
attcgaaatgatcagcaacacgaagatcgtgttcaccaagctcctgctgtgctgcttcgtgtcaggcgccgtcgcgaggtc
gtgggccaaccatcacgacaccaccacgtcaaccacgcagaccacccccaccactagtcctgtgcctaagaacttccata
atgatccactcatcgtcgaaactaaaagtggcctcgtcaaaggctacgctaagacagttatgggcagggaagtacacatctt
caccggtattccgtttgcgaagcccccctctaggaccgttaagattccgcaaaccggtacccatcgacccgtggcatggagt
acttgaagccactgcgatgccaaatagctgttatcaagaacggtatgagtacttccccggttttgagggagaggaaatgtgg
aatccaaacacaaatatatcagaggactgtctctatctaaacatttgggttccccagcacttacgagtccgtcatcatcaagat
aagcctcttgcggagaggcccaaagtgccgatactagtgtggatttacggtggcggctatatgagtggcacggcaacact
caacctatataaagcagacataatggcttcttccagtgatgtaatagtagcatctatgcaatatagggttggggcgttcggatt
tttatacttgaataaatatttctcgccgggcagtgaagaggcgccgggaaatatgggcttatgggatcaacaactcgctattc
gttggattaaagataatgctcgtgcttttggtggtgacccagaattgataactttgtttggagagtcggcaggcgggggaagc
gtgagtttgcatatgctttcaccagagatgaagggattattcaaacgaggcatcttgcaatctggaacgttaaacgctccatg
gagttggatgacaggagagagagcacaagacataggaaaagtgttagtagatgactgtaattgcaatagcagcctactag
ctgccgatccgagcttagtgatggactgtatgcgcggagtcgatgcaaaaactatatcagtacagcagtggaattcctatac
tggcatattgggattcccgtcagctcccacggttgacggcgtgtttttgcccaaagacccggacacaatgatgaaagaagg
caatttccataataccgaggtgcttcttggaagtaatcaagacgaaggaacctatttcttactatacgatttccttgactacttcg
agaaagacgggcccagcttcttgcagcgggagaaatttctagaaattgtcgacaccatattcaaggatttctccaaaataaa
gagggaagctatcgtattccaatatacggactgggaggaaattaccgatggctatctgaaccagaaaatgatagctgacgt
ggtgggcgactatttcttcgtgtgccctactaactacttcgcagaagtgctggccgattcaggagttgatgtttactactattac
ttcacacatcgcaccagcacaagtctctggggtgaatggatgggcgtgatgcacggcgatgagatggagtacgtcttcgg
gcacccgctgaacatgtcccttcaataccacaccagggagagggaccttgctgcacatatcatgcagtcttttacgagattc
gctttgactggcaaacctcacaagccggacgagaaatggccgctgtactctcgcacgtcgccccactactacacttacacc
gcggacggggctagcgggccagccggaccgcgcgggcctagggcctccgcttgcgctttctggaatgatttccttaaca
agctgaatgagctggagcacatgccatgtgacggggccgtgaccggcctttacagcagcgtcgccggcaccaccctgcc
gatagtgctgctgaccacgctcgccaccaccgtcgcactctaagcgaccgacattcactcgtagcaaacagtagacaaatt
atttatacaaactagaaaaataccgatgcaaataaatgttttcacttcatctataaccataaagtcaaaaatcttatctgaaatga
actttatgattgtcaatgaagatctagagtacgcaaatcgtatttcgaattttaatatcggctactaaagtactagaagtaaattgt

tgtattaattaaatatattttatcacaatcttacatatcaagcgtgtggaaagaaaacgggacagtgacaaggaacgttacaaa
aaaaaaaaaaaaaaaaaaaaaaaaa

**B. Ha-Ace1236-189 (extracted from Acc. no. AF369793) (SEQ ID NO:14)**

catggagttggatgacaggagagagagcacaagacataggaaaagtgttagtagatgactgtaattgcaatagcagccta
ctagctgccgatccgagcttagtgatggactgtatgcgcggagtcgatgcaaaaactatatcagtacagcagtggaattcct
atactggcatattgggattcccgtcag

**C. Ha-HpAce1236-189 (SEQ ID NO:15)**

catggagttggatgacaggagagagagcacaagacataggaaaagtgttagtagatgactgtaattgcaatagcagccta
ctagctgccgatccgagcttagtgatggactgtatgcgcggagtcgatgcaaaaactatatcagtacagcagtggaattcct
atactggcatattgggattcccgtcagacgagcccttggtaaggaaataattattttctttttccttttagtataaaatagttaagt
gatgttaattagtatgattataataatatagttgttataattgtgaaaaaataatttataaatatattgtttacataaacaacatagta
atgtaaaaaaatatgacaagtgatgtgtaagacgaagaagataaaagttgagagtaagtatattatttttaatgaatttgatcga
acatgtaagatgatatacggccggtaagaggttccaactttcaccataatgaaataagatcactaccgggcgtatttttgagt
tatcgagattttcaggagctaaggaagctaaaatggagaaaaaaatcactggatataccaccgttgatatatcccaatggcat
cgtaaagaacattttgaggcatttcagtcagttgctcaatgtacctataaccagaccgttcagctggatattacggcctttttaa
agaccgtaaagaaaaataagcacaagttttatccggcctttattcacattcttgcccgcctgatgaatgctcatccggaattcc
gtatggcaatgaaagacggtgagctggtgatatgggatagtgttcacccttgttacaccgttttccatgagcaaactgaaacg
ttttcatcgctctggagtgaataccacgacgatttccggcagtttctacacatatattcgcaagatgtggcgtgttacggtgaa
aacctggcctatttccctaaagggtttattgagaatatgtttttcgtctcagccaatccctgggtgagtttcaccagttttgattta
aacgtggccaatatggacaacttcttcgcccccgttttcaccatgggcaaatattatacgcaaggcgacaaggtgctgatgc
cgctggcgattcaggttcatcatgccgtctgtgatggcttccatgtcggcagaatgcttaatgaattacaacagtactgcgat
gagtggcagggcggggcgtaatcgcgtggatccggcttactaaaagccagataacagtatgcgtatttgcgcgctgatttt
gcggtataagaatatatactgatatgtcggtcccataatagtaattctagctggtttgatgaattaaatatcaatgataaaatact
atagtaaaaataagaataaataaattaaaataatatttttttatgattaatagtttattatataattaaatatctataccattactaaata
ttttagtttaaaagttaataaatattttgttagaaattccaatctgcttgtaatttatcaataaacaaaatattaaataacaagctaaa
gtaacaaataatatcaaactaatagaaacagtaatctaatgtaacaaaacataatctaatgctaatataacaaagcgcaagat
ctatcattttatatagtattattttcaatcaacattcttattaatttctaaataatacttgtagttttattaacttctaaatggattgactatt
aattaaatgaattagtcgaacatgaataaacaaggtaacatgatagatcatgtcattgtgttatcattgatcttacatttggattga
ttacagttggtctagagatttcgtctagatcgtctgacgggaatcccaatatgccagtataggaattccactgctgtactgatat
agtttttgcatcgactccgcgcatacagtccatcactaagctcggatcggcagctagtaggctgctattgcaattacagtcatc
tactaacacttttcctatgtcttgtgctctctctcctgtcatccaactcca

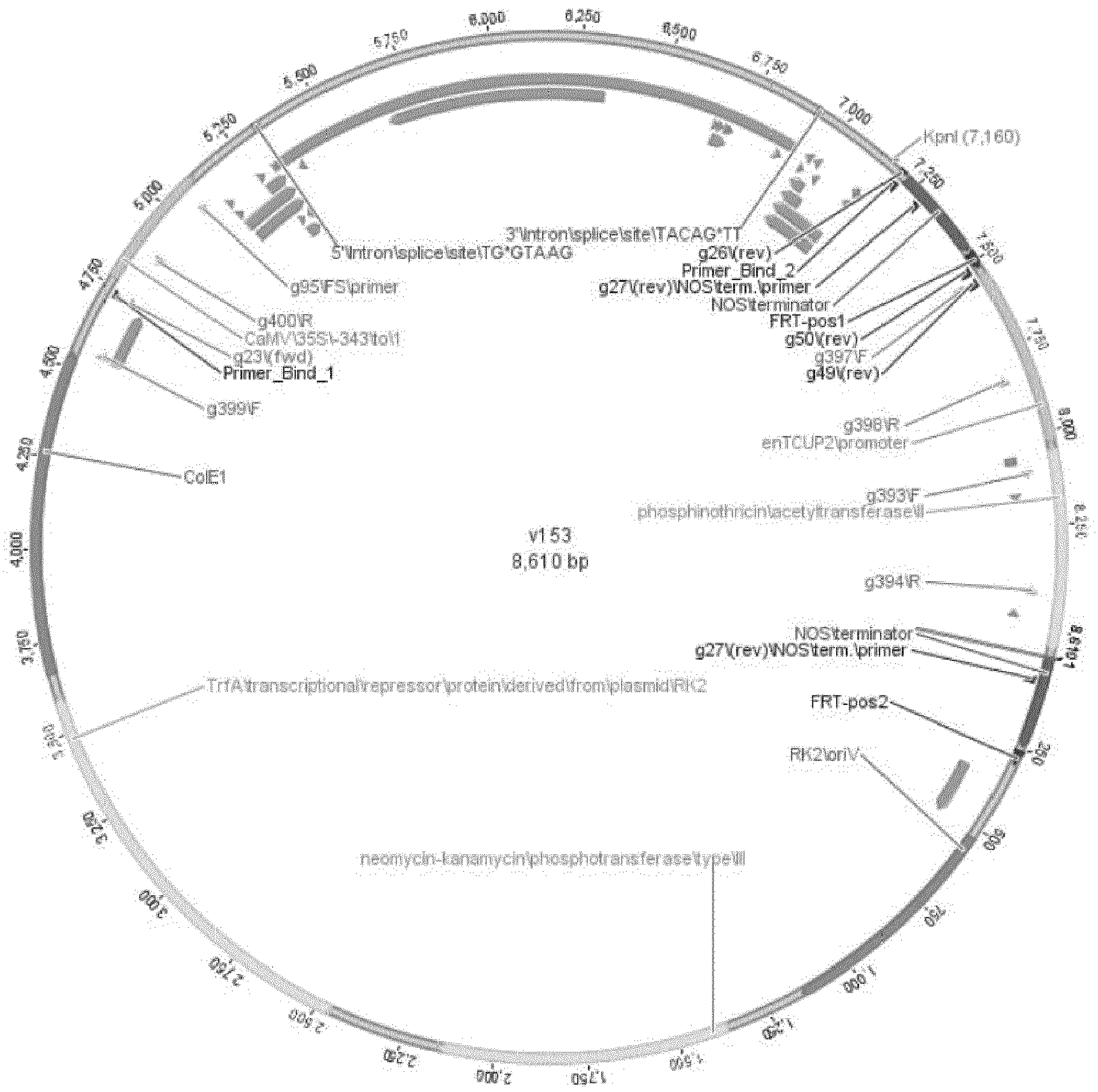
v153
8,610 bp
KpnI (7,160)
3'\Intron\splice\site\TACAG*TT
5'\Intron\splice\site\TG*GTAAG
g26\(rev)
Primer_Bind_2
g27\(rev)\NOS\term.\primer
NOS\terminator
FRT-pos1
g50\(rev)
g397\F
g49\(rev)
g95\FS\primer
g400\R
CaMV\35S\-343\to\1
g23\(fwd)
Primer_Bind_1
g399\F
ColE1
g398\R
enTCUP2\promoter
g393\F
phosphinothricin\acetyltransferase\II
g394\R
NOS\terminator
g27\(rev)\NOS\term.\primer
TrfA\transcriptional\repressor\protein\derived\from\plasmid\RK2
FRT-pos2
RK2\oriV
neomycin-kanamycin\phosphotransferase\type\II

Figure 7

**Figure 8**

v153: (SEQ ID NO:16)

GATCGTTCAAACATTTGGCAATAAAGTTTCTTAAGATTGAATCCTGTTGCC
GGTCTTGCGATGATTATCATATAATTTCTGTTGAATTACGTTAAGCATGTA
ATAATTAACATGTAATGCATGACGTTATTTATGAGATGGGTTTTTATGATT
AGAGTCCCGCAATTATACATTTAATACGCGATAGAAAACAAAATATAGCG
CGCAAACTAGGATAAATTATCGCGCGCGGTGTCATCTATGTTACTAGATC
CCTAGGGAAGTTCCTATTCCGAAGTTCCTATTCTCTGAAAAGTATAGGAA
CTTCTTTGCGTATTGGGCGCTCTTGGCCTTTTTGGCCACCGGTCGTACGGT
TAAAACCACCCCAGTACATTAAAAACGTCCGCAATGTGTTATTAAGTTGT
CTAAGCGTCAATTTGTTTACACCACAATATATCCTGCCACCAGCCAGCCA
ACAGCTCCCCGACCGGCAGCTCGGCACAAAATCACCACTCGATACAGGCA
GCCCATCAGTCCACTAGACGCTCACCGGGCTGGTTGCCCTCGCCGCTGGG
CTGGCGGCCGTCTATGGCCCTGCAAACGCGCCAGAAACGCCGTCGAAGCC
GTGTGCGAGACACCGCAGCCGCCGGCGTTGTGGATACCTCGCGGAAAACT
TGGCCCTCACTGACAGATGAGGGGCGGACGTTGACACTTGAGGGGCCGAC
TCACCCGGCGCGGCGTTGACAGATGAGGGGCAGGCTCGATTTCGGCCGGC
GACGTGGAGCTGGCCAGCCTCGCAAATCGGCGAAAACGCCTGATTTTACG
CGAGTTTCCCACAGATGATGTGGACAAGCCTGGGGATAAGTGCCCTGCGG
TATTGACACTTGAGGGGCGCGACTACTGACAGATGAGGGGCGCGATCCTT
GACACTTGAGGGGCAGAGTGCTGACAGATGAGGGGCGCACCTATTGACA
TTTGAGGGGCTGTCCACAGGCAGAAAATCCAGCATTTGCAAGGGTTTCCG
CCCGTTTTTCGGCCACCGCTAACCTGTCTTTTAACCTGCTTTTAAACCAAT
ATTTATAAACCTTGTTTTTAACCAGGGCTGCGCCCTGTGCGCGTGACCGCG
CACGCCGAAGGGGGGTGCCCCCCCTTCTCGAACCCTCCCGGCCCGCTCTC
GCGTTGGCAGCATCACCCATAATTGTGGTTTCAAAATCGGCTCCGTCGAT
ACTATGTTATACGCCAACTTTGAAAACAACTTTGAAAAAGCTGTTTTCTGG
TATTTAAGGTTTTAGAATGCAAGGAACAGTGAATTGGAGTTCGTCTTGTTA
TAATTAGCTTCTTGGGGTATCTTTAAATACTGTAGAAAAGAGGAAGGAAA
TAATAAATGGCTAAAATGAGAATATCACCGGAATTGAAAAAACTGATCG
AAAAATACCGCTGCGTAAAAGATACGGAAGGAATGTCTCCTGCTAAGGTA
TATAAGCTGGTGGGAGAAAATGAAAACCTATATTTAAAAATGACGGACA
GCCGGTATAAAGGGACCACCTATGATGTGGAACGGGAAAAGGACATGAT
GCTATGGCTGGAAGGAAAGCTGCCTGTTCCAAAGGTCCTGCACTTTGAAC

GGCATGATGGCTGGAGCAATCTGCTCATGAGTGAGGCCGATGGCGTCCTT
TGCTCGGAAGAGTATGAAGATGAACAAAGCCCTGAAAAGATTATCGAGC
TGTATGCGGAGTGCATCAGGCTCTTTCACTCCATCGACATATCGGATTGTC
CCTATACGAATAGCTTAGACAGCCGCTTAGCCGAATTGGATTACTTACTG
AATAACGATCTGGCCGATGTGGATTGCGAAAACTGGGAAGAAGACACTC
CATTTAAAGATCCGCGCGAGCTGTATGATTTTTAAAGACGGAAAAGCCC
GAAGAGGAACTTGTCTTTTCCCACGGCGACCTGGGAGACAGCAACATCTT
TGTGAAAGATGGCAAAGTAAGTGGCTTTATTGATCTTGGGAGAAGCGGCA
GGGCGGACAAGTGGTATGACATTGCCTTCTGCGTCCGGTCGATCAGGGAG
GATATTGGGGAAGAACAGTATGTCGAGCTATTTTTTGACTTACTGGGGAT
CAAGCCTGATTGGGAGAAAATAAAATATTATATTTTACTGGATGAATTGT
TTTAGTACCTAGATGTGGCGCAACGATGCCGGCGACAAGCAGGAGCGCAC
CGACTTCTTCCGCATCAAGTGTTTTGGCTCTCAGGCCGAGGCCCACGGCA
AGTATTTGGGCAAGGGGTCGCTGGTATTCGTGCAGGGCAAGATTCGGAAT
ACCAAGTACGAGAAGGACGGCCAGACGGTCTACGGGACCGACTTCATTG
CCGATAAGGTGGATTATCTGGACACCAAGGCACCAGGCGGGTCAAATCA
GGAATAAGGGCACATTGCCCCGGCGTGAGTCGGGGCAATCCCGCAAGGA
GGGTGAATGAATCGGACGTTTGACCGGAAGGCATACAGGCAAGAACTGA
TCGACGCGGGGTTTTCCGCCGAGGATGCCGAAACCATCGCAAGCCGCACC
GTCATGCGTGCGCCCCGCGAAACCTTCCAGTCCGTCGGCTCGATGGTCCA
GCAAGCTACGGCCAAGATCGAGCGCGACAGCGTGCAACTGGCTCCCCCTG
CCCTGCCCGCGCCATCGGCCGCCGTGGAGCGTTCGCGTCGTCTCGAACAG
GAGGCGGCAGGTTTGGCGAAGTCGATGACCATCGACACGCGAGGAACTA
TGACGACCAAGAAGCGAAAAACCGCCGGCGAGGACCTGGCAAAACAGGT
CAGCGAGGCCAAGCAAGCCGCGTTGCTGAAACACACGAAGCAGCAGATC
AAGGAAATGCAGCTTTCCTTGTTCGATATTGCGCCGTGGCCGGACACGAT
GCGAGCGATGCCAAACGACACGGCCCGCTCTGCCCTGTTCACCACGCGCA
ACAAGAAAATCCCGCGCGAGGCGCTGCAAAACAAGGTCATTTTCCACGTC
AACAAGGACGTGAAGATCACCTACACCGGCGTCGAGCTGCGGGCCGACG
ATGACGAACTGGTGTGGCAGCAGGTGTTGGAGTACGCGAAGCGCACCCCT
ATCGGCGAGCCGATCACCTTCACGTTCTACGAGCTTTGCCAGGACCTGGG
CTGGTCGATCAATGGCCGGTATTACACGAAGGCCGAGGAATGCCTGTCGC
GCCTACAGGCGACGGCGATGGGCTTCACGTCCGACCGCGTTGGGCACCTG
GAATCGGTGTCGCTGCTGCACCGCTTCCGCGTCCTGGACCGTGGCAAGAA

AACGTCCCGTTGCCAGGTCCTGATCGACGAGGAAATCGTCGTGCTGTTTG
CTGGCGACCACTACACGAAATTCATATGGGAGAAGTACCGCAAGCTGTCG
CCGACGGCCCGACGGATGTTCGACTATTTCAGCTCGCACCGGGAGCCGTA
CCCGCTCAAGCTGGAAACCTTCCGCCTCATGTGCGGATCGGATTCCACCC
GCGTGAAGAAGTGGCGCGAGCAGGTCGGCGAAGCCTGCGAAGAGTTGCG
AGGCAGCGGCCTGGTGGAACACGCCTGGGTCAATGATGACCTGGTGCATT
GCAAACGCTAGGGCCTTGTGGGGTCAGTTCCGGCTGGGGGTTCAGCAGCC
AGCGCTTTACTGAGATCCTCTTCCGCTTCCTCGCTCACTGACTCGCTGCGC
TCGGTCGTTCGGCTGCGGCGAGCGGTATCAGCTCACTCAAAGGCGGTAAT
ACGGTTATCCACAGAATCAGGGGATAACGCAGGAAAGAACATGTGAGCA
AAAGGCCAGCAAAAGGCCAGGAACCGTAAAAAGGCCGCGTTGCTGGCGT
TTTTCCATAGGCTCCGCCCCCCTGACGAGCATCACAAAAATCGACGCTCA
AGTCAGAGGTGGCGAAACCCGACAGGACTATAAAGATACCAGGCGTTTC
CCCCTGGAAGCTCCCTCGTGCGCTCTCCTGTTCCGACCCTGCCGCTTACCG
GATACCTGTCCGCCTTTCTCCCTTCGGGAAGCGTGGCGCTTTCTCATAGCT
CACGCTGTAGGTATCTCAGTTCGGTGTAGGTCGTTCGCTCCAAGCTGGGCT
GTGTGCACGAACCCCCCGTTCAGCCCGACCGCTGCGCCTTATCCGGTAAC
TATCGTCTTGAGTCCAACCCGGTAAGACACGACTTATCGCCACTGGCAGC
AGCCACTGGTAACAGGATTAGCAGAGCGAGGTATGTAGGCGGTGCTACA
GAGTTCTTGAAGTGGTGGCCTAACTACGGCTACACTAGAAGAACAGTATT
TGGTATCTGCGCTCTGCTGAAGCCAGTTACCTTCGGAAAAAGAGTTGGTA
GCTCTTGATCCGGCAAACAAACCACCGCTGGTAGCGGTGGTTTTTTTGTTT
GCAAGCAGCAGATTACGCGCAGAAAAAAAGGATCTCAAGAAGATCCTTT
GATCTTTTCTACGGGGTCTGACGCTCAGTGGAACGAAAACTCACGTTAAG
GGATTTTGGTCATGAGATTATCAAAAAGGATCTTCACCTAGATCCTTTTGG
ATCTCCTGTGGTTGGCATGCACATACAAATGGACGAACGGATAAACCTTT
TCACGCCCTTTTAAATATCCGATTATTCTAATAAACGCTCTTTTCTCTTAGG
TTTACCCGCCAATATATCCTGTCAAACACTGATAGTTTAAACTGAAGGCG
GGAAACGACAATCTGCTAGTGGATCTCCCAGTCACGACGTTGTAAAACGG
GCGCCCCGCGGAAAGCTTGCTAGCTGAGACTTTTCAACAAAGGATAATTT
CGGGAAACCTCCTCGGATTCCATTGCCCAGCTATCTGTCACTTCATCGAAA
GGACAGTAGAAAAGGAAGGTGGCTCCTACAAATGCCATCATTGCGATAA
AGGAAAGGCTATCATTCAAGATCTCTCTGCCGACAGTGGTCCCAAAGATG
GACCCCCACCCACGAGGAGCATCGTGGAAAAAGAAGACGTTCCAACCAC

GTCTTCAAAGCAAGTGGATTGATGTGACATCTCCACTGACGTAAGGGATG
ACGCACAATCCCACTATCCTTCGCAAGACCCTTCCTCTATATAAGGAAGTT
CATTTCATTTGGAGAGGACTCGAGGGGCCCGGAGCATGGAGTTGGATGAC
AGGAGAGAGAGCACAAGACATAGGAAAAGTGTTAGTAGATGACTGTAAT
TGCAATAGCAGCCTACTAGCTGCCGATCCGAGCTTAGTGATGGACTGTAT
GCGCGGAGTCGATGCAAAAACTATATCAGTACAGCAGTGGAATTCCTATA
CTGGCATATTGGGATTCCCGTCAGACGAGCCCTTGGTAAGGAAATAATTA
TTTTCTTTTTTCCTTTTAGTATAAAATAGTTAAGTGATGTTAATTAGTATGA
TTATAATAATATAGTTGTTATAATTGTGAAAAAATAATTTATAAATATATT
GTTTACATAAACAACATAGTAATGTAAAAAAATATGACAAGTGATGTGTA
AGACGAAGAAGATAAAAGTTGAGAGTAAGTATATTATTTTTAATGAATTT
GATCGAACATGTAAGATGATATACGGCCGGTAAGAGGTTCCAACTTTCAC
CATAATGAAATAAGATCACTACCGGGCGTATTTTTTGAGTTATCGAGATTT
TCAGGAGCTAAGGAAGCTAAAATGGAGAAAAAAATCACTGGATATACCA
CCGTTGATATATCCCAATGGCATCGTAAAGAACATTTTGAGGCATTTCAGT
CAGTTGCTCAATGTACCTATAACCAGACCGTTCAGCTGGATATTACGGCCT
TTTTAAAGACCGTAAAGAAAAATAAGCACAAGTTTTATCCGGCCTTTATT
CACATTCTTGCCCGCCTGATGAATGCTCATCCGGAATTCCGTATGGCAATG
AAAGACGGTGAGCTGGTGATATGGGATAGTGTTCACCCTTGTTACACCGT
TTTCCATGAGCAAACTGAAACGTTTTCATCGCTCTGGAGTGAATACCACG
ACGATTTCCGGCAGTTTCTACACATATATTCGCAAGATGTGGCGTGTTACG
GTGAAAACCTGGCCTATTTCCCTAAAGGGTTTATTGAGAATATGTTTTTCG
TCTCAGCCAATCCCTGGGTGAGTTTCACCAGTTTTGATTTAAACGTGGCCA
ATATGGACAACTTCTTCGCCCCCGTTTTCACCATGGGCAAATATTATACGC
AAGGCGACAAGGTGCTGATGCCGCTGGCGATTCAGGTTCATCATGCCGTC
TGTGATGGCTTCCATGTCGGCAGAATGCTTAATGAATTACAACAGTACTG
CGATGAGTGGCAGGGCGGGGCGTAATCGCGTGGATCCGGCTTACTAAAA
GCCAGATAACAGTATGCGTATTTGCGCGCTGATTTTTGCGGTATAAGAAT
ATATACTGATATGTCGGTCCCATAATAGTAATTCTAGCTGGTTTGATGAAT
TAAATATCAATGATAAAATACTATAGTAAAAATAAGAATAAATAAATTAA
AATAATATTTTTTTATGATTAATAGTTTATTATATAATTAAATATCTATACC
ATTACTAAATATTTTAGTTTAAAAGTTAATAAATATTTTGTTAGAAATTCC
AATCTGCTTGTAATTTATCAATAAACAAAATATTAAATAACAAGCTAAAG
TAACAAATAATATCAAACTAATAGAAACAGTAATCTAATGTAACAAAACA

TAATCTAATGCTAATATAACAAAGCGCAAGATCTATCATTTTATATAGTAT
TATTTTCAATCAACATTCTTATTAATTTCTAAATAATACTTGTAGTTTTATT
AACTTCTAAATGGATTGACTATTAATTAAATGAATTAGTCGAACATGAAT
AAACAAGGTAACATGATAGATCATGTCATTGTGTTATCATTGATCTTACAT
TTGGATTGATTACAGTTGGTCTAGAGATTTCGTCTAGATCGTCTGACGGA
ATCCCAATATGCCAGTATAGGAATTCCACTGCTGTACTGATATAGTTTTTG
CATCGACTCCGCGCATACAGTCCATCACTAAGCTCGGATCGGCAGCTAGT
AGGCTGCTATTGCAATTACAGTCATCTACTAACACTTTTCCTATGTCTTGT
GCTCTCTCTCCTGTCATCCAACTCCATGCTCCGAGCTCGGTACCGCTAGCC
GTACCTTTTACTAGTGATATCCCTGTGTGAAATTGTTATCCGCTACGCGTG
ATCGTTCAAACATTTGGCAATAAAGTTTCTTAAGATTGAATCCTGTTGCCG
GTCTTGCGATGATTATCATATAATTTCTGTTGAATTACGTTAAGCATGTAA
TAATTAACATGTAATGCATGACGTTATTTATGAGATGGGTTTTTATGATTA
GAGTCCCGCAATTATACATTTAATACGCGATAGAAAACAAAATATAGCGC
GCAAACTAGGATAAATTATCGCGCGCGGTGTCATCTATGTTACTAGATCC
CATGGGAAGTTCCTATTCCGAAGTTCCTATTCTCTGAAAAGTATAGGAACT
TCAGCGATCGCAGACGTCGGGATCTTCTGCAAGCATCTCTATTTCCTGAAG
GTCTAACCTCGAAGATTTAAGATTTAATTACGTTTATAATTACAAAATTGA
TTCTAGTATCTTTAATTTAATGCTTATACATTATTAATTAATTTAGTACTTT
CAATTTGTTTTCAGAAATTATTTTACTATTTTTTATAAAATAAAAGGGAGA
AAATGGCTATTTAAATACTAGCCTATTTTATTTCAATTTTAGCTTAAAATC
AGCCCCAATTAGCCCCAATTTCAAATTCAAATGGTCCAGCCCAATTCCTA
AATAACCCACCCCTAACCCGCCCGGTTTCCCCTTTTGATCCATGCAGTCAA
CGCCCAGAATTTCCCTATATAATTTTTTAATTCCCAAACACCCCTAACTCT
ATCCCATTTCTCACCAACCGCCACATAGATCTATCCTCTTATCTCTCAAAC
TCTCTCGAACCTTCCCCTAACCCTAGCAGCCTCTCATCATCCTCACCTCAA
AACCCACCGGGGCCGGCCATGTCTCCGGAGAGAAGGCCAGTTGAGATTA
GGCCAGCTACAGCAGCTGATATGGCCGCTGTTTGTGACATCGTTAACCAT
TACATTGAGACTTCTACAGTGAACTTTAGGACAGAGCCACAAACACCACA
AGAGTGGATTGATGATCTTGAGAGGTTGCAAGATAGATACCCTTGGTTGG
TTGCTGAGGTTGAGGGTGTTGTGGCTGGTATTGCTTACGCTGGACCTTGGA
AGGCTAGGAACGCTTACGATTGGACAGTTGAGAGTACTGTTTACGTGTCA
CATAGGCATCAAAGGTTGGGCCTCGGATCTACATTGTACACACATTTGCTT
AAGTCTATGGAGGCGCAAGGTTTTAAGTCTGTGGTTGCTGTTATTGGCCTT

CCAAACGATCCATCTGTTAGGTTGCATGAGGCTTTGGGATACACAGCCAG
GGGTACATTGCGCGCAGCTGGATACAAGCATGGTGGATGGCATGATGTTG
GTTTTTGGCAAAGGGATTTTGAGTTGCCAGCTCCTCCAAGGCCAGTTAGA
CCAGTTACCCAGATCTGAGGCGCGCC

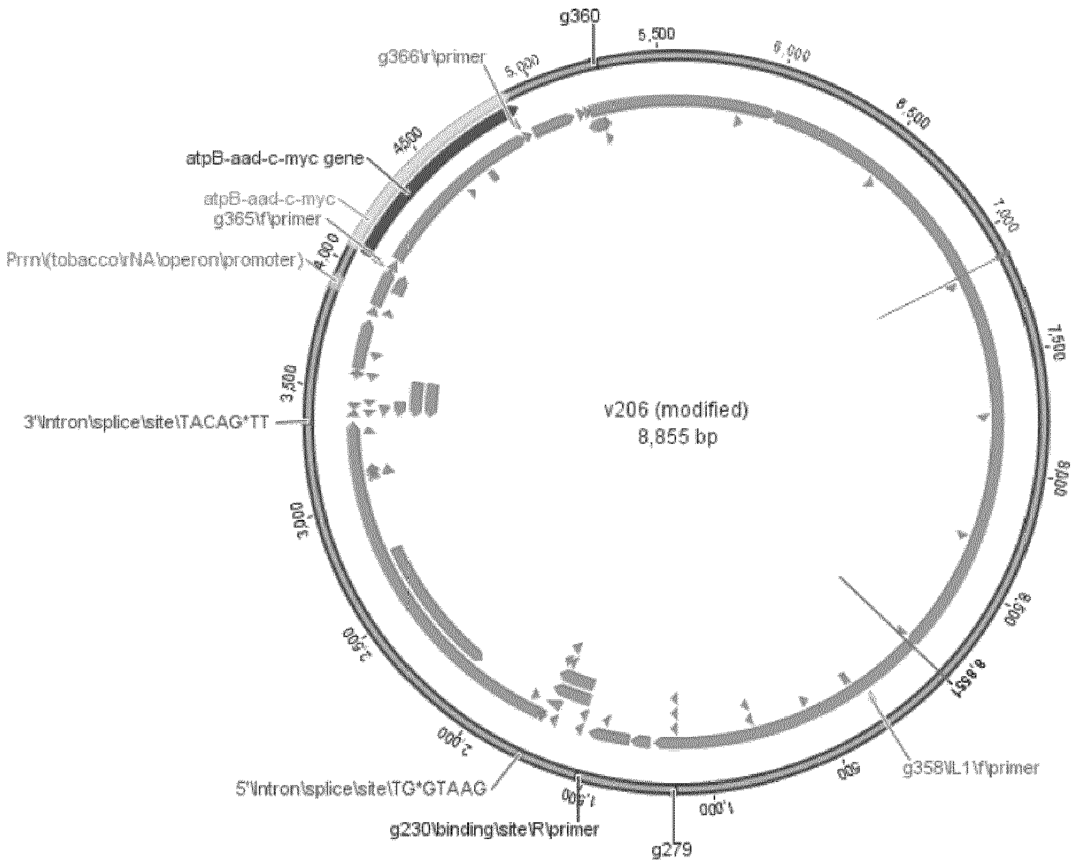
g360
5,500
6,000
6,500
7,000
7,500
8,000
8,500
8,855
500
1,000
1,500
2,000
2,500
3,000
3,500
4,000
4500
5,000
g366\r\primer
atpB-aad-c-myc gene
atpB-aad-c-myc
g365\f\primer
Prrn\(tobacco\rNA\operon\promoter)
3'\Intron\splice\site\TACAG*TT
v206 (modified)
8,855 bp
5'\Intron\splice\site\TG*GTAAG
g230\binding\site\R\primer
g279
g358\IL1\f\primer

Figure 9

**Figure 10**

v206 (modified) (SEQ ID NO:17):

GGGCCTTGTACACACCGCCCGTCACACTATGGGAGCTGGCCATGCCCGAA
GTCGTTACCTTAACCGCAAGGAGGGGGATGCCGAAGGCAGGGCTAGTGA
CTGGAGTGAAGTCGTAACAAGGTAGCCGTACTGGAAGGTGCGGCTGGATC
ACCTCCTTTTCAGGGAGAGCTAATGCTTGTTGGGTATTTTGGTTTGACACT
GCTTCACACCCCCAAAAAAAAGAAGGGAGCTACGTCTGAGTTAAACTTGG
AGATGGAAGTCTTCTTTCCTTTCTCGACGGTGAAGTAAGACCAAGCTCAT
GAGCTTATTATCCTAGGTCGGAACAAGTTGATAGGACCCCCTTTTTACGT
CCCCATGTTCCCCCCGTGTGGCGACATGGGGGCGAAAAAAGGAAAGAGA
GGGATGGGGTTTCTCTCGCTTTTGGCATAGCGGGCCCCCAGTGGGAGGCT
CGCACGACGGGCTATTAGCTCAGTGGTAGAGCGCGCCCCTGATAATTGCG
TCGTTGTGCCTGGGCTGTGAGGGCTCTCAGCCACATGGATAGTTCAATGT
GCTCATCGGCGCCTGACCCTGAGATGTGGATCATCCAAGGCACATTAGCA
TGGCGTACTCCTCCTGTTCGAACCGGGGTTTGAAACCAAACTCCTCCTCAG
GAGGATAGATGGGGCGATTCGGGTGAGATCCAATGTAGATCCAACTTTCG
ATTCACTCGTGGGATCCGGGCGGTCCGGGGGGGACCACCACGGCTCCTCT
CTTCTCGAGAATCCATACATCCCTTATCAGTGTATGGACAGCTATCTCTCG
AGCACAGGTTTAGCAATGGGAAAATAAAATGGAGCACCTAACAACGCAT
CTTCACAGACCAAGAACTACGAGATCGCCCCTTTCATTCTGGGGTGACGG
AGGGATCGTACCATTCGAGCCGTTTTTTTCTTGACTCGAAATGGGAGCAG
GTTTGAAAAAGGATCTTAGAGTGTCTAGGGTTGGGCCAGGAGGGTCTCTT
AACGCCTTCTTTTTTCTTCTCATCGGAGTTATTTCACAAAGACTTGCCAGG
GTAAGGAAGAAGGGGGGAACAAGCACACTTGGAGAGCGCAGTACAACGG
AGAGTTGTATGCTGCGTTCGGGAAGGATGAATCGCTCCCGAAAAGGAATC
TATTGATTCTCTCCCAATTGGTTGGACCGTAGGTGCGATGATTTACTTCAC
GGGCGAGGTCTCTGGTTCAAGTCCAGGATGGCCCAGCTGCATTTAAATGG
CGCGCCGCTCCCCCGCCGTCGTTCAATGAGAATGGATAAGAGGCTCGTGG
GATTGACGTGAGGGGGCAGGGATGGCTATATTTCTGGGAGACGCGTATGT
ATTTGGCAAATCAAATACCATGGTCTAATAATCAAACATTCTGATTAGTTG
ATAATATTAGTATTAGTTGGAAATTTTGTGAAAGATTCCTGTGAAAAGTTT
CATTAACACGGAATTCGTGTCGAGTAGACCTTGTTGTTGTGAGAATTCTTA
ATTCATGAGTTGTAGGGAGGGATTTCTTAAGCTCGAGGGATCCGTCGAGG

GGCCCGGAGCATGGAGTTGGATGACAGGAGAGAGAGCACAAGACATAGG
AAAAGTGTTAGTAGATGACTGTAATTGCAATAGCAGCCTACTAGCTGCCG
ATCCGAGCTTAGTGATGGACTGTATGCGCGGAGTCGATGCAAAAACTATA
TCAGTACAGCAGTGGAATTCCTATACTGGCATATTGGGATTCCCGTCAGA
CGAGCCCTTGGTAAGGAAATAATTATTTTCTTTTTTCCTTTTAGTATAAAA
TAGTTAAGTGATGTTAATTAGTATGATTATAATAATATAGTTGTTATAATT
GTGAAAAAATAATTTATAAATATATTGTTTACATAAACAACATAGTAATG
TAAAAAAATATGACAAGTGATGTGTAAGACGAAGAAGATAAAAGTTGAG
AGTAAGTATATTATTTTTAATGAATTTGATCGAACATGTAAGATGATATAC
GGCCGGTAAGAGGTTCCAACTTTCACCATAATGAAATAAGATCACTACCG
GGCGTATTTTTTGAGTTATCGAGATTTTCAGGAGCTAAGGAAGCTAAAAT
GGAGAAAAAAATCACTGGATATACCACCGTTGATATATCCCAATGGCATC
GTAAAGAACATTTTGAGGCATTTCAGTCAGTTGCTCAATGTACCTATAACC
AGACCGTTCAGCTGGATATTACGGCCTTTTTAAAGACCGTAAAGAAAAAT
AAGCACAAGTTTTATCCGGCCTTTATTCACATTCTTGCCCGCCTGATGAAT
GCTCATCCGGAATTCCGTATGGCAATGAAAGACGGTGAGCTGGTGATATG
GGATAGTGTTCACCCTTGTTACACCGTTTTCCATGAGCAAACTGAAACGTT
TTCATCGCTCTGGAGTGAATACCACGACGATTTCCGGCAGTTTCTACACAT
ATATTCGCAAGATGTGGCGTGTTACGGTGAAAACCTGGCCTATTTCCCTA
AAGGGTTTATTGAGAATATGTTTTTCGTCTCAGCCAATCCCTGGGTGAGTT
TCACCAGTTTTGATTTAAACGTGGCCAATATGGACAACTTCTTCGCCCCCG
TTTTCACCATGGGCAAATATTATACGCAAGGCGACAAGGTGCTGATGCCG
CTGGCGATTCAGGTTCATCATGCCGTCTGTGATGGCTTCCATGTCGGCAGA
ATGCTTAATGAATTACAACAGTACTGCGATGAGTGGCAGGGCGGGGCGTA
ATCGCGTGGATCCGGCTTACTAAAAGCCAGATAACAGTATGCGTATTTGC
GCGCTGATTTTTGCGGTATAAGAATATATACTGATATGTCGGTCCCATAAT
AGTAATTCTAGCTGGTTTGATGAATTAAATATCAATGATAAAATACTATA
GTAAAAATAAGAATAAATAAATTAAAATAATATTTTTTTATGATTAATAG
TTTATTATATAATTAAATATCTATACCATTACTAAATATTTTAGTTTAAAA
GTTAATAAATATTTTGTTAGAAATTCCAATCTGCTTGTAATTTATCAATAA
ACAAAATATTAAATAACAAGCTAAAGTAACAAATAATATCAAACTAATA
GAAACAGTAATCTAATGTAACAAAACATAATCTAATGCTAATATAACAAA
GCGCAAGATCTATCATTTTATATAGTATTATTTTCAATCAACATTCTTATT
AATTTCTAAATAATACTTGTAGTTTATTAACTTCTAAATGGATTGACTAT

TAATTAAATGAATTAGTCGAACATGAATAAACAAGGTAACATGATAGATC
ATGTCATTGTGTTATCATTGATCTTACATTTGGATTGATTACAGTTGGTCT
AGAGATTTCGTCTAGATCGTCTGACGGGAATCCCAATATGCCAGTATAGG
AATTCCACTGCTGTACTGATATAGTTTTTGCATCGACTCCGCGCATACAGT
CCATCACTAAGCTCGGATCGGCAGCTAGTAGGCTGCTATTGCAATTACAG
TCATCTACTAACACTTTTCCTATGTCTTGTGCTCTCTCTCCTGTCATCCAAC
TCCATGCTCCGAGCTCGGTACCGCTAGCAAGCTTAGACATTAGCAGATAA
ATTAGCAGGAAATAAAGAAGGATAAGGAGAAAGAACTCAAGTAATTATC
CTTCGTTCTCTTAATTGAATTGCAATTAAACTCGGCCCAATCTTTTACTAA
AAGGATTGAGCCGAATACAACAAAGATTCTATTGCATATATTTTGACTAA
GTATATACTTACCTAGATATACAAGATTTGAAATACAAAATCTAGTCTAG
AGCGGCCGCAGTAGCTTATAACTTCGTATAGCATACATTATACGAAGTTA
TAGATCCGCTCCCCCGCCGTCGTTCAATGAGAATGGATAAGAGGCTCGTG
GGATTGACGTGAGGGGGCAGGGATGGCTATATTTCTGGGAGAATTAACCG
ATCGACGTGCAAGCGGACATTTATTTAAATTCGATAATTTTTGCAAAAAC
ATTTCGACATATTTATTTATTTTATTATTATGAGAATCAATCCTACTACTTC
TGGTTCTGGGGTTTCCACGGCTAGTAGCGAAGCGGTGATCGCCGAAGTAT
CGACTCAACTATCAGAGGTAGTTGGCGTCATCGAGCGCCATCTCGAACCG
ACGTTGCTGGCCGTACATTTGTACGGCTCCGCAGTGGATGGCGGCCTGAA
GCCACACAGTGATATTGATTTGCTGGTTACGGTGACCGTAAGGCTTGATG
AAACAACGCGGCGAGCTTTGATCAACGACCTTTTGGAAACTTCGGCTTCC
CCTGGAGAGAGCGAGATTCTCCGCGCTGTAGAAGTCACCATTGTTGTGCA
CGACGACATCATTCCGTGGCGTTATCCAGCTAAGCGCGAACTGCAATTTG
GAGAATGGCAGCGCAATGACATTCTTGCAGGTATCTTCGAGCCAGCCACG
ATCGACATTGATCTGGCTATCTTGCTGACAAAAGCAAGAGAACATAGCGT
TGCCTTGGTAGGTCCAGCGGCGGAGGAACTCTTTGATCCGGTTCCTGAAC
AGGATCTATTTGAGGCGCTAAATGAAACCTTAACGCTATGGAACTCGCCG
CCCGACTGGGCTGGCGATGAGCGAAATGTAGTGCTTACGTTGTCCCGCAT
TTGGTACAGCGCAGTAACCGGCAAAATCGCGCCGAAGGATGTCGCTGCCG
ACTGGGCAATGGAGCGCCTGCCGGCCCAGTATCAGCCCGTCATACTTGAA
GCTAGACAGGCTTATCTTGGACAAGAAGAAGATCGCTTGGCCTCGCGCGC
AGATCAGTTGGAAGAATTTGTCCACTACGTGAAAGGCGAGATCACCAAGG
TAGTGGGCAAAGAACAAAAACTCATTTCTGAAGAAGACTTGTGAGTCTAG
CTAGAGCGATCCTGGCCTAGTCTATAGGAGGTTTTGAAAAGAAAGGAGCA

ATAATCATTTTCTTGTTCTATCAAGAGGGTGCTATTGCTCCTTTCTTTTTTT
CTTTTTATTTATTTACTAGTATTTTACTTACATAGACTTTTTTGTTTACATTA
TAGAAAAAGAAGGAGAGGTTATTTTCTTGCATTTATTCATGGGGGATCAA
AGCTGATCTATAACTTCGTATAGCATACATTATACGAAGTTATGGTACACT
ATTTAAATGCGCTCCACTTGGCTCGGGGGGATATAGCTCAGTTGGTAGAG
CTCCGCTCTTGCAATTGGGTCGTTGCGATTACGGGTTGGATGTCTAATTGT
CCAGGCGGTAATGATAGTATCTTGTACCTGAACCGGTGGCTCACTTTTTCT
AAGTAATGGGGAAGAGGACCGAAACGTGCCACTGAAAGACTCTACTGAG
ACAAAGATGGGCTGTCAAGAACGTAGAGGAGGTAGGATGGGCAGTTGGT
CAGATCTAGTATGGATCGTACATGGACGGTAGTTGGAGTCGGCGGCTCTC
CCAGGGTTCCCTCATCTGAGATCTCTGGGGAAGAGGATCAAGTTGGCCCT
TGCGAACAGCTTGATGCACTATCTCCCTTCAACCCTTTGAGCGAAATGCG
GCAAAAGAAAAGGAAGGAAAATCCATGGACCGACCCCATCATCTCCACC
CCGTAGGAACTACGAGATCACCCCAAGGACGCCTTCGGCATCCAGGGGTC
ACGGACCGACCATAGAACCCTGTTCAATAAGTGGAACGCATTAGCTGTCC
GCTCTCAGGTTGGGCAGTCAGGGTCGGAGAAGGGCAATGACTCATTCTTA
GTTAGAATGGGATTCCAACTCAGCACCTTTTGAGTGAGATTTTGAGAAGA
GTTGCTCTTTGGAGAGCACAGTACGATGAAAGTTGTAAGCTGTGTTCGGG
GGGGAGTTATTGTCTATCGTTGGCCTCTATGGTAGAATCAGTCGGGGGAC
CTGAGAGGCGGTGGTTTACCCTGCGGCGGATGTCAGCGGTTCGAGTCCGC
TTATCTCCAACTCGTGAACTTAGCCGATACAAAGCTCTGGCGTAATAGCG
AAGAGGCCCGCACCGATCGCCCTTCCCAACAGTTGCGCAGCCTGAATGGC
GAATGGCGCCTGATGCGGTATTTTCTCCTTACGCATCTGTGCGGTATTTCA
CACCGCATACGTCAAAGCAACCATAGTACGCGCCCTGTAGCGGCGCATTA
AGCGCGGCGGGTGTGGTGGTTACGCGCAGCGTGACCGCTACACTTGCCAG
CGCCCTAGCGCCCGCTCCTTTCGCTTTCTTCCCTTCCTTTCTCGCCACGTTC
GCCGGCTTTCCCCGTCAAGCTCTAAATCGGGGGCTCCCTTTAGGGTTCCGA
TTTAGTGCTTTACGGCACCTCGACCCCAAAAAACTTGATTTGGGTGATGGT
TCACGTAGTGGGCCATCGCCCTGATAGACGGTTTTTCGCCCTTTGACGTTG
GAGTCCACGTTCTTTAATAGTGGACTCTTGTTCCAAACTGGAACAACACTC
AACCCTATCTCGGGCTATTCTTTTGATTTATAAGGGATTTTGCCGATTTCG
GCCTATTGGTTAAAAAATGAGCTGATTTAACAAAAATTTAACGCGAATTT
TAACAAAATATTAACGTTTACAATTTTATGGTGCACTCTCAGTACAATCTG
CTCTGATGCCGCATAGTTAAGCCAGCCCCGACACCCGCCAACACCCGCTG

ACGCGCCCTGACGGGCTTGTCTGCTCCCGGCATCCGCTTACAGACAAGCT
GTGACCGTCTCCGGGAGCTGCATGTGTCAGAGGTTTTCACCGTCATCACC
GAAACGCGCGAGACGAAAGGGCCTCGTGATACGCCTATTTTTATAGGTTA
ATGTCATGATAATAATGGTTTCTTAGACGTCAGGTGGCACTTTTCGGGGA
AATGTGCGCGGAACCCCTATTTGTTTATTTTTCTAAATACATTCAAATATG
TATCCGCTCATGAGACAATAACCCTGATAAATGCTTCAATAATATTGAAA
AAGGAAGAGTATGAGTATTCAACATTTCCGTGTCGCCCTTATTCCCTTTTT
TGCGGCATTTTGCCTTCCTGTTTTTGCTCACCCAGAAACGCTGGTGAAAGT
AAAAGATGCTGAAGATCAGTTGGGTGCACGAGTGGGTTACATCGAACTGG
ATCTCAACAGCGGTAAGATCCTTGAGAGTTTTCGCCCCGAAGAACGTTTT
CCAATGATGAGCACTTTTAAAGTTCTGCTATGTGGCGCGGTATTATCCCGT
ATTGACGCCGGGCAAGAGCAACTCGGTCGCCGCATACACTATTCTCAGAA
TGACTTGGTTGAGTATTCACCAGTCACAGAAAAGCATCTTACGGATGGCA
TGACAGTAAGAGAATTATGCAGTGCTGCCATAACCATGAGTGATAACACT
GCGGCCAACTTACTTCTGACAACGATCGGAGGACCGAAGGAGCTAACCGC
TTTTTTGCACAACATGGGGGATCATGTAACTCGCCTTGATCGTTGGGAACC
GGAGCTGAATGAAGCCATACCAAACGACGAGCGTGACACCACGATGCCT
GTAGCAATGGCAACAACGTTGCGCAAACTATTAACTGGCGAACTACTTAC
TCTAGCTTCCCGGCAACAATTAATAGACTGGATGGAGGCGGATAAAGTTG
CAGGACCACTTCTGCGCTCGGCCCTTCCGGCTGGCTGGTTTATTGCTGATA
AATCTGGAGCCGGTGAGCGTGGGTCTCGCGGTATCATTGCAGCACTGGGG
CCAGATGGTAAGCCCTCCCGTATCGTAGTTATCTACACGACGGGGAGTCA
GGCAACTATGGATGAACGAAATAGACAGATCGCTGAGATAGGTGCCTCA
CTGATTAAGCATTGGTAACTGTCAGACCAAGTTTACTCATATATACTTTAG
ATTGATTTAAAACTTCATTTTTAATTTAAAAGGATCTAGGTGAAGATCCTT
TTTGATAATCTCATGACCAAAATCCCTTAACGTGAGTTTTCGTTCCACTGA
GCGTCAGACCCCGTAGAAAAGATCAAAGGATCTTCTTGAGATCCTTTTTTT
CTGCGCGTAATCTGCTGCTTGCAAACAAAAAAACCACCGCTACCAGCGGT
GGTTTGTTTGCCGGATCAAGAGCTACCAACTCTTTTTCCGAAGGTAACTGG
CTTCAGCAGAGCGCAGATACCAAATACTGTCCTTCTAGTGTAGCCGTAGT
TAGGCCACCACTTCAAGAACTCTGTAGCACCGCCTACATACCTCGCTCTGC
TAATCCTGTTACCAGTGGCTGCTGCCAGTGGCGATAAGTCGTGTCTTACCG
GGTTGGACTCAAGACGATAGTTACCGGATAAGGCGCAGCGGTCGGGCTG
AACGGGGGGTTCGTGCACACAGCCCAGCTTGGAGCGAACGACCTACACC

GAACTGAGATACCTACAGCGTGAGCTATGAGAAAGCGCCACGCTTCCCGA
AGGGAGAAAGGCGGACAGGTATCCGGTAAGCGGCAGGGTCGGAACAGGA
GAGCGCACGAGGGAGCTTCCAGGGGGAAACGCCTGGTATCTTTATAGTCC
TGTCGGGTTTCGCCACCTCTGACTTGAGCGTCGATTTTTGTGATGCTCGTC
AGGGGGGCGGAGCCTATGGAAAAACGCCAGCAACGCGGCCTTTTTACGG
TTCCTGGCCTTTTGCTGGCCTTTTGCTCACATGTTCTTTCCTGCGTTATCCC
CTGATTCTGTGGATAACCGTATTACCGCCTTTGAGTGAGCTGATACCGCTC
GCCGCAGCCGAACGACCGAGCGCAGCGAGTCAGTGAGCGAGGAAGCGGA
AGAGCGCCCAATACGCAAACCGCCTCTCCCCGCGCGTTGGCCGATTCATT
AATGCAG

V301 map

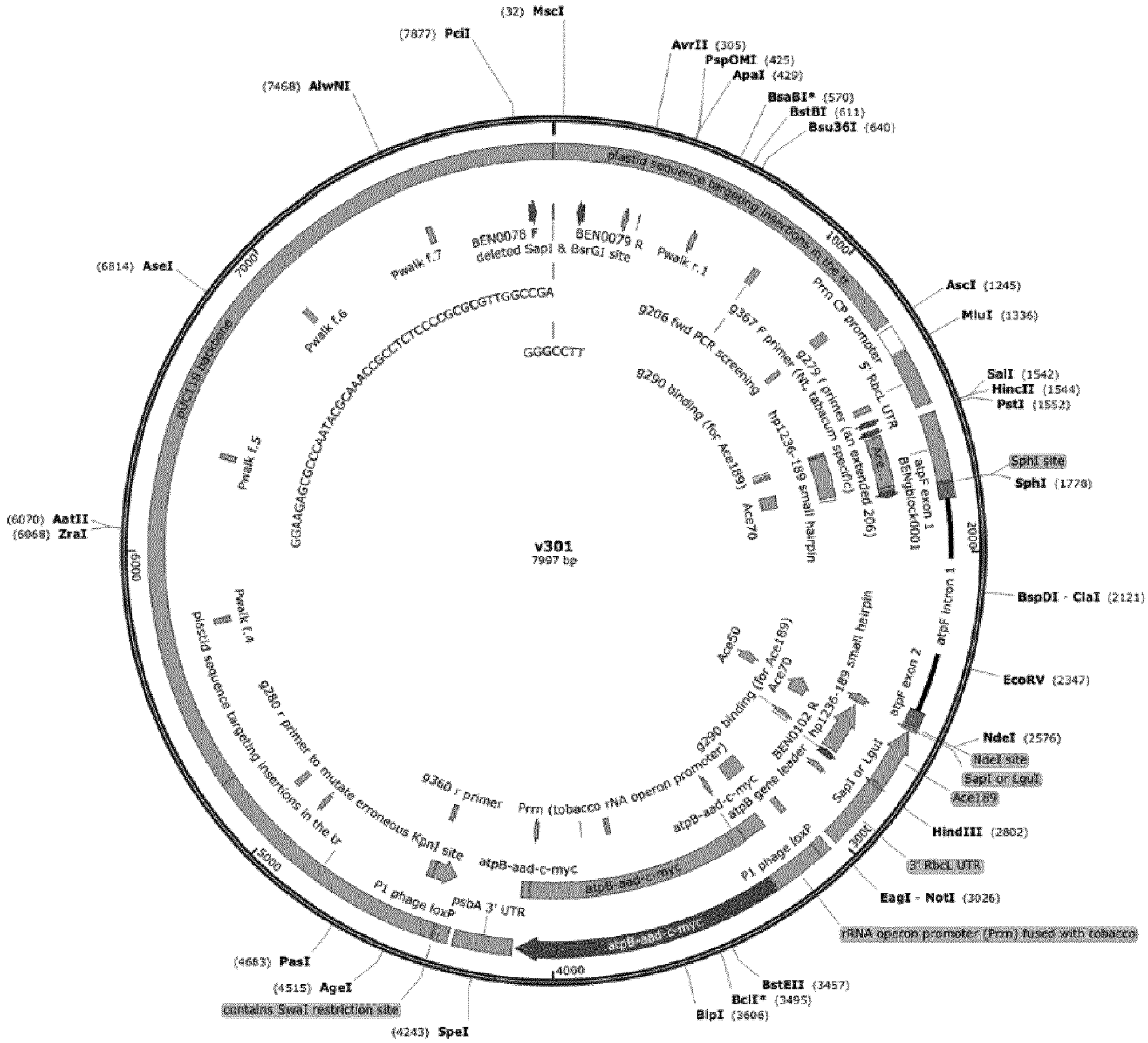


# Figure 11

## Figure 12

V301 sequence (SEQ ID NO:18)

gtacacaccgcccgtcacactatgggagctggccatgcccgaagtcgttaccttaaccgcaaggagggggatgccgaag
gcagggctagtgactggagtgaagtcgtaacaaggtagccgtactggaaggtgcggctggatcacctccttttcagggag
agctaatgcttgttgggtattttggtttgacactgcttcacacccccaaaaaaaagaagggagctacgtctgagttaaacttgg
agatggaagtcttctttcctttctcgacggtgaagtaagaccaagctcatgagcttattatcctaggtcggaacaagttgatag
gacccccttttttacgtccccatgttccccccgtgtggcgacatgggggcgaaaaaaggaaagagagggatggggtttctc
tcgcttttggcatagcgggcccccagtgggaggctcgcacgacgggctattagctcagtggtagagcgcgcccctgataa
ttgcgtcgttgtgcctgggctgtgagggctctcagccacatggatagttcaatgtgctcatcggcgcctgaccctgagatgt
ggatcatccaaggcacattagcatggcgtactcctcctgttcgaaccggggtttgaaaccaaactcctcctcaggaggata
gatggggcgattcgggtgagatccaatgtagatccaactttcgattcactcgtgggatccgggcggtccgggggggacca
ccacggctcctctcttctcgagaatccatacatcccttatcagtgtatggacagctatctctcgagcacaggtttagcaatggg
aaaataaaatggagcacctaacaacgcatcttcacagaccaagaactacgagatcgcccctttcattctggggtgacggag
ggatcgtaccattcgagccgtttttttcttgactcgaaatgggagcaggtttgaaaaaggatcttagagtgtctagggttgggc
caggagggtctcttaacgccttctttttcttctcatcggagttatttcacaaagacttgccagggtaaggaagaagggggga
acaagcacacttggagagcgcagtacaacggagagttgtatgctgcgttcgggaaggatgaatcgctcccgaaaaggaa
tctattgattctctcccaattggttggaccgtaggtgcgatgatttacttcacgggcgaggtctctggttcaagtccaggatgg
cccagctgcatttaaatggcgcgccgctcccccgccgtcgttcaatgagaatggataagaggctcgtgggattgacgtga
gggggcagggatggctatatttctgggagacgcgtatgtatttggcaaatcaaataccatggtctaataatcaaacattctga
ttagttgataatattagtattagttggaaattttgtgaaagattcctgtgaaaagtttcattaacacggaattcgtgtcgagtagac
cttgttgttgtgagaattcttaattcatgagttgtagggagggatttcttaagctcgagggatccgtcgacctgcagtctcgaag
agcggagcatggagttggatgacaggagagagagcacaagacataggaaaagtgttagtagatgactgtaattgcaata
gcagcctactagctgccgatccgagcttagtgatggactgtatgcgcggagtcgatgcaaaaactatatcagtacagcagt
ggaattcctatactggcatattgggattcccgtcagcacgagctcttcgcacgcatgcaaatctaagtgtagtgcttggtgtat
tgattttttttggaaagggagtgtgtgcgagttgtttatttcaagaataggctggaccacccagctgtacccttttccgttataact
aggaaagagaggtgcatgatctcgcgaattacttctgaataaattcagaaattatatgtaagaaccatagcatttcgcgattca
ttggtaaatcaattttgattctctattaaccaataatgtggaactattaacatggttaaaacaaactgtttgaagtctagacgcag
catggtactctttctaccactatgttaatatagaggtggtttcaaaataaatattttatcgatataggatactcatattgataaaatg
atttgaaccgcttattgtaaattgtaaaaacggggattttacccccctttatctaatgctgaatcgacgacctattctaagtaagaa
gagctttttggatttgaaaaaaaaaaaaagaaacaactttgctgacaattacatattttttattttgtcagaagagtcctccgaat
attttggtcttacattagtttcgatatctttttggaatatgaacaaagaagagaagataggctcattacattcataaaaaaagatat
gagaatttaccttaagtaattgagcgtgagagccaaatgaatcgaaagattcatgtttggttcgggaagggattatggaatttt
ggaaaggaatggaaaaataatctactttcattaagtgatttattagataatcgaaaacagaggatcttgaatactattcgacata
tgtgtgcgaagagctcgagctgacgggaatcccaatatgccagtataggaattccactgctgtactgatatagtttttgcatc

gactccgcgcatacagtccatcactaagctcggatcggcagctagtaggctgctattgcaattacagtcatctactaacactt
ttcctatgtcttgtgctctctctcctgtcatccaactccatgctccgctcttcgagaaagcttagacattagcagataaattagca
ggaaataaagaaggataaggagaaagaactcaagtaattatccttcgttctcttaattgaattgcaattaaactcggcccaatc
ttttactaaaaggattgagccgaatacaacaaagattctattgcatatattttgactaagtatatacttacctagatatacaagatt
tgaaatacaaaatctagtctagagcggccgcagtagcttataacttcgtatagcatacattatacgaagttatagatccgctcc
cccgccgtcgttcaatgagaatggataagaggctcgtgggattgacgtgagggggcagggatggctatatttctgggaga
attaaccgatcgacgtgcaagcggacatttattttaaattcgataatttttgcaaaaacatttcgacatatttatttattttattattat
gagaatcaatcctactacttctggttctggggtttccacggctagtagcgaagcggtgatcgccgaagtatcgactcaactat
cagaggtagttggcgtcatcgagcgccatctcgaaccgacgttgctggccgtacatttgtacggctccgcagtggatggcg
gcctgaagccacacagtgatattgatttgctggttacggtgaccgtaaggcttgatgaaacaacgcggcgagctttgatcaa
cgaccttttggaaacttcggcttcccctggagagagcgagattctccgcgctgtagaagtcaccattgttgtgcacgacgac
atcattccgtggcgttatccagctaagcgcgaactgcaatttggagaatggcagcgcaatgacattcttgcaggtatcttcga
gccagccacgatcgacattgatctggctatcttgctgacaaaagcaagagaacatagcgttgccttggtaggtccagcggc
ggaggaactctttgatccggttcctgaacaggatctatttgaggcgctaaatgaaaccttaacgctatggaactcgccgccc
gactgggctggcgatgagcgaaatgtagtgcttacgttgtcccgcatttggtacagcgcagtaaccggcaaaatcgcgcc
gaaggatgtcgctgccgactgggcaatggagcgcctgccggcccagtatcagcccgtcatacttgaagctagacaggctt
atcttggacaagaagaagatcgcttggcctcgcgcgcagatcagttggaagaatttgtccactacgtgaaaggcgagatca
ccaaggtagtgggcaaagaacaaaaactcatttctgaagaagacttgtgagtctagctagagcgatcctggcctagtctata
ggaggttttgaaaagaaaggagcaataatcattttcttgttctatcaagagggtgctattgctcctttctttttttctttttatttattta
ctagtattttacttacatagacttttttgtttacattatagaaaaagaaggagaggttattttcttgcatttattcatgggggatcaaa
gctgatctataacttcgtatagcatacattatacgaagttatggtacactatttaaatgcgctccacttggctcgggggatata
gctcagttggtagagctccgctcttgcaattgggtcgttgcgattacgggttggatgtctaattgtccaggcggtaatgatagt
atcttgtacctgaaccggtggctcactttttctaagtaatggggaagaggaccgaaacgtgccactgaaagactctactgag
acaaagatgggctgtcaagaacgtagaggaggtaggatgggcagttggtcagatctagtatggatcgtacatggacggta
gttggagtcggcggctctcccagggttccctcatctgagatctctggggaagaggatcaagttggcccttgcgaacagctt
gatgcactatctcccttcaaccctttgagcgaaatgcggcaaaagaaaaggaaggaaaatccatggaccgaccccatcat
ctccaccccgtaggaactacgagatcaccccaaggacgccttcggcatccaggggtcacggaccgaccatagaaccctg
ttcaataagtggaacgcattagctgtccgctctcaggttgggcagtcagggtcggagaagggcaatgactcattcttagtta
gaatgggattccaactcagcaccttttgagtgagattttgagaagagttgctctttggagagcacagtacgatgaaagttgta
agctgtgttcgggggggagttattgtctatcgttggcctctatggtagaatcagtcgggggacctgagaggcggtggtttac
cctgcggcggatgtcagcggttcgagtccgcttatctccaactcgtgaacttagccgatacaaagctctggcgtaatagcga
agaggcccgcaccgatcgcccttcccaacagttgcgcagcctgaatggcgaatggcgcctgatgcggtattttctccttac
gcatctgtgcggtatttcacaccgcatacgtcaaagcaaccatagtacgcgccctgtagcggcgcattaagcgcggcggg
tgtggtggttacgcgcagcgtgaccgctacacttgccagcgccctagcgcccgctcctttcgctttcttcccttcctttctcgc

cacgttcgccggctttccccgtcaagctctaaatcgggggctccctttagggttccgatttagtgctttacggcacctcgacc
ccaaaaaacttgatttgggtgatggttcacgtagtgggccatcgccctgatagacggtttttcgccctttgacgttggagtcca
cgttctttaatagtggactcttgttccaaactggaacaacactcaaccctatctcgggctattcttttgatttataagggattttgc
cgatttcggcctattggttaaaaaatgagctgatttaacaaaaatttaacgcgaattttaacaaaatattaacgtttacaattttat
ggtgcactctcagtacaatctgctctgatgccgcatagttaagccagccccgacacccgccaacacccgctgacgcgccc
tgacgggcttgtctgctcccggcatccgcttacagacaagctgtgaccgtctccgggagctgcatgtgtcagaggttttcac
cgtcatcaccgaaacgcgcgagacgaaagggcctcgtgatacgcctatttttataggttaatgtcatgataataatggtttctt
agacgtcaggtggcacttttcggggaaatgtgcgcggaacccctatttgtttatttttctaaatacattcaaatatgtatccgctc
atgagacaataaccctgataaatgcttcaataatattgaaaaaggaagagtatgagtattcaacatttccgtgtcgcccttattc
ccttttttgcggcattttgccttcctgtttttgctcacccagaaacgctggtgaaagtaaaagatgctgaagatcagttgggtgc
acgagtgggttacatcgaactggatctcaacagcggtaagatccttgagagttttcgccccgaagaacgttttccaatgatg
agcacttttaaagttctgctatgtggcgcggtattatcccgtattgacgccgggcaagagcaactcggtcgccgcatacact
attctcagaatgacttggttgagtattcaccagtcacagaaaagcatcttacggatggcatgacagtaagagaattatgcagt
gctgccataaccatgagtgataacactgcggccaacttacttctgacaacgatcggaggaccgaaggagctaaccgcttttt
tgcacaacatgggggatcatgtaactcgccttgatcgttgggaaccggagctgaatgaagccataccaaacgacgagcgt
gacaccacgatgcctgtagcaatggcaacaacgttgcgcaaactattaactggcgaactacttactctagcttcccggcaac
aattaatagactggatggaggcggataaagttgcaggaccacttctgcgctcggcccttccggctggctggtttattgctgat
aaatctggagccggtgagcgtgggtctcgcggtatcattgcagcactggggccagatggtaagccctcccgtatcgtagtt
atctacacgacggggagtcaggcaactatggatgaacgaaatagacagatcgctgagataggtgcctcactgattaagca
ttggtaactgtcagaccaagtttactcatatatactttagattgatttaaaacttcatttttaatttaaaaggatctaggtgaagatc
ctttttgataatctcatgaccaaaatcccttaacgtgagttttcgttccactgagcgtcagaccccgtagaaaagatcaaagga
tcttcttgagatcctttttttctgcgcgtaatctgctgcttgcaaacaaaaaaaccaccgctaccagcggtggtttgtttgccgg
atcaagagctaccaactctttttccgaaggtaactggcttcagcagagcgcagataccaaatactgtccttctagtgtagccg
tagttaggccaccacttcaagaactctgtagcaccgcctacatacctcgctctgctaatcctgttaccagtggctgctgccag
tggcgataagtcgtgtcttaccgggttggactcaagacgatagttaccggataaggcgcagcggtcgggctgaacgggg
ggttcgtgcacacagcccagcttggagcgaacgacctacaccgaactgagatacctacagcgtgagctatgagaaagcg
ccacgcttcccgaagggagaaaggcggacaggtatccggtaagcggcagggtcggaacaggagagcgcacgaggga
gcttccagggggaaacgcctggtatctttatagtcctgtcgggtttcgccacctctgacttgagcgtcgatttttgtgatgctcg
tcaggggggcggagcctatggaaaaacgccagcaacgcggcctttttacggttcctggccttttgctggccttttgctcaca
tgttctttcctgcgttatcccctgattctgtggataaccgtattaccgcctttgagtgagctgataccgctcgccgcagccgaac
gaccgagcgcagcgagtcagtgagcgaggaagc

Europäisches Patentamt
European Patent Office
Office européen des brevets

# EUROPEAN SEARCH REPORT

Application Number
EP 20 16 5687

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | HOTTO AMBER M ET AL: "Overexpression of a natural chloroplast-encoded antisense RNA in tobacco destabilizes 5S rRNA and retards plant growth", BMC PLANT BIOLOGY, BIOMED CENTRAL, LONDON, GB, vol. 10, no. 1, 29 September 2010 (2010-09-29), page 213, XP021073726, ISSN: 1471-2229, DOI: 10.1186/1471-2229-10-213 * abstract * * Results * * page Methods * ----- | 1-12 | INV. C12N15/113 C12N15/29 C12N15/82 |
| T | H Martens: "Epigenetisches Genesilencing: RNA Interferenz und Antisense RNA", , 8 January 2003 (2003-01-08), XP055391333, Retrieved from the Internet: URL:http://www.biospektrum.de/blatt/d_bs_pdf&_id=932824 [retrieved on 2017-07-17] ----- | | TECHNICAL FIELDS SEARCHED (IPC) C12N |
| X | XUE XUE-YI ET AL: "New Approaches to Agricultural Insect Pest Control Based on RNA Interference", ADVANCES IN INSECT PHYSIO, ACADEMIC PRESS, vol. 42, 1 January 2012 (2012-01-01), pages 73-117, XP009176987, ISSN: 0065-2806, DOI: 10.1016/B978-0-12-387680-5.00003-3 * the whole document * ----- -/-- | 1-12 | |

The present search report has been drawn up for all claims

| Place of search | Date of completion of the search | Examiner |
|---|---|---|
| Munich | 13 July 2020 | Keller, Yves |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

2

EPO FORM 1503 03.82 (P04C01)

page 1 of 5

Europäisches Patentamt
European Patent Office
Office européen des brevets

# EUROPEAN SEARCH REPORT

Application Number
EP 20 16 5687

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| T | NIDHI THAKUR ET AL: "Enhanced Whitefly Resistance in Transgenic Tobacco Plants Expressing Double Stranded RNA of v-ATPase A Gene", PLOS ONE, vol. 9, no. 3, 3 March 2014 (2014-03-03), page e87235, XP055190071, DOI: 10.1371/journal.pone.0087235 ----- | 1-12 | |
| T | FANG ZHU ET AL: "Ingested RNA interference for managing the populations of the Colorado potato beetle, Leptinotarsa decemlineata", PEST MANAGEMENT SCIENCE, WILEY & SONS, BOGNOR REGIS; GB, vol. 67, no. 2, 1 February 2011 (2011-02-01), pages 175-182, XP002661389, ISSN: 1526-498X, DOI: 10.1002/PS.2048 * the whole document * ----- | | |
| | | | TECHNICAL FIELDS SEARCHED (IPC) |
| T | WO 2007/104570 A2 (DEVGEN NV [BE]; VAN DE CRAEN MARC [BE] ET AL.) 20 September 2007 (2007-09-20) * the whole document * * page 33 - page 34 * * claims 1-57 * ----- | | |
| X | WO 2007/011479 A2 (MONSANTO TECHNOLOGY LLC; ALLEN EDWARDS [US] ET AL.) 25 January 2007 (2007-01-25) * abstract * * paragraph [0036] * * claims 1-12 * * paragraph [0039] * * paragraph [0070] - paragraph [0071] * * paragraph [0092] * ----- | 1-12 | |
| | -/-- | | |

The present search report has been drawn up for all claims

| Place of search | Date of completion of the search | Examiner |
|---|---|---|
| Munich | 13 July 2020 | Keller, Yves |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 20 16 5687

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2012/054919 A2 (DONALD DANFORTH PLANT SCI CT [US]; SAYRE RICHARD [US]; KUMAR ANIL [US]) 26 April 2012 (2012-04-26)<br>* the whole document *<br>----- | 1-12 | |
| X | JAMES A BAUM ET AL: "Control of coleopteran insect pests through RNA interference",<br>NATURE BIOTECHNOLOGY,<br>vol. 25, no. 11,<br>1 November 2007 (2007-11-01), pages 1322-1326, XP055477988,<br>us<br>ISSN: 1087-0156, DOI: 10.1038/nbt1359<br>* the whole document *<br>----- | 1-12 | |
| T | A. E. MARTINEZ DE ALBA ET AL: "Two Chloroplastic Viroids Induce the Accumulation of Small RNAs Associated with Posttranscriptional Gene Silencing",<br>JOURNAL OF VIROLOGY,<br>vol. 76, no. 24,<br>15 December 2002 (2002-12-15), pages 13094-13096, XP055645025,<br>US<br>ISSN: 0022-538X, DOI: 10.1128/JVI.76.24.13094-13096.2002<br>* the whole document *<br>-----<br>-/-- | | TECHNICAL FIELDS SEARCHED (IPC) |

The present search report has been drawn up for all claims

2

| Place of search | Date of completion of the search | Examiner |
|---|---|---|
| Munich | 13 July 2020 | Keller, Yves |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 3 of 5

Europäisches Patentamt
European Patent Office
Office européen des brevets

# EUROPEAN SEARCH REPORT

Application Number
EP 20 16 5687

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| T | R. RAJAGOPAL: "Silencing of Midgut Aminopeptidase N of Spodoptera litura by Double-stranded RNA Establishes Its Role as Bacillus thuringiensis Toxin Receptor", JOURNAL OF BIOLOGICAL CHEMISTRY, vol. 277, no. 49, 27 November 2002 (2002-11-27), pages 46849-46851, XP055019558, ISSN: 0021-9258, DOI: 10.1074/jbc.C200523200<br>* the whole document *<br>----- | | |
| T | YING-BO MAO ET AL: "Cotton plants expressingdouble-stranded RNA show enhanced resistance to bollworms", TRANSGENIC RESEARCH, KLUWER ACADEMIC PUBLISHERS-PLENUM PUBLISHERS, NE, vol. 20, no. 3, 17 October 2010 (2010-10-17), pages 665-673, XP019900633, ISSN: 1573-9368, DOI: 10.1007/S11248-010-9450-1<br>* the whole document *<br>----- | | TECHNICAL FIELDS SEARCHED (IPC) |
| T | MAO YING-BO ET AL: "Silencing a cotton bollworm P450 monooxygenase gene by plant-mediated RNAi impairs larval tolerance of gossypol", NATURE BIOTECHNOLOGY,, vol. 25, no. 11, 1 November 2007 (2007-11-01), pages 1307-1313, XP002524148, DOI: 10.1038/NBT1352<br>* the whole document *<br>-----<br>-/-- | | |

The present search report has been drawn up for all claims

| Place of search | Date of completion of the search | Examiner |
|---|---|---|
| Munich | 13 July 2020 | Keller, Yves |

2

EPO FORM 1503 03.82 (P04C01)

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

Europäisches Patentamt
European Patent Office
Office européen des brevets

# EUROPEAN SEARCH REPORT

Application Number
EP 20 16 5687

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| T | TIAN HONGGANG ET AL: "Developmental control of a lepidopteran pest Spodoptera exigua by ingestion of bacteria expressing dsRNA of a non-midgut gene", PLOS ONE, US, vol. 4, no. 7, 1 January 2009 (2009-01-01), pages E6225-1, XP002601508, ISSN: 1932-6203 * the whole document * ----- | | |
| T | Rna Processing ET AL: "RNA PROCESSING Edited by Paula Grabowski", , 1 August 2011 (2011-08-01), XP55644811, Retrieved from the Internet: URL:http://library.umac.mo/ebooks/b28055512.pdf [retrieved on 2019-11-20] * the whole document * ----- | | |
| | | | TECHNICAL FIELDS SEARCHED (IPC) |

The present search report has been drawn up for all claims

2

| Place of search | Date of completion of the search | Examiner |
|---|---|---|
| Munich | 13 July 2020 | Keller, Yves |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 5 of 5

## ANNEX TO THE EUROPEAN SEARCH REPORT ON EUROPEAN PATENT APPLICATION NO. EP 20 16 5687

This annex lists the patent family members relating to the patent documents cited in the above-mentioned European search report. The members are as contained in the European Patent Office EDP file on
The European Patent Office is in no way liable for these particulars which are merely given for the purpose of information.

13-07-2020

| Patent document cited in search report | Publication date | Patent family member(s) | Publication date |
|---|---|---|---|
| WO 2007104570 A2 | 20-09-2007 | US 2010068172 A1 | 18-03-2010 |
| | | WO 2007104570 A2 | 20-09-2007 |
| WO 2007011479 A2 | 25-01-2007 | CA 2627081 A1 | 25-01-2007 |
| | | EP 1941034 A2 | 09-07-2008 |
| | | US 2006200878 A1 | 07-09-2006 |
| | | US 2007083947 A1 | 12-04-2007 |
| | | US 2007083949 A1 | 12-04-2007 |
| | | US 2007083950 A1 | 12-04-2007 |
| | | US 2007083951 A1 | 12-04-2007 |
| | | US 2007083952 A1 | 12-04-2007 |
| | | US 2007089184 A1 | 19-04-2007 |
| | | US 2007089185 A1 | 19-04-2007 |
| | | US 2007089186 A1 | 19-04-2007 |
| | | US 2007089187 A1 | 19-04-2007 |
| | | US 2007089188 A1 | 19-04-2007 |
| | | US 2007089189 A1 | 19-04-2007 |
| | | US 2007089190 A1 | 19-04-2007 |
| | | US 2007089191 A1 | 19-04-2007 |
| | | US 2007089192 A1 | 19-04-2007 |
| | | US 2007089193 A1 | 19-04-2007 |
| | | US 2007089194 A1 | 19-04-2007 |
| | | US 2007089195 A1 | 19-04-2007 |
| | | US 2007089196 A1 | 19-04-2007 |
| | | US 2007094748 A1 | 26-04-2007 |
| | | US 2007113301 A1 | 17-05-2007 |
| | | US 2007113302 A1 | 17-05-2007 |
| | | US 2007118917 A1 | 24-05-2007 |
| | | US 2007118918 A1 | 24-05-2007 |
| | | US 2008229456 A1 | 18-09-2008 |
| | | US 2009013434 A1 | 08-01-2009 |
| | | US 2010223694 A1 | 02-09-2010 |
| | | US 2011035838 A1 | 10-02-2011 |
| | | US 2011035839 A1 | 10-02-2011 |
| | | US 2015184179 A1 | 02-07-2015 |
| | | US 2018002713 A1 | 04-01-2018 |
| | | WO 2007011479 A2 | 25-01-2007 |
| WO 2012054919 A2 | 26-04-2012 | AU 2011316776 A1 | 06-06-2013 |
| | | CA 2820536 A1 | 26-04-2012 |
| | | CL 2013001102 A1 | 04-04-2014 |
| | | CN 103269579 A | 28-08-2013 |
| | | EP 2629599 A2 | 28-08-2013 |
| | | US 2013315883 A1 | 28-11-2013 |
| | | WO 2012054919 A2 | 26-04-2012 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2012054919 A
- US 20090263364 A
- US 20070011775 A
- US 20080194504 A
- US 20060095987 A
- US 20120174258
- US 20120174259
- US 20120174260
- US 20120198586
- US 20130091600
- US 20130091601
- US 20130097730
- US 577811
- US 577854
- US 20120164205
- US 20110054007 A
- US 20090265818 A
- US 6326193 B
- EP 1210875 A
- EP 2633048 A
- WO 2011153418 A
- WO 2007080126 PCT
- WO 2012143542 PCT
- WO 2005110068 PCT
- WO 2006047495 PCT
- US 20050188438
- US 20060080749 A
- US 20090300796 A
- WO 2003052110 A
- WO 2004066183 PCT
- WO 2004005485 PCT
- WO 2005019408 PCT
- WO 2006046148 PCT
- WO 2007087153 PCT
- WO 2007095496 PCT
- WO 2009133126 PCT
- US 20020048814 A
- US 20030018993 A
- WO 9401550 A
- WO 9805770 PCT
- WO 9630530 A
- US 6437217 B
- US 5641876 A
- US 6426446 B
- US 6429362 B
- US 6232526 B
- US 6177611 B
- US 5322938 A
- US 5352605 A
- US 5359142 A
- US 5530196 A
- US 6433252 B
- US 6429357 B
- US 6294714 B
- US 6140078 A
- US 6252138 B
- US 6175060 B
- US 6388170 B
- US 6635806 B
- US 757089
- US 6051753 A
- US 5378619 A
- US 5850019 A
- US 6677503 B
- US 5110732 A
- US 5459252 A
- US 5837848 A
- US 5362865 A
- US 5659122 A
- US 5550318 A
- US 5633435 A
- US 5780708 A
- US 6118047 A
- WO 2007035650 A
- US 4945050 A
- US 6680426 B
- US 6551962 B
- US 20080289063 A1


### Non-patent literature cited in the description


- **TERENIUS O. et al.** *J. Insect Physiol.,* 2011, vol. 57, 231-245
- **FIRE et al.** *Nature,* 1998, vol. 391, 806-811
- **WATERHOUSE et al.** *Proc Natl Acad Sci USA,* 1998, vol. 95, 13959-139
- **WESLEY V et al.** *Plant J.,* 2001, vol. 27, 581-590
- **MARTINEZ et al.** *Cell,* 2002, vol. 110, 563-574
- **MCMANUS ; SHARP.** *Nature Rev. Genetics,* 2002, vol. 3, 737-747

- **MAO Y. et al.** *Nat. Biotechnol.,* 2007, vol. 25, 1307-1313
- **KUMAR P. et al.** *PLoS ONE,* 2012, vol. 7, e31347
- **BOGARAD L.** *TIBTECH,* 2000, vol. 18, 257-263
- **MCBRIDE K et al.** *Nature Biotechnology,* 1995, vol. 13, 362-365
- **VERMA D ; DANIELL H.** *Am Soc Plant Biol.,* 2007, vol. 145, 1129-1143
- **CASAS-MOLLANO J. et al.** *Genetics,* 2008, vol. 179, 69-81
- **STERN et al.** *Annu. Rev. Plant Biol.,* 2010, vol. 61, 125-55
- **MARTINEZ DE ALBA A.E. et al.** *Am. Soc Microbiol.,* 2002, vol. 76, 13094-13096
- **FENNER B. et al.** *J. Virol.,* 2006, vol. 80, 6822-6833
- **SALOMON W. et al.** *Nucl. Acids. Res.,* 2010, vol. 38, 3771-3779
- **WATERHOUSE PM et al.** *Proc Natl Acad Sci USA,* 1998, vol. 95, 13959-139
- **TIMMONS L ; FIRE A.** *Nature,* 1998, vol. 395, 854
- **SMITH NA et al.** *Nature,* 2000, vol. 407, 319-320
- **AINE L. PLANT ; JOHN C. GRAY.** *Photosynthesis Research,* 1988, vol. 16, 23-39
- **WARD et al.** *Plant Mol. Biol.,* 1993, vol. 22, 361-366
- **SCHENA et al.** *Proc. Natl. Acad. Sci. USA,* 1991, vol. 88, 0421
- **EBERT et al.** *Proc. Natl. Acad. Sci. USA,* 1987, vol. 84 (16), 5745-9
- **LAWTON et al.** *Plant Mol. Biol.,* 1987, vol. 9, 315-24
- **ODELL et al.** *Nature,* 1985, vol. 313, 810-2
- **WALKER et al.** *Proc. Natl. Acad. Sci. USA,* 1987, vol. 84 (19), 6624-8
- **YANG ; RUSSELL.** *Proc. Natl. Acad. Sci. USA,* 1990, vol. 87, 4144-8
- **CHANDLER et al.** *Plant Cell,* 1989, vol. 1, 1175-83
- **DEPICKER et al.** *J. Mol. Appl. Genet.,* 1982, vol. 1, 561-73
- **BEVAN et al.** *Nature,* 1983, vol. 304, 184-7
- **OPPERMAN et al.** *Science,* 1994, vol. 263, 221-3
- **HIREL et al.** *Plant Mol. Biol.,* 1992, vol. 20, 207-18
- **TURNER ; FOSTER.** *Molecular Biotech.,* 1995, vol. 3 (3), 225-36
- **CARRINGTON ; FREED.** *J. Virol.,* 1990, vol. 64, 1590-7
- **VERMA ; DANIELL.** *Plant Physiol.,* 2007, vol. 145, 1129-43
- **RASALA et al.** *Plant Biotech. J.,* 2011, vol. 9, 674-83
- **HANLEY-BOWDOIN ; CHUA.** *TIBS,* 1987, vol. 12, 67-70
- **MULLET et al.** *Plant Molec. Biol.,* 1985, vol. 4, 39-54
- **HANLEY-BOWDOIN.** PhD. Dissertation. The Rockefeller University, 1986
- **KREBBERS et al.** *Nucleic Acids Res.,* 1982, vol. 10, 4985-5002
- **ZURAWSKI et al.** *Nucleic Acids Res.,* 1981, vol. 9, 3251-3270
- **ZURAWSKI et al.** *Proc. Nat'l Acad Sci. U.S.A.,* 1982, vol. 79, 7699-7703
- **FRALEY et al.** *Proc. Natl. Acad. Sci. USA,* 1983, vol. 80, 4803-7
- **INGELBRECHT et al.** *Plant Cell,* 1989, vol. 1, 671-80
- **CORUZZI et al.** *EMBO J.,* 1984, vol. 3, 1671-9
- **JEFFERSON et al.** *Plant Mol. Biol. Rep.,* 1987, vol. 5, 387-405
- Molecular cloning of the maize R-nj allele by transposon tagging with Ac. **DELLAPORTA et al.** In 18th Stadler Genetics Symposium. Plenum, 1988, 263-82
- **SUTCLIFFE et al.** *Proc. Natl. Acad. Sci. USA,* 1978, vol. 75, 3737-41
- **OW et al.** *Science,* 1986, vol. 234, 856-9
- **ZUKOWSKI et al.** *Gene,* 1983, vol. 46 (2-3), 247-55
- **IKATU et al.** *Bio Technol.,* 1990, vol. 8, 241-2
- **KATZ et al.** *J. Gen. Microbiol.,* 1983, vol. 129, 2703-14
- **SANFORD.** *Trends In Biotech.,* 1988, vol. 6, 299-302
- **FROMM et al.** *Proc. Nat'l. Acad. Sci. (USA),* 1985, vol. 82, 5824-5828
- **FROMM et al.** *Nature,* 1986, vol. 319, 791-3
- **FEIGNER et al.** *Proc. Natl. Acad. Sci. USA,* 1987, vol. 84, 7413-7
- **MUELLER et al.** *Cell,* 1978, vol. 15, 579-85
- **KLEIN et al.** *Nature,* 1987, vol. 327, 70
- **FREYTAG et al.** *Plant Cell Rep.,* 1988, vol. 7, 30-34
- **SVAB et al.** *Proc. Nat'l Acad. Sci. U.S.A.,* 1990, 8526-8530
- **HANSON et al.** *Journal of Experimental Botany,* 2012, vol. 64, 753-768
- **GOLDS et al.** *Nature Biotechnology,* 1993, vol. 11, 95-97
- **RIOS, Q. et al.** *Plant J.,* 2002, vol. 32, 243-53
- Phil. Trans. R. Soc. Lond. B. The Royal Society, 1998, vol. 353, 1777-1786
- **VERMA ; DANIELLE.** *Plant Physiol.,* 2007, vol. 145 (4), 1129-1143
- **MALIGA.** Chloroplast Biotechnology. Humana Press, 2014

- **HUANG et al.** *Econ. Entomol.,* 2006, vol. 99, 194-202

SEQUENCE LISTING

<110> The University of Sydney

<120> RNA production in higher plants

<130> 392-3 PCTEPT1

<150> US 61/951,569
<151> 2014-03-12

<150> PCT/AU2015/050101
<151> 2015-03-12

<150> EP15761116.1
<151> 2015-03-12

<160> 18

<170> PatentIn version 3.5

<210> 1
<211> 28
<212> DNA
<213> Artificial Sequence

<220>
<223> Synthetic oligonucleotide g187f

<400> 1
cgctcgagcg gagtgagacc aattctcg 28

<210> 2
<211> 28
<212> DNA
<213> Artificial Sequence

<220>
<223> Synthetic oligonucleotide g188r

<400> 2
cgggtacccg gagtgagacc ctgtgtat 28

<210> 3
<211> 35
<212> DNA
<213> Artificial Sequence

<220>
<223> Synthetic oligonucleotide g216f/r

<400> 3
accaggtctc aggagcatgg agttggatga cagga 35

<210> 4
<211> 36
<212> DNA
<213> Artificial Sequence

<220>

<223> Synthetic oligonucleotide g217f/r

<400> 4
accaggtctc ctcgagctga cgggaatccc aatatg 36

<210> 5
<211> 20
<212> DNA
<213> Artificial Sequence

<220>
<223> Synthetic oligonucleotide g25f

<400> 5
tgatagatca tgtcattgtg 20

<210> 6
<211> 20
<212> DNA
<213> Artificial Sequence

<220>
<223> Synthetic oligonucleotide g165f

<400> 6
tgaagtagag aaaccggcgt 20

<210> 7
<211> 20
<212> DNA
<213> Artificial Sequence

<220>
<223> Synthetic oligonucleotide g166r

<400> 7
ccaagcagcc tgtttcgatt 20

<210> 8
<211> 87
<212> DNA
<213> Artificial Sequence

<220>
<223> Synthetic oligonucleotide g280r

<400> 8
aacgacccaa ttgcaagagc ggagctctac caactgagct atatcccccc gagccaagtg 60

gagcgcattt aaatagtgta ccataac 87

<210> 9
<211> 22
<212> DNA
<213> Artificial Sequence

<220>
<223> Synthetic oligonucleotide g276r

<400> 9
cgaaggataa ttacttgagt tc 22

<210> 10
<211> 72
<212> DNA
<213> Artificial Sequence

<220>
<223> Synthetic oligonucleotide g301r

<400> 10
tactcctact cataatgttt tgttactact cataactgtt tcttactctt tgatattatt 60

tgttacttta gc 72

<210> 11
<211> 24
<212> DNA
<213> Artificial Sequence

<220>
<223> Synthetic oligonucleotide BEN0002F

<400> 11
gcatgcaaat ctaagtgtag tgct 24

<210> 12
<211> 27
<212> DNA
<213> Artificial Sequence

<220>
<223> Synthetic oligonucleotide BEN0003R

<400> 12
catatgtcga atagtattca agatcct 27

<210> 13
<211> 2580
<212> DNA
<213> Helicoverpa armigera

<400> 13
acgcggggag tctactctgt cacgtcgatg gagacgcatc gcggtcggcc tctctctcaa 60

tattattact ttattaccat acaaaacgtg agtgagtgtc tgtgaactta tttcaacttg 120

agtgacggtt gctccggacc atacagcgag ctgacagttc agccaaggtc atcgatcagc 180

aatttgacac cagttgtgtt tatatactat atgtacgacc acaacagaat accgtgtgcc 240

cctagaaatg tgttttaaca agtatctcat taagctggcg gattacttca gatcagtttc 300

tgatacgtct cgttaatgcc cgtgcgaaca acaacattcg aaatgatcag caacacgaag 360

atcgtgttca ccaagctcct gctgtgctgc ttcgtgtcag gcgccgtcgc gaggtcgtgg 420

gccaaccatc acgacaccac cacgtcaacc acgcagacca cccccaccac tagtcctgtg 480
cctaagaact tccataatga tccactcatc gtcgaaacta aaagtggcct cgtcaaaggc 540
tacgctaaga cagttatggg cagggaagta cacatcttca ccggtattcc gtttgcgaag 600
ccccctctag gaccgttaag attccgcaaa ccggtaccca tcgacccgtg gcatggagta 660
cttgaagcca ctgcgatgcc aaatagctgt tatcaagaac ggtatgagta cttccccggt 720
tttgagggag aggaaatgtg gaatccaaac acaaatatat cagaggactg tctctatcta 780
aacatttggg ttccccagca cttacgagtc cgtcatcatc aagataagcc tcttgcggag 840
aggcccaaag tgccgatact agtgtggatt tacggtggcg gctatatgag tggcacggca 900
acactcaacc tatataaagc agacataatg gcttcttcca gtgatgtaat agtagcatct 960
atgcaatata gggttggggc gttcggattt ttatacttga ataaatattt ctcgccgggc 1020
agtgaagagg cgccgggaaa tatgggctta tgggatcaac aactcgctat tcgttggatt 1080
aaagataatg ctcgtgcttt tggtggtgac ccagaattga taactttgtt tggagagtcg 1140
gcaggcgggg gaagcgtgag tttgcatatg ctttcaccag agatgaaggg attattcaaa 1200
cgaggcatct tgcaatctgg aacgttaaac gctccatgga gttggatgac aggagagaga 1260
gcacaagaca taggaaaagt gttagtagat gactgtaatt gcaatagcag cctactagct 1320
gccgatccga gcttagtgat ggactgtatg cgcggagtcg atgcaaaaac tatatcagta 1380
cagcagtgga attcctatac tggcatattg ggattcccgt cagctcccac ggttgacggc 1440
gtgtttttgc ccaaagaccc ggacacaatg atgaaagaag gcaatttcca taataccgag 1500
gtgcttcttg gaagtaatca agacgaagga acctatttct tactatacga tttccttgac 1560
tacttcgaga aagacgggcc cagcttcttg cagcgggaga aatttctaga aattgtcgac 1620
accatattca aggatttctc caaaataaag agggaagcta tcgtattcca atatacggac 1680
tgggaggaaa ttaccgatgg ctatctgaac cagaaaatga tagctgacgt ggtgggcgac 1740
tatttcttcg tgtgccctac taactacttc gcagaagtgc tggccgattc aggagttgat 1800
gtttactact attacttcac acatcgcacc agcacaagtc tctggggtga atggatgggc 1860
gtgatgcacg gcgatgagat ggagtacgtc ttcgggcacc cgctgaacat gtcccttcaa 1920
taccacacca gggagaggga ccttgctgca catatcatgc agtcttttac gagattcgct 1980
ttgactggca aacctcacaa gccggacgag aaatggccgc tgtactctcg cacgtcgccc 2040
cactactaca cttacaccgc ggacggggct agcgggccag ccggaccgcg cgggcctagg 2100
gcctccgctt gcgctttctg gaatgatttc cttaacaagc tgaatgagct ggagcacatg 2160
ccatgtgacg ggccgtgac cggcctttac agcagcgtcg ccggcaccac cctgccgata 2220
gtgctgctga ccacgctcgc caccaccgtc gcactctaag cgaccgacat tcactcgtag 2280
caaacagtag acaaattatt tatacaaact agaaaaatac cgatgcaaat aaatgttttc 2340

acttcatcta taaccataaa gtcaaaaatc ttatctgaaa tgaactttat gattgtcaat 2400

gaagatctag agtacgcaaa tcgtatttcg aattttaata tcggctacta aagtactaga 2460

agtaaattgt tgtattaatt aaatatattt tatcacaatc ttacatatca agcgtgtgga 2520

aagaaaacgg gacagtgaca aggaacgtta caaaaaaaaa aaaaaaaaaa aaaaaaaaaa 2580

<210> 14
<211> 189
<212> DNA
<213> Helicoverpa armigera

<400> 14
catggagttg gatgacagga gagagagcac aagacatagg aaaagtgtta gtagatgact 60

gtaattgcaa tagcagccta ctagctgccg atccgagctt agtgatggac tgtatgcgcg 120

gagtcgatgc aaaaactata tcagtacagc agtggaattc ctatactggc atattgggat 180

tcccgtcag 189

<210> 15
<211> 2011
<212> DNA
<213> Artificial Sequence

<220>
<223> Inverted repeat nucleotide sequence derived from Helicoverpa armigera

<400> 15
catggagttg gatgacagga gagagagcac aagacatagg aaaagtgtta gtagatgact 60

gtaattgcaa tagcagccta ctagctgccg atccgagctt agtgatggac tgtatgcgcg 120

gagtcgatgc aaaaactata tcagtacagc agtggaattc ctatactggc atattgggat 180

tcccgtcaga cgagcccttg gtaaggaaat aattattttc ttttttcctt ttagtataaa 240

atagttaagt gatgttaatt agtatgatta taataatata gttgttataa ttgtgaaaaa 300

ataatttata aatatattgt ttacataaac aacatagtaa tgtaaaaaaa tatgacaagt 360

gatgtgtaag acgaagaaga taaaagttga gagtaagtat attattttta atgaatttga 420

tcgaacatgt aagatgatat acggccggta agaggttcca actttcacca taatgaaata 480

agatcactac cgggcgtatt ttttgagtta tcgagatttt caggagctaa ggaagctaaa 540

atggagaaaa aaatcactgg atataccacc gttgatatat cccaatggca tcgtaaagaa 600

cattttgagg catttcagtc agttgctcaa tgtacctata accagaccgt tcagctggat 660

attacggcct ttttaaagac cgtaaagaaa aataagcaca agttttatcc ggcctttatt 720

cacattcttg cccgcctgat gaatgctcat ccggaattcc gtatggcaat gaaagacggt 780

gagctggtga tatgggatag tgttcaccct tgttacaccg ttttccatga gcaaactgaa 840

acgttttcat cgctctggag tgaataccac gacgatttcc ggcagtttct acacatatat 900

tcgcaagatg tggcgtgtta cggtgaaaac ctggcctatt tccctaaagg gtttattgag 960

aatatgtttt tcgtctcagc caatccctgg gtgagtttca ccagttttga tttaaacgtg 1020

gccaatatgg acaacttctt cgcccccgtt ttcaccatgg gcaaatatta tacgcaaggc 1080

gacaaggtgc tgatgccgct ggcgattcag gttcatcatg ccgtctgtga tggcttccat 1140

gtcggcagaa tgcttaatga attacaacag tactgcgatg agtggcaggg cggggcgtaa 1200

tcgcgtggat ccggcttact aaaagccaga taacagtatg cgtatttgcg cgctgatttt 1260

tgcggtataa gaatatatac tgatatgtcg gtcccataat agtaattcta gctggtttga 1320

tgaattaaat atcaatgata aaatactata gtaaaaataa gaataaataa attaaaataa 1380

tatttttta tgattaatag tttattatat aattaaatat ctataccatt actaaatatt 1440

ttagtttaaa agttaataaa tattttgtta gaaattccaa tctgcttgta atttatcaat 1500

aaacaaaata ttaaataaca agctaaagta acaaataata tcaaactaat agaaacagta 1560

atctaatgta acaaaacata atctaatgct aatataacaa agcgcaagat ctatcatttt 1620

atatagtatt attttcaatc aacattctta ttaatttcta aataatactt gtagttttat 1680

taacttctaa atggattgac tattaattaa atgaattagt cgaacatgaa taaacaaggt 1740

aacatgatag atcatgtcat tgtgttatca ttgatcttac atttggattg attacagttg 1800

gtctagagat ttcgtctaga tcgtctgacg ggaatcccaa tatgccagta taggaattcc 1860

actgctgtac tgatatagtt tttgcatcga ctccgcgcat acagtccatc actaagctcg 1920

gatcggcagc tagtaggctg ctattgcaat tacagtcatc tactaacact tttcctatgt 1980

cttgtgctct ctctcctgtc atccaactcc a 2011

<210> 16
<211> 8610
<212> DNA
<213> Artificial Sequence

<220>
<223> V153 vector sequence

<400> 16
gatcgttcaa acatttggca ataaagtttc ttaagattga atcctgttgc cggtcttgcg 60

atgattatca tataatttct gttgaattac gttaagcatg taataattaa catgtaatgc 120

atgacgttat ttatgagatg ggtttttatg attagagtcc cgcaattata catttaatac 180

gcgatagaaa acaaaatata gcgcgcaaac taggataaat tatcgcgcgc ggtgtcatct 240

atgttactag atccctaggg aagttcctat tccgaagttc ctattctctg aaaagtatag 300

gaacttcttt gcgtattggg cgctcttggc ctttttggcc accggtcgta cggttaaaac 360

caccccagta cattaaaaac gtccgcaatg tgttattaag ttgtctaagc gtcaatttgt 420

ttacaccaca atatatcctg ccaccagcca gccaacagct ccccgaccgg cagctcggca 480

caaaatcacc actcgataca ggcagcccat cagtccacta gacgctcacc gggctggttg 540

ccctcgccgc tgggctggcg gccgtctatg gccctgcaaa cgcgccagaa acgccgtcga 600

agccgtgtgc gagacaccgc agccgccggc gttgtggata cctcgcggaa aacttggccc 660

tcactgacag atgaggggcg gacgttgaca cttgaggggc cgactcaccc ggcgcggcgt 720

tgacagatga ggggcaggct cgatttcggc cggcgacgtg gagctggcca gcctcgcaaa 780

tcggcgaaaa cgcctgattt tacgcgagtt tcccacagat gatgtggaca agcctgggga 840

taagtgccct gcggtattga cacttgaggg gcgcgactac tgacagatga ggggcgcgat 900

ccttgacact tgaggggcag agtgctgaca gatgaggggc gcacctattg acatttgagg 960

ggctgtccac aggcagaaaa tccagcattt gcaagggttt ccgcccgttt ttcggccacc 1020

gctaacctgt cttttaacct gcttttaaac caatatttat aaaccttgtt tttaaccagg 1080

gctgcgccct gtgcgcgtga ccgcgcacgc cgaagggggg tgcccccct tctcgaaccc 1140

tcccggcccg ctctcgcgtt ggcagcatca cccataattg tggtttcaaa atcggctccg 1200

tcgatactat gttatacgcc aactttgaaa acaactttga aaaagctgtt ttctggtatt 1260

taaggtttta gaatgcaagg aacagtgaat tggagttcgt cttgttataa ttagcttctt 1320

ggggtatctt taaatactgt agaaaagagg aaggaaataa taaatggcta aaatgagaat 1380

atcaccggaa ttgaaaaaac tgatcgaaaa ataccgctgc gtaaaagata cggaaggaat 1440

gtctcctgct aaggtatata agctggtggg agaaaatgaa aacctatatt taaaaatgac 1500

ggacagccgg tataaaggga ccacctatga tgtggaacgg gaaaaggaca tgatgctatg 1560

gctggaagga aagctgcctg ttccaaaggt cctgcacttt gaacggcatg atggctggag 1620

caatctgctc atgagtgagg ccgatggcgt cctttgctcg gaagagtatg aagatgaaca 1680

aagccctgaa aagattatcg agctgtatgc ggagtgcatc aggctctttc actccatcga 1740

catatcggat tgtccctata cgaatagctt agacagccgc ttagccgaat tggattactt 1800

actgaataac gatctggccg atgtggattg cgaaaactgg gaagaagaca ctccatttaa 1860

agatccgcgc gagctgtatg atttttaaa gacggaaaag cccgaagagg aacttgtctt 1920

ttcccacggc gacctgggag acagcaacat ctttgtgaaa gatggcaaag taagtggctt 1980

tattgatctt gggagaagcg gcagggcgga caagtggtat gacattgcct tctgcgtccg 2040

gtcgatcagg gaggatattg gggaagaaca gtatgtcgag ctattttttg acttactggg 2100

gatcaagcct gattgggaga aaataaaata ttatatttta ctggatgaat tgttttagta 2160

cctagatgtg gcgcaacgat gccggcgaca agcaggagcg caccgacttc ttccgcatca 2220

agtgttttgg ctctcaggcc gaggcccacg gcaagtattt gggcaagggg tcgctggtat 2280

tcgtgcaggg caagattcgg aataccaagt acgagaagga cggccagacg gtctacggga 2340
ccgacttcat tgccgataag gtggattatc tggacaccaa ggcaccaggc gggtcaaatc 2400
aggaataagg gcacattgcc ccggcgtgag tcggggcaat cccgcaagga gggtgaatga 2460
atcggacgtt tgaccggaag gcatacaggc aagaactgat cgacgcgggg ttttccgccg 2520
aggatgccga aaccatcgca agccgcaccg tcatgcgtgc gccccgcgaa accttccagt 2580
ccgtcggctc gatggtccag caagctacgg ccaagatcga gcgcgacagc gtgcaactgg 2640
ctccccctgc cctgcccgcg ccatcggccg ccgtggagcg ttcgcgtcgt ctcgaacagg 2700
aggcggcagg tttggcgaag tcgatgacca tcgacacgcg aggaactatg acgaccaaga 2760
agcgaaaaac cgccggcgag gacctggcaa aacaggtcag cgaggccaag caagccgcgt 2820
tgctgaaaca cacgaagcag cagatcaagg aaatgcagct ttccttgttc gatattgcgc 2880
cgtggccgga cacgatgcga gcgatgccaa acgacacggc ccgctctgcc ctgttcacca 2940
cgcgcaacaa gaaaatcccg cgcgaggcgc tgcaaaacaa ggtcattttc cacgtcaaca 3000
aggacgtgaa gatcacctac accggcgtcg agctgcgggc cgacgatgac gaactggtgt 3060
ggcagcaggt gttggagtac gcgaagcgca cccctatcgg cgagccgatc accttcacgt 3120
tctacgagct ttgccaggac ctgggctggt cgatcaatgg ccggtattac acgaaggccg 3180
aggaatgcct gtcgcgccta caggcgacgg cgatgggctt cacgtccgac cgcgttgggc 3240
acctggaatc ggtgtcgctg ctgcaccgct tccgcgtcct ggaccgtggc aagaaaacgt 3300
cccgttgcca ggtcctgatc gacgaggaaa tcgtcgtgct gtttgctggc gaccactaca 3360
cgaaattcat atgggagaag taccgcaagc tgtcgccgac ggcccgacgg atgttcgact 3420
atttcagctc gcaccgggag ccgtacccgc tcaagctgga aaccttccgc ctcatgtgcg 3480
gatcggattc cacccgcgtg aagaagtggc gcgagcaggt cggcgaagcc tgcgaagagt 3540
tgcgaggcag cggcctggtg gaacacgcct gggtcaatga tgacctggtg cattgcaaac 3600
gctagggcct tgtggggtca gttccggctg ggggttcagc agccagcgct ttactgagat 3660
cctcttccgc ttcctcgctc actgactcgc tgcgctcggt cgttcggctg cggcgagcgg 3720
tatcagctca ctcaaaggcg gtaatacggt tatccacaga atcaggggat aacgcaggaa 3780
agaacatgtg agcaaaaggc cagcaaaagg ccaggaaccg taaaaaggcc gcgttgctgg 3840
cgtttttcca taggctccgc ccccctgacg agcatcacaa aaatcgacgc tcaagtcaga 3900
ggtggcgaaa cccgacagga ctataaagat accaggcgtt tccccctgga agctccctcg 3960
tgcgctctcc tgttccgacc ctgccgctta ccggatacct gtccgccttt ctcccttcgg 4020
gaagcgtggc gctttctcat agctcacgct gtaggtatct cagttcggtg taggtcgttc 4080
gctccaagct gggctgtgtg cacgaacccc ccgttcagcc cgaccgctgc gccttatccg 4140
gtaactatcg tcttgagtcc aacccggtaa gacacgactt atcgccactg gcagcagcca 4200

ctggtaacag gattagcaga gcgaggtatg taggcggtgc tacagagttc ttgaagtggt 4260

ggcctaacta cggctacact agaagaacag tatttggtat ctgcgctctg ctgaagccag 4320

ttaccttcgg aaaaagagtt ggtagctctt gatccggcaa acaaaccacc gctggtagcg 4380

gtggtttttt tgtttgcaag cagcagatta cgcgcagaaa aaaaggatct caagaagatc 4440

ctttgatctt ttctacgggg tctgacgctc agtggaacga aaactcacgt taagggattt 4500

tggtcatgag attatcaaaa aggatcttca cctagatcct tttggatctc ctgtggttgg 4560

catgcacata caaatggacg aacggataaa ccttttcacg ccctttaaa tatccgatta 4620

ttctaataaa cgctcttttc tcttaggttt acccgccaat atatcctgtc aaacactgat 4680

agtttaaact gaaggcggga aacgacaatc tgctagtgga tctcccagtc acgacgttgt 4740

aaaacgggcg ccccgcggaa agcttgctag ctgagacttt tcaacaaagg ataatttcgg 4800

gaaacctcct cggattccat tgcccagcta tctgtcactt catcgaaagg acagtagaaa 4860

aggaaggtgg ctcctacaaa tgccatcatt gcgataaagg aaaggctatc attcaagatc 4920

tctctgccga cagtggtccc aaagatggac ccccacccac gaggagcatc gtggaaaaag 4980

aagacgttcc aaccacgtct tcaaagcaag tggattgatg tgacatctcc actgacgtaa 5040

gggatgacgc acaatcccac tatccttcgc aagacccttc ctctatataa ggaagttcat 5100

ttcatttgga gaggactcga ggggcccgga gcatggagtt ggatgacagg agagagagca 5160

caagacatag gaaaagtgtt agtagatgac tgtaattgca atagcagcct actagctgcc 5220

gatccgagct tagtgatgga ctgtatgcgc ggagtcgatg caaaaactat atcagtacag 5280

cagtggaatt cctatactgg catattggga ttcccgtcag acgagccctt ggtaaggaaa 5340

taattatttt ctttttcct tttagtataa aatagttaag tgatgttaat tagtatgatt 5400

ataataatat agttgttata attgtgaaaa aataatttat aaatatattg tttacataaa 5460

caacatagta atgtaaaaaa atatgacaag tgatgtgtaa gacgaagaag ataaaagttg 5520

agagtaagta tattattttt aatgaatttg atcgaacatg taagatgata tacggccggt 5580

aagaggttcc aactttcacc ataatgaaat aagatcacta ccgggcgtat tttttgagtt 5640

atcgagattt tcaggagcta aggaagctaa aatggagaaa aaaatcactg gatataccac 5700

cgttgatata tcccaatggc atcgtaaaga acattttgag gcatttcagt cagttgctca 5760

atgtacctat aaccagaccg ttcagctgga tattacggcc tttttaaaga ccgtaaagaa 5820

aaataagcac aagttttatc cggcctttat tcacattctt gcccgcctga tgaatgctca 5880

tccggaattc cgtatggcaa tgaaagacgg tgagctggtg atatgggata gtgttcaccc 5940

ttgttacacc gttttccatg agcaaactga aacgttttca tcgctctgga gtgaatacca 6000

cgacgatttc cggcagtttc tacacatata ttcgcaagat gtggcgtgtt acggtgaaaa 6060

cctggcctat ttccctaaag ggtttattga gaatatgttt ttcgtctcag ccaatccctg 6120

ggtgagtttc accagttttg atttaaacgt ggccaatatg gacaacttct tcgcccccgt 6180

tttcaccatg ggcaaatatt atacgcaagg cgacaaggtg ctgatgccgc tggcgattca 6240

ggttcatcat gccgtctgtg atggcttcca tgtcggcaga atgcttaatg aattacaaca 6300

gtactgcgat gagtggcagg gcggggcgta atcgcgtgga tccggcttac taaaagccag 6360

ataacagtat gcgtatttgc gcgctgattt ttgcggtata agaatatata ctgatatgtc 6420

ggtcccataa tagtaattct agctggtttg atgaattaaa tatcaatgat aaaatactat 6480

agtaaaaata agaataaata aattaaaata atattttttt atgattaata gtttattata 6540

taattaaata tctataccat tactaaatat tttagtttaa aagttaataa atattttgtt 6600

agaaattcca atctgcttgt aatttatcaa taaacaaaat attaaataac aagctaaagt 6660

aacaaataat atcaaactaa tagaaacagt aatctaatgt aacaaaacat aatctaatgc 6720

taatataaca aagcgcaaga tctatcattt tatatagtat tattttcaat caacattctt 6780

attaatttct aaataatact tgtagtttta ttaacttcta aatggattga ctattaatta 6840

aatgaattag tcgaacatga ataaacaagg taacatgata gatcatgtca ttgtgttatc 6900

attgatctta catttggatt gattacagtt ggtctagaga tttcgtctag atcgtctgac 6960

gggaatccca atatgccagt ataggaattc cactgctgta ctgatatagt ttttgcatcg 7020

actccgcgca tacagtccat cactaagctc ggatcggcag ctagtaggct gctattgcaa 7080

ttacagtcat ctactaacac ttttcctatg tcttgtgctc tctctcctgt catccaactc 7140

catgctccga gctcggtacc gctagccgta ccttttacta gtgatatccc tgtgtgaaat 7200

tgttatccgc tacgcgtgat cgttcaaaca tttggcaata aagtttctta agattgaatc 7260

ctgttgccgg tcttgcgatg attatcatat aatttctgtt gaattacgtt aagcatgtaa 7320

taattaacat gtaatgcatg acgttattta tgagatgggt ttttatgatt agagtcccgc 7380

aattatacat ttaatacgcg atagaaaaca aaatatagcg cgcaaactag gataaattat 7440

cgcgcgcggt gtcatctatg ttactagatc ccatgggaag ttcctattcc gaagttccta 7500

ttctctgaaa agtataggaa cttcagcgat cgcagacgtc gggatcttct gcaagcatct 7560

ctatttcctg aaggtctaac ctcgaagatt taagatttaa ttacgtttat aattacaaaa 7620

ttgattctag tatctttaat ttaatgctta tacattatta attaatttag tactttcaat 7680

ttgttttcag aaattatttt actatttttt ataaaataaa agggagaaaa tggctattta 7740

aatactagcc tattttattt caattttagc ttaaaatcag ccccaattag ccccaatttc 7800

aaattcaaat ggtccagccc aattcctaaa taacccaccc ctaacccgcc cggtttcccc 7860

ttttgatcca tgcagtcaac gcccagaatt tccctatata atttttttaat tcccaaacac 7920

ccctaactct atcccatttc tcaccaaccg ccacatagat ctatcctctt atctctcaaa 7980

ctctctcgaa ccttccccta accctagcag cctctcatca tcctcacctc aaaacccacc 8040

ggggccggcc atgtctccgg agagaaggcc agttgagatt aggccagcta cagcagctga 8100

tatggccgct gtttgtgaca tcgttaacca ttacattgag acttctacag tgaactttag 8160

gacagagcca caaacaccac aagagtggat tgatgatctt gagaggttgc aagatagata 8220

cccttggttg gttgctgagg ttgagggtgt tgtggctggt attgcttacg ctggaccttg 8280

gaaggctagg aacgcttacg attggacagt tgagagtact gtttacgtgt cacataggca 8340

tcaaaggttg ggcctcggat ctacattgta cacacatttg cttaagtcta tggaggcgca 8400

aggttttaag tctgtggttg ctgttattgg ccttccaaac gatccatctg ttaggttgca 8460

tgaggctttg ggatacacag ccaggggtac attgcgcgca gctggataca agcatggtgg 8520

atggcatgat gttggttttt ggcaaaggga ttttgagttg ccagctcctc caaggccagt 8580

tagaccagtt acccagatct gaggcgcgcc 8610

<210> 17
<211> 8855
<212> DNA
<213> Artificial Sequence

<220>
<223> V206(modified) vector sequence

<400> 17
gggccttgta cacaccgccc gtcacactat gggagctggc catgcccgaa gtcgttacct 60

taaccgcaag gagggggatg ccgaaggcag ggctagtgac tggagtgaag tcgtaacaag 120

gtagccgtac tggaaggtgc ggctggatca cctccttttc agggagagct aatgcttgtt 180

gggtattttg gtttgacact gcttcacacc cccaaaaaaa agaagggagc tacgtctgag 240

ttaaacttgg agatggaagt cttctttcct ttctcgacgg tgaagtaaga ccaagctcat 300

gagcttatta tcctaggtcg gaacaagttg ataggacccc ctttttacg tccccatgtt 360

ccccccgtgt ggcgacatgg gggcgaaaaa aggaaagaga gggatggggt ttctctcgct 420

tttggcatag cgggcccccca gtgggaggct cgcacgacgg gctattagct cagtggtaga 480

gcgcgcccct gataattgcg tcgttgtgcc tgggctgtga gggctctcag ccacatggat 540

agttcaatgt gctcatcggc gcctgaccct gagatgtgga tcatccaagg cacattagca 600

tggcgtactc ctcctgttcg aaccggggtt tgaaaccaaa ctcctcctca ggaggataga 660

tggggcgatt cgggtgagat ccaatgtaga tccaactttc gattcactcg tgggatccgg 720

gcggtccggg ggggaccacc acggctcctc tcttctcgag aatccataca tcccttatca 780

gtgtatggac agctatctct cgagcacagg tttagcaatg ggaaaataaa atggagcacc 840

taacaacgca tcttcacaga ccaagaacta cgagatcgcc cctttcattc tggggtgacg 900

gagggatcgt accattcgag ccgttttttt cttgactcga aatgggagca ggtttgaaaa 960
aggatcttag agtgtctagg gttgggccag gagggtctct taacgccttc ttttttcttc 1020
tcatcggagt tatttcacaa agacttgcca gggtaaggaa gaaggggga acaagcacac 1080
ttggagagcg cagtacaacg gagagttgta tgctgcgttc gggaaggatg aatcgctccc 1140
gaaaaggaat ctattgattc tctcccaatt ggttggaccg taggtgcgat gatttacttc 1200
acgggcgagg tctctggttc aagtccagga tggcccagct gcatttaaat ggcgcgccgc 1260
tccccccgccg tcgttcaatg agaatggata agaggctcgt gggattgacg tgagggggca 1320
gggatggcta tatttctggg agacgcgtat gtatttggca aatcaaatac catggtctaa 1380
taatcaaaca ttctgattag ttgataatat tagtattagt tggaaatttt gtgaaagatt 1440
cctgtgaaaa gtttcattaa cacggaattc gtgtcgagta gaccttgttg ttgtgagaat 1500
tcttaattca tgagttgtag ggagggattt cttaagctcg agggatccgt cgaggggccc 1560
ggagcatgga gttggatgac aggagagaga gcacaagaca taggaaaagt gttagtagat 1620
gactgtaatt gcaatagcag cctactagct gccgatccga gcttagtgat ggactgtatg 1680
cgcggagtcg atgcaaaaac tatatcagta cagcagtgga attcctatac tggcatattg 1740
ggattcccgt cagacgagcc cttggtaagg aaataattat tttctttttt ccttttagta 1800
taaaatagtt aagtgatgtt aattagtatg attataataa tatagttgtt ataattgtga 1860
aaaaataatt tataaatata ttgtttacat aaacaacata gtaatgtaaa aaaatatgac 1920
aagtgatgtg taagacgaag aagataaaag ttgagagtaa gtatattatt tttaatgaat 1980
ttgatcgaac atgtaagatg atatacggcc ggtaagaggt tccaactttc accataatga 2040
aataagatca ctaccgggcg tattttttga gttatcgaga ttttcaggag ctaaggaagc 2100
taaaatggag aaaaaaatca ctggatatac caccgttgat atatcccaat ggcatcgtaa 2160
agaacatttt gaggcatttc agtcagttgc tcaatgtacc tataaccaga ccgttcagct 2220
ggatattacg gcctttttaa agaccgtaaa gaaaaataag cacaagtttt atccggcctt 2280
tattcacatt cttgcccgcc tgatgaatgc tcatccggaa ttccgtatgg caatgaaaga 2340
cggtgagctg gtgatatggg atagtgttca cccttgttac accgttttcc atgagcaaac 2400
tgaaacgttt tcatcgctct ggagtgaata ccacgacgat ttccggcagt ttctacacat 2460
atattcgcaa gatgtggcgt gttacggtga aaacctggcc tatttcccta aagggtttat 2520
tgagaatatg tttttcgtct cagccaatcc ctgggtgagt ttcaccagtt ttgatttaaa 2580
cgtggccaat atggacaact tcttcgcccc cgttttcacc atgggcaaat attatacgca 2640
aggcgacaag gtgctgatgc cgctggcgat tcaggttcat catgccgtct gtgatggctt 2700
ccatgtcggc agaatgctta atgaattaca acagtactgc gatgagtggc agggcggggc 2760
gtaatcgcgt ggatccggct tactaaaagc cagataacag tatgcgtatt tgcgcgctga 2820

tttttgcggt ataagaatat atactgatat gtcggtccca taatagtaat tctagctggt 2880

ttgatgaatt aaatatcaat gataaaatac tatagtaaaa ataagaataa ataaattaaa 2940

ataatatttt tttatgatta atagtttatt atataattaa atatctatac cattactaaa 3000

tattttagtt taaaagttaa taaatatttt gttagaaatt ccaatctgct tgtaatttat 3060

caataaacaa aatattaaat aacaagctaa agtaacaaat aatatcaaac taatagaaac 3120

agtaatctaa tgtaacaaaa cataatctaa tgctaatata acaaagcgca agatctatca 3180

ttttatatag tattattttc aatcaacatt cttattaatt tctaaataat acttgtagtt 3240

ttattaactt ctaaatggat tgactattaa ttaaatgaat tagtcgaaca tgaataaaca 3300

aggtaacatg atagatcatg tcattgtgtt atcattgatc ttacatttgg attgattaca 3360

gttggtctag agatttcgtc tagatcgtct gacgggaatc ccaatatgcc agtataggaa 3420

ttccactgct gtactgatat agtttttgca tcgactccgc gcatacagtc catcactaag 3480

ctcggatcgg cagctagtag gctgctattg caattacagt catctactaa cacttttcct 3540

atgtcttgtg ctctctctcc tgtcatccaa ctccatgctc cgagctcggt accgctagca 3600

agcttagaca ttagcagata aattagcagg aaataaagaa ggataaggag aaagaactca 3660

agtaattatc cttcgttctc ttaattgaat tgcaattaaa ctcggcccaa tcttttacta 3720

aaaggattga gccgaataca acaaagattc tattgcatat attttgacta agtatatact 3780

tacctagata tacaagattt gaaatacaaa atctagtcta gagcggccgc agtagcttat 3840

aacttcgtat agcatacatt atacgaagtt atagatccgc tcccccgccg tcgttcaatg 3900

agaatggata agaggctcgt gggattgacg tgaggggca gggatggcta tatttctggg 3960

agaattaacc gatcgacgtg caagcggaca tttattttaa attcgataat tttgcaaaa 4020

acatttcgac atatttattt attttattat tatgagaatc aatcctacta cttctggttc 4080

tggggtttcc acggctagta gcgaagcggt gatcgccgaa gtatcgactc aactatcaga 4140

ggtagttggc gtcatcgagc gccatctcga accgacgttg ctggccgtac atttgtacgg 4200

ctccgcagtg gatggcggcc tgaagccaca cagtgatatt gatttgctgg ttacggtgac 4260

cgtaaggctt gatgaaacaa cgcggcgagc tttgatcaac gaccttttgg aaacttcggc 4320

ttcccctgga gagagcgaga ttctccgcgc tgtagaagtc accattgttg tgcacgacga 4380

catcattccg tggcgttatc cagctaagcg cgaactgcaa tttggagaat ggcagcgcaa 4440

tgacattctt gcaggtatct tcgagccagc cacgatcgac attgatctgg ctatcttgct 4500

gacaaaagca agagaacata gcgttgcctt ggtaggtcca gcggcggagg aactctttga 4560

tccggttcct gaacaggatc tatttgaggc gctaaatgaa accttaacgc tatggaactc 4620

gccgcccgac tgggctggcg atgagcgaaa tgtagtgctt acgttgtccc gcatttggta 4680

cagcgcagta accggcaaaa tcgcgccgaa ggatgtcgct gccgactggg caatggagcg 4740
cctgccggcc cagtatcagc ccgtcatact tgaagctaga caggcttatc ttggacaaga 4800
agaagatcgc ttggcctcgc gcgcagatca gttggaagaa tttgtccact acgtgaaagg 4860
cgagatcacc aaggtagtgg gcaaagaaca aaaactcatt tctgaagaag acttgtgagt 4920
ctagctagag cgatcctggc ctagtctata ggaggttttg aaaagaaagg agcaataatc 4980
atttcttgt tctatcaaga gggtgctatt gctcctttct tttttcttt ttatttattt 5040
actagtattt tacttacata gactttttg tttacattat agaaaaagaa ggagaggtta 5100
ttttcttgca tttattcatg gggatcaaa gctgatctat aacttcgtat agcatacatt 5160
atacgaagtt atggtacact atttaaatgc gctccacttg gctcgggggg atatagctca 5220
gttggtagag ctccgctctt gcaattgggt cgttgcgatt acgggttgga tgtctaattg 5280
tccaggcggt aatgatagta tcttgtacct gaaccggtgg ctcacttttt ctaagtaatg 5340
gggaagagga ccgaaacgtg ccactgaaag actctactga gacaaagatg ggctgtcaag 5400
aacgtagagg aggtaggatg ggcagttggt cagatctagt atggatcgta catggacggt 5460
agttggagtc ggcggctctc ccagggttcc ctcatctgag atctctgggg aagaggatca 5520
agttggccct tgcgaacagc ttgatgcact atctcccttc aacccttga gcgaaatgcg 5580
gcaaaagaaa aggaaggaaa atccatggac cgaccccatc atctccaccc cgtaggaact 5640
acgagatcac cccaaggacg ccttcggcat ccaggggtca cggaccgacc atagaaccct 5700
gttcaataag tggaacgcat tagctgtccg ctctcaggtt gggcagtcag ggtcggagaa 5760
gggcaatgac tcattcttag ttagaatggg attccaactc agcacctttt gagtgagatt 5820
ttgagaagag ttgctctttg gagagcacag tacgatgaaa gttgtaagct gtgttcgggg 5880
gggagttatt gtctatcgtt ggcctctatg gtagaatcag tcgggggacc tgagaggcgg 5940
tggtttaccc tgcggcggat gtcagcggtt cgagtccgct tatctccaac tcgtgaactt 6000
agccgataca aagctctggc gtaatagcga agaggcccgc accgatcgcc cttcccaaca 6060
gttgcgcagc ctgaatggcg aatggcgcct gatgcggtat tttctcctta cgcatctgtg 6120
cggtatttca caccgcatac gtcaaagcaa ccatagtacg cgccctgtag cggcgcatta 6180
agcgcggcgg gtgtggtggt tacgcgcagc gtgaccgcta cacttgccag cgccctagcg 6240
cccgctcctt tcgctttctt cccttccttt ctcgccacgt tcgccggctt tccccgtcaa 6300
gctctaaatc gggggctccc tttagggttc cgatttagtg ctttacggca cctcgacccc 6360
aaaaaacttg atttgggtga tggttcacgt agtgggccat cgccctgata gacggttttt 6420
cgccctttga cgttggagtc cacgttcttt aatagtggac tcttgttcca aactggaaca 6480
acactcaacc ctatctcggg ctattctttt gatttataag ggattttgcc gatttcggcc 6540
tattggttaa aaaatgagct gatttaacaa aaatttaacg cgaattttaa caaaatatta 6600

acgtttacaa ttttatggtg cactctcagt acaatctgct ctgatgccgc atagttaagc 6660
cagccccgac acccgccaac acccgctgac gcgccctgac gggcttgtct gctcccggca 6720
tccgcttaca gacaagctgt gaccgtctcc gggagctgca tgtgtcagag gttttcaccg 6780
tcatcaccga aacgcgcgag acgaaagggc ctcgtgatac gcctattttt ataggttaat 6840
gtcatgataa taatggtttc ttagacgtca ggtggcactt ttcggggaaa tgtgcgcgga 6900
acccctattt gtttattttt ctaaatacat tcaaatatgt atccgctcat gagacaataa 6960
ccctgataaa tgcttcaata atattgaaaa aggaagagta tgagtattca acatttccgt 7020
gtcgccctta ttcccttttt tgcggcattt tgccttcctg tttttgctca cccagaaacg 7080
ctggtgaaag taaaagatgc tgaagatcag ttgggtgcac gagtgggtta catcgaactg 7140
gatctcaaca gcggtaagat ccttgagagt tttcgccccg aagaacgttt tccaatgatg 7200
agcactttta aagttctgct atgtggcgcg gtattatccc gtattgacgc cgggcaagag 7260
caactcggtc gccgcataca ctattctcag aatgacttgg ttgagtattc accagtcaca 7320
gaaaagcatc ttacggatgg catgacagta agagaattat gcagtgctgc cataaccatg 7380
agtgataaca ctgcggccaa cttacttctg acaacgatcg gaggaccgaa ggagctaacc 7440
gcttttttgc acaacatggg ggatcatgta actcgccttg atcgttggga accggagctg 7500
aatgaagcca taccaaacga cgagcgtgac accacgatgc ctgtagcaat ggcaacaacg 7560
ttgcgcaaac tattaactgg cgaactactt actctagctt cccggcaaca attaatagac 7620
tggatggagg cggataaagt tgcaggacca cttctgcgct cggcccttcc ggctggctgg 7680
tttattgctg ataaatctgg agccggtgag cgtgggtctc gcggtatcat tgcagcactg 7740
gggccagatg gtaagccctc ccgtatcgta gttatctaca cgacggggag tcaggcaact 7800
atggatgaac gaaatagaca gatcgctgag ataggtgcct cactgattaa gcattggtaa 7860
ctgtcagacc aagtttactc atatatactt tagattgatt taaaacttca tttttaattt 7920
aaaaggatct aggtgaagat cctttttgat aatctcatga ccaaaatccc ttaacgtgag 7980
ttttcgttcc actgagcgtc agaccccgta gaaaagatca aaggatcttc ttgagatcct 8040
ttttttctgc gcgtaatctg ctgcttgcaa acaaaaaaac caccgctacc agcggtggtt 8100
tgtttgccgg atcaagagct accaactctt tttccgaagg taactggctt cagcagagcg 8160
cagataccaa atactgtcct tctagtgtag ccgtagttag gccaccactt caagaactct 8220
gtagcaccgc ctacatacct cgctctgcta atcctgttac cagtggctgc tgccagtggc 8280
gataagtcgt gtcttaccgg gttggactca agacgatagt taccggataa ggcgcagcgg 8340
tcgggctgaa cggggggttc gtgcacacag cccagcttgg agcgaacgac ctacaccgaa 8400
ctgagatacc tacagcgtga gctatgagaa agcgccacgc ttcccgaagg gagaaaggcg 8460

gacaggtatc cggtaagcgg cagggtcgga acaggagagc gcacgaggga gcttccaggg 8520

ggaaacgcct ggtatcttta tagtcctgtc gggtttcgcc acctctgact tgagcgtcga 8580

tttttgtgat gctcgtcagg ggggcggagc ctatggaaaa acgccagcaa cgcggccttt 8640

ttacggttcc tggccttttg ctggcctttt gctcacatgt tctttcctgc gttatcccct 8700

gattctgtgg ataaccgtat taccgccttt gagtgagctg ataccgctcg ccgcagccga 8760

acgaccgagc gcagcgagtc agtgagcgag gaagcggaag agcgcccaat acgcaaaccg 8820

cctctccccg cgcgttggcc gattcattaa tgcag 8855

<210> 18
<211> 7997
<212> DNA
<213> Artificial Sequence

<220>
<223> v301 vector sequence

<400> 18
gtacacaccg cccgtcacac tatgggagct ggccatgccc gaagtcgtta ccttaaccgc 60

aaggaggggg atgccgaagg cagggctagt gactggagtg aagtcgtaac aaggtagccg 120

tactggaagg tgcggctgga tcacctcctt ttcagggaga gctaatgctt gttgggtatt 180

ttggtttgac actgcttcac acccccaaaa aaaagaaggg agctacgtct gagttaaact 240

tggagatgga agtcttcttt cctttctcga cggtgaagta agaccaagct catgagctta 300

ttatcctagg tcggaacaag ttgataggac cccctttttt acgtccccat gttccccccg 360

tgtggcgaca tgggggcgaa aaaaggaaag agagggatgg ggtttctctc gcttttggca 420

tagcgggccc ccagtgggag gctcgcacga cgggctatta gctcagtggt agagcgcgcc 480

cctgataatt gcgtcgttgt gcctgggctg tgagggctct cagccacatg gatagttcaa 540

tgtgctcatc ggcgcctgac cctgagatgt ggatcatcca aggcacatta gcatggcgta 600

ctcctcctgt tcgaaccggg gtttgaaacc aaactcctcc tcaggaggat agatggggcg 660

attcgggtga gatccaatgt agatccaact ttcgattcac tcgtgggatc cggcggtcc 720

ggggggggacc accacggctc ctctcttctc gagaatccat acatccctta tcagtgtatg 780

gacagctatc tctcgagcac aggtttagca atgggaaaat aaaatggagc acctaacaac 840

gcatcttcac agaccaagaa ctacgagatc gccccttca ttctggggtg acggagggat 900

cgtaccattc gagccgtttt tttcttgact cgaaatggga gcaggtttga aaaaggatct 960

tagagtgtct agggttgggc caggagggtc tcttaacgcc ttcttttttc ttctcatcgg 1020

agttatttca caaagacttg ccagggtaag gaagaagggg ggaacaagca cacttggaga 1080

gcgcagtaca acggagagtt gtatgctgcg ttcgggaagg atgaatcgct cccgaaaagg 1140

aatctattga ttctctccca attggttgga ccgtaggtgc gatgatttac ttcacgggcg 1200

aggtctctgg ttcaagtcca ggatggccca gctgcattta aatggcgcgc cgctcccccg 1260

ccgtcgttca atgagaatgg ataagaggct cgtgggattg acgtgagggg gcagggatgg 1320

ctatatttct gggagacgcg tatgtatttg gcaaatcaaa taccatggtc taataatcaa 1380

acattctgat tagttgataa tattagtatt agttggaaat tttgtgaaag attcctgtga 1440

aaagtttcat taacacggaa ttcgtgtcga gtagaccttg ttgttgtgag aattcttaat 1500

tcatgagttg tagggaggga tttcttaagc tcgagggatc cgtcgacctg cagtctcgaa 1560

gagcggagca tggagttgga tgacaggaga gagagcacaa gacataggaa aagtgttagt 1620

agatgactgt aattgcaata gcagcctact agctgccgat ccgagcttag tgatggactg 1680

tatgcgcgga gtcgatgcaa aaactatatc agtacagcag tggaattcct atactggcat 1740

attgggattc ccgtcagcac gagctcttcg cacgcatgca aatctaagtg tagtgcttgg 1800

tgtattgatt ttttttggaa agggagtgtg tgcgagttgt ttatttcaag aataggctgg 1860

accacccagc tgtacccttt tccgttataa ctaggaaaga gaggtgcatg atctcgcgaa 1920

ttacttctga ataaattcag aaattatatg taagaaccat agcatttcgc gattcattgg 1980

taaatcaatt ttgattctct attaaccaat aatgtggaac tattaacatg gttaaaacaa 2040

actgtttgaa gtctagacgc agcatggtac tctttctacc actatgttaa tatagaggtg 2100

gtttcaaaat aaatatttta tcgatatagg atactcatat tgataaaatg atttgaaccg 2160

cttattgtaa attgtaaaaa cggggatttt acccccttta tctaatgctg aatcgacgac 2220

ctattctaag taagaagagc tttttggatt tgaaaaaaaa aaaaagaaac aactttgctg 2280

acaattacat atttttttatt ttgtcagaag agtcctccga atattttggt cttacattag 2340

tttcgatatc tttttggaat atgaacaaag aagagaagat aggctcatta cattcataaa 2400

aaaagatatg agaatttacc ttaagtaatt gagcgtgaga gccaaatgaa tcgaaagatt 2460

catgtttggt tcgggaaggg attatggaat tttggaaagg aatggaaaaa taatctactt 2520

tcattaagtg atttattaga taatcgaaaa cagaggatct tgaatactat tcgacatatg 2580

tgtgcgaaga gctcgagctg acgggaatcc caatatgcca gtataggaat tccactgctg 2640

tactgatata gtttttgcat cgactccgcg catacagtcc atcactaagc tcggatcggc 2700

agctagtagg ctgctattgc aattacagtc atctactaac acttttccta tgtcttgtgc 2760

tctctctcct gtcatccaac tccatgctcc gctcttcgag aaagcttaga cattagcaga 2820

taaattagca ggaaataaag aaggataagg agaaagaact caagtaatta tccttcgttc 2880

tcttaattga attgcaatta aactcggccc aatcttttac taaaaggatt gagccgaata 2940

caacaaagat tctattgcat atattttgac taagtatata cttacctaga tatacaagat 3000

ttgaaataca aaatctagtc tagagcggcc gcagtagctt ataacttcgt atagcataca 3060

ttatacgaag ttatagatcc gctccccgc cgtcgttcaa tgagaatgga taagaggctc 3120
gtgggattga cgtgaggggg cagggatggc tatatttctg ggagaattaa ccgatcgacg 3180
tgcaagcgga catttatttt aaattcgata atttttgcaa aaacatttcg acatatttat 3240
ttattttatt attatgagaa tcaatcctac tacttctggt tctgggttt ccacggctag 3300
tagcgaagcg gtgatcgccg aagtatcgac tcaactatca gaggtagttg gcgtcatcga 3360
gcgccatctc gaaccgacgt tgctggccgt acatttgtac ggctccgcag tggatggcgg 3420
cctgaagcca cacagtgata ttgatttgct ggttacggtg accgtaaggc ttgatgaaac 3480
aacgcggcga gctttgatca acgacctttt ggaaacttcg gcttcccctg gagagagcga 3540
gattctccgc gctgtagaag tcaccattgt tgtgcacgac gacatcattc cgtggcgtta 3600
tccagctaag cgcgaactgc aatttggaga atggcagcgc aatgacattc ttgcaggtat 3660
cttcgagcca gccacgatcg acattgatct ggctatcttg ctgacaaaag caagagaaca 3720
tagcgttgcc ttggtaggtc cagcggcgga ggaactcttt gatccggttc ctgaacagga 3780
tctatttgag gcgctaaatg aaaccttaac gctatggaac tcgccgcccg actgggctgg 3840
cgatgagcga aatgtagtgc ttacgttgtc ccgcatttgg tacagcgcag taaccggcaa 3900
aatcgcgccg aaggatgtcg ctgccgactg ggcaatggag cgcctgccgg cccagtatca 3960
gcccgtcata cttgaagcta gacaggctta tcttggacaa gaagaagatc gcttggcctc 4020
gcgcgcagat cagttggaag aatttgtcca ctacgtgaaa ggcgagatca ccaaggtagt 4080
gggcaaagaa caaaaactca tttctgaaga agacttgtga gtctagctag agcgatcctg 4140
gcctagtcta taggaggttt tgaaaagaaa ggagcaataa tcattttctt gttctatcaa 4200
gagggtgcta ttgctccttt cttttttct ttttatttat ttactagtat tttacttaca 4260
tagacttttt tgtttacatt atagaaaaag aaggagaggt tattttcttg catttattca 4320
tgggggatca aagctgatct ataacttcgt atagcataca ttatacgaag ttatggtaca 4380
ctatttaaat gcgctccact tggctcgggg ggatatagct cagttggtag agctccgctc 4440
ttgcaattgg gtcgttgcga ttacgggttg gatgtctaat tgtccaggcg gtaatgatag 4500
tatcttgtac ctgaaccggt ggctcacttt ttctaagtaa tggggaagag gaccgaaacg 4560
tgccactgaa agactctact gagacaaaga tgggctgtca agaacgtaga ggaggtagga 4620
tgggcagttg gtcagatcta gtatggatcg tacatggacg gtagttggag tcggcggctc 4680
tcccagggtt ccctcatctg agatctctgg ggaagaggat caagttggcc cttgcgaaca 4740
gcttgatgca ctatctccct tcaacccttt gagcgaaatg cggcaaaaga aaaggaagga 4800
aaatccatgg accgacccca tcatctccac cccgtaggaa ctacgagatc accccaagga 4860
cgccttcggc atccaggggt cacggaccga ccatagaacc ctgttcaata agtggaacgc 4920
attagctgtc cgctctcagg ttgggcagtc agggtcggag aagggcaatg actcattctt 4980

agttagaatg ggattccaac tcagcacctt ttgagtgaga ttttgagaag agttgctctt 5040
tggagagcac agtacgatga aagttgtaag ctgtgttcgg gggggagtta ttgtctatcg 5100
ttggcctcta tggtagaatc agtcggggga cctgagaggc ggtggtttac cctgcggcgg 5160
atgtcagcgg ttcgagtccg cttatctcca actcgtgaac ttagccgata caaagctctg 5220
gcgtaatagc gaagaggccc gcaccgatcg cccttcccaa cagttgcgca gcctgaatgg 5280
cgaatggcgc ctgatgcggt attttctcct tacgcatctg tgcggtattt cacaccgcat 5340
acgtcaaagc aaccatagta cgcgccctgt agcggcgcat taagcgcggc gggtgtggtg 5400
gttacgcgca gcgtgaccgc tacacttgcc agcgccctag cgcccgctcc tttcgctttc 5460
ttcccttcct ttctcgccac gttcgccggc tttccccgtc aagctctaaa tcgggggctc 5520
cctttagggt tccgatttag tgctttacgg cacctcgacc ccaaaaaact tgatttgggt 5580
gatggttcac gtagtgggcc atcgccctga tagacggttt ttcgcccttt gacgttggag 5640
tccacgttct ttaatagtgg actcttgttc caaactggaa caacactcaa ccctatctcg 5700
ggctattctt ttgatttata agggattttg ccgatttcgg cctattggtt aaaaaatgag 5760
ctgatttaac aaaaatttaa cgcgaatttt aacaaaatat taacgtttac aattttatgg 5820
tgcactctca gtacaatctg ctctgatgcc gcatagttaa gccagccccg acacccgcca 5880
acacccgctg acgcgccctg acgggcttgt ctgctcccgg catccgctta cagacaagct 5940
gtgaccgtct ccgggagctg catgtgtcag aggttttcac cgtcatcacc gaaacgcgcg 6000
agacgaaagg gcctcgtgat acgcctattt ttataggtta atgtcatgat aataatggtt 6060
tcttagacgt caggtggcac ttttcgggga aatgtgcgcg gaacccctat ttgtttattt 6120
ttctaaatac attcaaatat gtatccgctc atgagacaat aaccctgata aatgcttcaa 6180
taatattgaa aaaggaagag tatgagtatt caacatttcc gtgtcgccct tattcccttt 6240
tttgcggcat tttgccttcc tgtttttgct cacccagaaa cgctggtgaa agtaaaagat 6300
gctgaagatc agttgggtgc acgagtgggt tacatcgaac tggatctcaa cagcggtaag 6360
atccttgaga gttttcgccc cgaagaacgt tttccaatga tgagcacttt taaagttctg 6420
ctatgtggcg cggtattatc ccgtattgac gccgggcaag agcaactcgg tcgccgcata 6480
cactattctc agaatgactt ggttgagtat tcaccagtca cagaaaagca tcttacggat 6540
ggcatgacag taagagaatt atgcagtgct gccataacca tgagtgataa cactgcggcc 6600
aacttacttc tgacaacgat cggaggaccg aaggagctaa ccgctttttt gcacaacatg 6660
ggggatcatg taactcgcct tgatcgttgg gaaccggagc tgaatgaagc cataccaaac 6720
gacgagcgtg acaccacgat gcctgtagca atggcaacaa cgttgcgcaa actattaact 6780
ggcgaactac ttactctagc ttcccggcaa caattaatag actggatgga ggcggataaa 6840

```
gttgcaggac cacttctgcg ctcggccctt ccggctggct ggtttattgc tgataaatct     6900
ggagccggtg agcgtgggtc tcgcggtatc attgcagcac tggggccaga tggtaagccc     6960
tcccgtatcg tagttatcta cacgacgggg agtcaggcaa ctatggatga acgaaataga     7020
cagatcgctg agataggtgc ctcactgatt aagcattggt aactgtcaga ccaagtttac     7080
tcatatatac tttagattga tttaaaactt catttttaat ttaaaaggat ctaggtgaag     7140
atcctttttg ataatctcat gaccaaaatc ccttaacgtg agttttcgtt ccactgagcg     7200
tcagaccccg tagaaaagat caaaggatct tcttgagatc ctttttttct gcgcgtaatc     7260
tgctgcttgc aaacaaaaaa accaccgcta ccagcggtgg tttgtttgcc ggatcaagag     7320
ctaccaactc tttttccgaa ggtaactggc ttcagcagag cgcagatacc aaatactgtc     7380
cttctagtgt agccgtagtt aggccaccac ttcaagaact ctgtagcacc gcctacatac     7440
ctcgctctgc taatcctgtt accagtggct gctgccagtg gcgataagtc gtgtcttacc     7500
gggttggact caagacgata gttaccggat aaggcgcagc ggtcgggctg aacggggggt     7560
tcgtgcacac agcccagctt ggagcgaacg acctacaccg aactgagata cctacagcgt     7620
gagctatgag aaagcgccac gcttcccgaa gggagaaagg cggacaggta tccggtaagc     7680
ggcagggtcg gaacaggaga gcgcacgagg gagcttccag ggggaaacgc ctggtatctt     7740
tatagtcctg tcgggtttcg ccacctctga cttgagcgtc gatttttgtg atgctcgtca     7800
ggggggcgga gcctatggaa aaacgccagc aacgcggcct ttttacggtt cctggccttt     7860
tgctggcctt ttgctcacat gttctttcct gcgttatccc ctgattctgt ggataaccgt     7920
attaccgcct ttgagtgagc tgataccgct cgccgcagcc gaacgaccga gcgcagcgag     7980
tcagtgagcg aggaagc                                                    7997
```